# EUROPEAN PATENT APPLICATION

(11) **EP 2 119 706 A1**
(43) Date of publication of application: **18.11.2009**
(21) Application number: 08711837.8
(22) Date of filing: 22.02.2008
(51) Int. Cl.: C07D 213/75, A61K 31/4412, A61K 31/4427, A61K 31/496, A61K 31/506, A61K 31/5377, A61P 35/00, C07D 401/12, C07D 401/14, C07D 403/12, C12N 15/09, C12Q 1/68, G01N 33/15, G01N 33/48, G01N 33/50, G01N 33/53, G01N 33/574

(54) **PYRIDINE OR PYRIMIDINE DERIVATIVE HAVING EXCELLENT CELL GROWTH INHIBITION EFFECT AND EXCELLENT ANTI-TUMOR EFFECT ON CELL STRAIN HAVING AMPLIFICATION OF HGFR GENE**

(30) Priority: 23.02.2007 JP 2007044424
(71) Applicant: Eisai R&D Management Co., Ltd., Tokyo 112-8088 (JP)
(72) Inventor: OBAISHI, Hiroshi, Tsukuba-shi Ibaraki 300-2635 (JP); NAKAGAWA, Takayuki, Tsukuba-shi Ibaraki 300-2635 (JP); MATSUSHIMA, Tomohiro, Tsukuba-shi Ibaraki 300-2635 (JP); FUNASAKA, Setsuo, Tsukuba-shi Ibaraki 300-2635 (JP); SHIROTORI, Shuji, Tsukuba-shi Ibaraki 300-2635 (JP); TAKAHASHI, Keiko, Tsukuba-shi Ibaraki 300-2635 (JP)
(74) Representative: HOFFMANN EITLE
(86) International application number: PCT/JP2008/053066
(87) International publication number: WO 2008/102870

(57) **Abstract**

A pyridine or pyrimidine derivative represented by the formula (I) has an excellent HGFR inhibitory activity and exhibits strong cell proliferation inhibitory effect and anti-tumor effect against cancer cell lines with amplified HGFR gene. 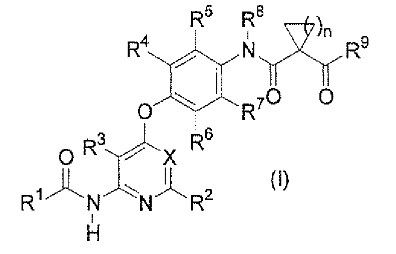 wherein R¹ represents a 3- to 10-membered non-aromatic heterocyclic group or the like; R² and R³ represent hydrogen; R⁴, R⁵, R⁶, and R⁷ may be the same or different and each represents hydrogen, halogen, C₁₋₆ alkyl or the like; R⁸ represents hydrogen or the like; R⁹ represents a 3- to 10-membered non-aromatic heterocyclic group or the like; n represents an integer of 1 or 2; X represents -CH=, nitrogen.

SEQUENCE LISTING

<110> Eisai R&D Manamgement Co., Ltd.

<120> PYRIDINE OR PYRIMIDINE DERIVATIVE HAVING EXCELLENT CELL GROWTH INHIBITION
EFFECT AND EXCELLENT ANTI-TUMOR EFFECT ON CELL STRAIN HAVING AMPLIFICATION OF
HGFR GENE

<130> FP08-0059

<150> JP 2007-44424
<151> 2007-02-23

<160> 2

<170> PatentIn version 3.1

<210> 1
<211> 33
<212> DNA
<213> Artificial

<220>
<223> an artificially synthesized primer sequence

<400> 1
ccggccggat ccaaaaagag aaagcaaatt aaa 33

<210> 2
<211> 33
<212> DNA
<213> Artificial

<220>
<223> an artificially synthesized primer sequence

<400> 2
ttaattctgc agctatgatg tctcccagaa gga 33

## Description

### Technical Field

The present invention relates to a pyridine or pyrimidine derivative, a salt thereof or a solvate of the foregoing. More particularly, the present invention relates to a method for predicting anti-tumor effect of a pyridine or pyrimidine derivative, a method for examining sensitivity of tumor cells to a pyridine or pyrimidine derivative, and a method for administering a pyridine or pyrimidine derivative to a tumor patient. Furthermore, the present invention relates to a pharmaceutical composition against tumors in which expression of hepatocyte growth factor receptor is amplified (also referred to as enhanced), a hepatocyte growth factor receptor inhibitor against tumors in which expression of hepatocyte growth factor receptor is amplified, and an anti-tumor agent against tumors in which expression of hepatocyte growth factor receptor is amplified. In addition, the present invention relates to a method for treating a disease comprising administering an effective dose of the pharmaceutical composition, use of a pyridine or pyrimidine derivative for the manufacture of the pharmaceutical composition, and a pyridine or pyrimidine derivative for the pharmaceutical composition.

### Background Art

Overexpression of hepatocyte growth factor receptor (hereafter referred to as "HGFR" and also referred to as "c-Met") is reported in various kinds of tumors such as a pancreatic cancer, a gastric cancer, a colorectal cancer, a breast cancer, a prostate cancer, a lung cancer, a renal cancer, a brain tumor or an ovarian cancer (non-patent document 1). HGFR expressed in these cancer cells is considered to be involved in cancer malignancy (aberrant growth, invasion or enhanced metastasis), because HGFR cause autophosphorylation of intracellular tyrosine kinase constitutively or upon stimulation by hepatocyte growth factor (hereafter referred to as "HGF").

It is also reported that HGFR is expressed in vascular endothelial cells and is involved in tumor angiogenesis since HGF stimulates HGFR to facilitate proliferation and migration of vascular endothelial cells (non-patent document 2).

Furthermore, it is also reported that there is a possibility that HGFR inhibitors exhibit high sensitivity against gastric cancer patients with amplified HGFR gene (non-patent document 3).

Therefore, a compound having inhibitory activity against HGFR is expected to be useful as an anti-tumor agent etc., especially an anti-tumor agent against tumors with amplified HGFR gene.

On the other hand, patent document 1 is a reference disclosing a pyridine or pyrimidine derivative having HGFR inhibitory activity.

[Patent document 1] WO 2005/082855
[Non-patent document 1] Oncol. Rep., 5: 1013-1024, 1998
[Non-patent document 2] Adv. Cancer Res., 67: 257-279, 1995
[Non-patent document 3] Proc. Natl. Acad. Sci. USA, 103(7): 2316-2321, 2006

### Disclosure of the Invention

### Problems to be Solved by the Invention

An object of the present invention is to find out a novel compound having inhibitory activity against HGFR and to provide an effective method for treating tumors based on the compound.

### Means for Solving the Problems

The present inventors have succeeded in synthesizing a novel pyridine or pyrimidine derivative represented by the formula (I), and found out that the compound has excellent inhibitory activity against HGFR. In addition, the present inventors have found that the compound exhibits stronger cell proliferation inhibitory effect and anti-tumor effect against cancer cell lines with amplified HGFR gene compared to cancer cell lines with no amplified HGFR gene. The present inventors have completed the present invention based on these findings.

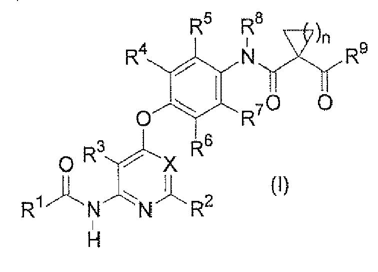

### (please refer to Best Mode for Carrying Out the Invention for the definitions of the respective substituents)

Specifically, the present invention provides [1] to [10] below.
[1] A method for predicting anti-tumor effect of a pyridine or pyrimidine derivative comprising the steps of:
   assaying expression level of hepatocyte growth factor receptor in tumor cells; and
   determining whether a pyridine or pyrimidine derivative is effective or not against the tumor cells by using the expression level of hepatocyte growth factor receptor as an index based on the assayed expression level,
   wherein the pyridine or pyrimidine derivative is at least one compound, salt thereof or solvate of the foregoing selected from the compound represented by the formula (I): 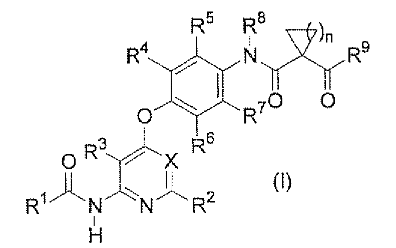
   (please refer to Best Mode for Carrying Out the Invention for the definitions of the respective substituents).
[2] A method for examining sensitivity of tumor cells to a pyridine or pyrimidine derivative comprising the steps of:
   assaying expression levels of hepatocyte growth factor receptor in tumor cells extracted from a tumor patient before and after administration of a pyridine or pyrimidine derivative; and
   determining that the tumor cells are sensitive to the pyridine or pyrimidine derivative if the expression level of hepatocyte growth factor receptor after administration of the pyridine or pyrimidine derivative is lower than the expression level of hepatocyte growth factor receptor before administration of the pyridine or pyrimidine derivative,
   wherein the pyridine or pyrimidine derivative is at least one compound, salt thereof or solvate of the foregoing selected from the compound represented by the formula (I): 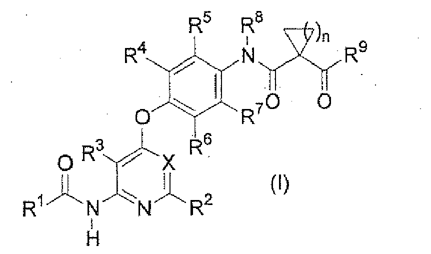 (please refer to Best Mode for Carrying Out the Invention for the definitions of the respective substituents).
[3] A pharmaceutical composition against tumors in which expression of hepatocyte growth factor receptor is enhanced, comprising at least one compound, salt thereof or solvate of the foregoing selected from the compound represented by the formula (I): 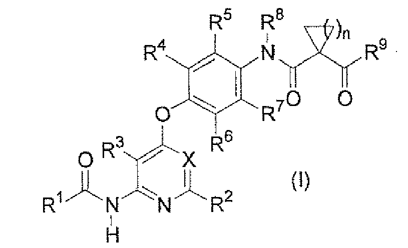 (please refer to Best Mode for Carrying Out the Invention for the definitions of the respective substituents).
[4] A hepatocyte growth factor receptor inhibitor against tumors in which expression of hepatocyte growth factor receptor is enhanced, comprising at least one compound, salt thereof or solvate of the foregoing selected from the compound represented by the formula (I): 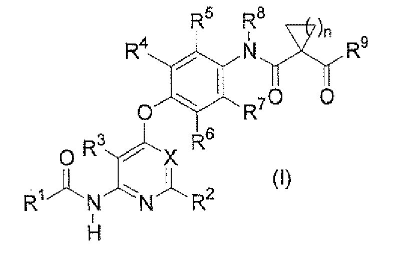 (please refer to Best Mode for Carrying Out the Invention for the definitions of the respective substituents).
[5] An anti-tumor agent against tumors in which expression of hepatocyte growth factor receptor is enhanced, comprising at least one compound, salt thereof or solvate of the foregoing selected from the compound represented by the formula (I): 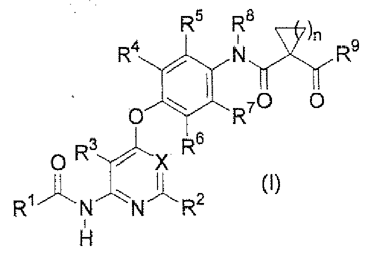 (please refer to Best Mode for Carrying Out the Invention for the definitions of the respective substituents).
[6] A method for administering a pyridine or pyrimidine derivative to a tumor patient, comprising the steps of:
   assaying expression level of hepatocyte growth factor receptor in tumor cells of a tumor patient;
   determining whether a pyridine or pyrimidine derivative is effective or not against the tumor by using the expression level of hepatocyte growth factor receptor as an index based on the assayed expression level; and
   administering the pyridine or pyrimidine derivative to the tumor patient in case of having determined effective,
   wherein the pyridine or pyrimidine derivative is at least one compound, salt thereof or solvate of the foregoing selected from the compound represented by the formula (I): 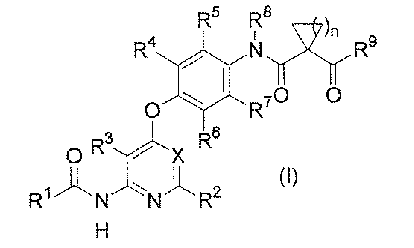 (please refer to Best Mode for Carrying Out the Invention for the definitions of the respective substituents).
[7] A method for administering a pyridine or pyrimidine derivative to a tumor patient, comprising the steps of:
   assaying expression levels of hepatocyte growth factor receptor in tumor cells of a tumor patient and a non-tumor individual; and
   administering a pyridine or pyrimidine derivative to the tumor patient if the expression level of hepatocyte growth factor receptor in tumor cells of the tumor patient is higher than the expression level of hepatocyte growth factor receptor in tumor cells of the non-tumor individual,
   wherein the pyridine or pyrimidine derivative is at least one compound, salt thereof or solvate of the foregoing selected from the compound represented by the formula (I): 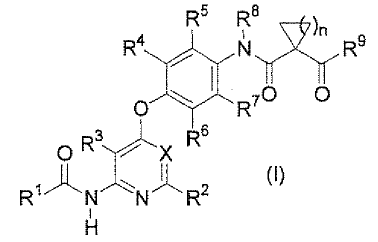 (please refer to Best Mode for Carrying Out the Invention for the definitions of the respective substituents).
[8] At least one compound, salt thereof or solvate of the foregoing selected from the compound represented by the formula (I) for treating tumors in which expression of hepatocyte growth factor receptor is enhanced: 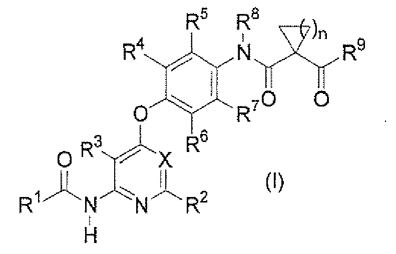 (please refer to Best Mode for Carrying Out the Invention for the definitions of the respective substituents).
[9] Use of at least one compound, salt thereof or solvate of the foregoing selected from the compound represented by the formula (I) for treating tumors in which expression of hepatocyte growth factor receptor is enhanced: 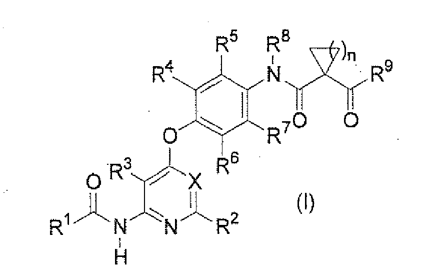 (please refer to Best Mode for Carrying Out the Invention for the definitions of the respective substituents).
[10] Use of at least one compound, salt thereof or solvate of the foregoing selected from the compound represented by the formula (I) for the manufacture of a therapeutic agent against tumors in which expression of hepatocyte growth factor receptor is enhanced: 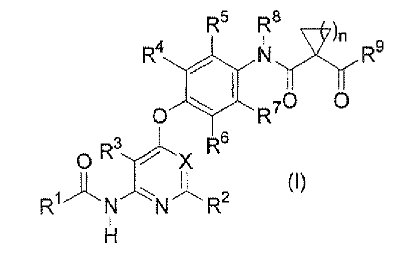 (please refer to Best Mode for Carrying Out the Invention for the definitions of the respective substituents).

### Effect of the Invention

The pyridine or pyrimidine derivative according to the present invention has an excellent HGFR inhibitory activity and exhibits strong cell proliferation inhibitory effect and anti-tumor effect against cancer cell lines with amplified HGFR gene. Thus, the pyridine or pyrimidine derivative according to the present invention is useful as an anti-tumor agent, especially as an anti-tumor agent against tumors with amplified HGFR gene. The present invention also provides a method for predicting effect against a tumor patient with amplified HGFR gene. Furthermore, since the present invention can predict effect against a tumor patient with amplified HGFR gene, it is possible to select patients against whom a compound is expected to exhibit more effect prior to administering the compound to the patients, and it is possible to contribute improved QOL of patients.

### Brief Description of the Drawings

Figure 1 shows the results of western blotting in pharmacological test example 2.
Figure 2 shows fluorescent staining images in pharmacological test example 3. (a) MKN-45 (b) SNU-5 (c) EBC-1
Figure 3 shows fluorescent staining images in pharmacological test example 3. (a) MKN-74 (b) SNU-1 (c) A549
Figure 4 shows the results of western blotting in pharmacological test example 4 (examples 15 and 61).
Figure 5 shows the results of western blotting in pharmacological test example 4 (examples 91 and 92).
Figure 6 shows the results of western blotting in pharmacological test example 4 (examples 94 and 96).

### Best Mode for Carrying Out the Invention

The symbols, terms etc. as used herein will be defined and the present invention will be described in details below. The following descriptions are for purposes of illustration for explaining the present invention and not limitation to the described embodiments only. All the technical terms, scientific terms and specialized terms have the same meanings as understood by those skilled in the art of the present invention, and are used for purposes of merely explaining a particular embodiment and not limitation. The present invention can be worked in various forms without departing from the scope of the invention. All the prior art references and patent documents such as unexamined patent publications and granted patent publications cited in the specification are hereby incorporated by reference and can be used for working the present invention.

First of all, the pyridine or pyrimidine derivative according to the present invention is explained. The pyridine or pyrimidine derivative according to the present invention is at least one compound, salt thereof or solvate of the foregoing selected from the compound represented by the formula (I): 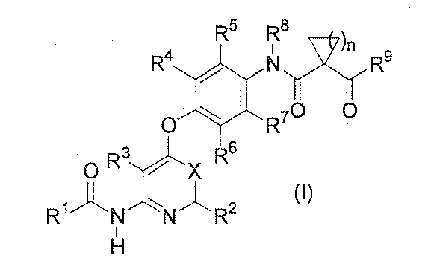 wherein R¹ represents a 3- to 10-membered non-aromatic heterocyclic group wherein the group is limited to a group having nitrogen as a ring constituent atom and the nitrogen having a bonding hand, or a group represented by the formula -NR^{11a}R^{11b}, wherein R^{11a} and R^{11b} may be the same or different and each represents hydrogen, C₁₋₆ alkyl, C₃₋₆ alkenyl, C₃₋₆ alkynyl, C₃₋₁₀ cycloalkyl, C₆₋₁₀ aryl, 5- to 10-membered heteroaryl or a 4- to 10-membered non-aromatic heterocyclic group, and R^{11a} and R^{11b} may be substituted with a substituent selected from Substituent Group A or Substituent Group B and R¹ may be substituted with a substituent selected from Substituent Group A or Substituent Group B;
R² and R³ represent hydrogen;
R⁴, R⁵, R⁶ and R⁷ may be the same or different and each represents hydrogen, halogen, hydroxyl, cyano, trifluoromethyl, C₁₋₆ alkyl, C₂₋₆ alkenyl, C₂₋₆ alkynyl, C₁₋₆ alkoxy, amino, mono-C₁₋₆ alkylamino, di-C₁₋₆ alkylamino or a group represented by the formula -CO-R¹², wherein R¹² represents hydrogen, hydroxyl, C₁₋₆ alkyl, C₁₋₆ alkoxy, amino, mono-C₁₋₆ alkylamino or di-C₁₋₆ alkylamino;
R⁸ represents hydrogen or C₁₋₆ alkyl;
R⁹ represents a 3- to 10-membered non-aromatic heterocyclic group
wherein the group is limited to a group having nitrogen as a ring constituent atom and the nitrogen having a bonding hand, or a group represented by the formula - NR^{11a}R^{11b}, wherein R^{11a} and R^{11b} represent the same meaning as described above and R⁹ may be substituted with a substituent selected from Substituent Group A or Substituent Group B;
n represents an integer of 1 or 2; and
X represents a group represented by the formula -C(R¹⁰)= or nitrogen,
wherein R¹⁰ represents hydrogen, halogen, cyano, C₁₋₆ alkyl, C₂₋₆ alkenyl, C₂₋₆ alkynyl or a group represented by the formula -CO-R¹², wherein R¹² represents the same meaning as recited above;
wherein Substituent Group A consists of halogen, hydroxyl, mercapto, nitro, cyano and oxo;
wherein Substituent Group B consists of C₁₋₆ alkyl, C₂₋₆ alkenyl, C₂₋₆ alkynyl, C₃₋₁₀ cycloalkyl, C₆₋₁₀ aryl, 5- to 10-membered heteroaryl, a 3- to 10-membered non-aromatic heterocyclic group, C₁₋₆ alkoxy, C₃₋₆ alkenyloxy, C₃₋₆ alkynyloxy, C₃₋₁₀ cycloalkoxy, C₆₋₁₀ aryloxy, 5- to 10-membered heteroaryloxy, 4-to 10-membered non-aromatic heterocyclicoxy, C₁₋₆ alkylthio, C₃₋₆ alkenylthio, C₃₋₆ alkynylthio, C₃₋₁₀ cycloalkylthio, C₆₋₁₀ arylthio, 5- to 10-membered heteroarylthio, 4- to 10-membered non-aromatic heterocyclicthio and a group represented by the formula -T¹-T²-T³, and each group in Substituent Group B may be substituted with a substituent selected from Substituent Group C, wherein T¹ represents a direct bond or C₁₋₆ alkylene, T² represents carbonyl, sulfinyl, sulfonyl, a group represented by the formula -C(=O)-O-, a group represented by the formula -O-C(=O)-, a group represented by the formula -SO₂-O-, a group represented by the formula -O-SO₂-, a group represented by the formula -NR^{T1}-, a group represented by the formula -C(=O)-NR^{T1}-, a group represented by the formula -NR^{T1}-C(=O)-, a group represented by the formula -SO₂-NR^{T1}- or a group represented by the formula -NR^{T1}-SO₂-, T³ represents hydrogen, C₁₋₆ alkyl, C₃₋₆ alkenyl, C₃₋₆ alkynyl, C₃₋₁₀ cycloalkyl, C₆₋₁₀ aryl, 5- to 10-membered heteroaryl or a 4- to 10-membered non-aromatic heterocyclic group, and R^{T1} represents hydrogen or C₁₋₆ alkyl; and
wherein Substituent Group C consists of halogen, hydroxyl, mercapto, nitro, cyano, oxo, C₁₋₆ alkyl, C₂₋₆ alkenyl, C₂₋₆ alkynyl, C₃₋₁₀ cycloalkyl, C₆₋₁₀ aryl, 5- to 10-membered heteroaryl, a 3- to 10-membered non-aromatic heterocyclic group, C₁₋₆ alkoxy, C₁₋₆ alkylthio, mono-C₁₋₆ alkylamino and di-C₁₋₆ alkylamino.

Several of the structural formulas for the compounds throughout the present specification represent only one isomeric form for convenience, but the invention encompasses any and all of the geometric isomers as well as optical isomers based on asymmetric carbons, stereoisomers and tautomers, and mixtures of those isomers, which are implied by the structures of the compounds, without being limited to any of the formulas shown for convenience. The compounds of the invention therefore include all those having asymmetric carbons therein and existing in optically active or racemic form, with no particular restrictions on the invention. There are also no restrictions when polymorphic crystalline forms thereof exist, and the compounds may be in one crystalline form or a mixture of different crystalline forms, while ansolvates and solvates of the pyridine or pyrimidine derivative of the invention are also included.

The so-called metabolite, a compound which a pyridine or pyrimidine derivative according to the present invention is metabolized in a living body through oxidation, reduction, hydrolysis, conjugation and the others to provide, and the so-called prodrug, a compound which is metabolized in a living body through oxidation, reduction, hydrolysis, conjugation and the others to provide a pyridine or pyrimidine derivative according to the present invention, are also included within the claimed scope of the present invention.

The "salt" includes a salt of an inorganic acid, a salt of an organic acid, a salt of an inorganic base, a salt of an organic base and a salt of an acidic or basic amino acid, among them, a pharmacologically acceptable salt is preferable.

The preferable salt of an inorganic acid includes, for example, a salt of hydrochloric acid, hydrobromic acid, sulfuric acid, nitric acid and phosphoric acid. The preferable salt of an organic acid includes, for example, a salt of acetic acid, succinic acid, fumaric acid, maleic acid, tartaric acid, citric acid, lactic acid, stearic acid, benzoic acid, methanesulfonic acid, ethanesulfonic acid, and p-toluenesulfonic acid.

The preferable salt of an inorganic base includes, for example, an alkali metal salt such as sodium salt and potassium salt, an alkali earth metal salt such as calcium salt and magnesium salt, aluminum salt, and ammonium salt. The preferable salt of an organic base includes, for example, a salt of diethylamine, diethanolamine, meglumine, and N,N-dibenzylethylenediamine.

The preferable salt of an acidic amino acid includes, for example, a salt of aspartic acid and glutamic acid. The preferable salt of a basic amino acid includes, for example, a salt of arginine, lysine and ornithine.

The "solvate" is a solvate of the pyridine or pyrimidine derivative or salt thereof according to the present invention, and preferably pharmacologically acceptable solvate. The solvent includes, for example, water, alcohols such as methanol, ethanol and n-propanol, dimethylformamide, dimethylsulfoxide, acetone.

The "halogen" represents fluorine, chlorine, bromine or iodine.

The "C₁₋₆ alkyl" represents an alkyl of straight or branched chain having a carbon number of 1 to 6, and includes, for specific example, methyl, ethyl, 1-propyl (n-propyl), 2-propyl (i-propyl), 2-methyl-1-propyl (i-butyl), 2-methyl-2-propyl (t-butyl), 1-butyl (n-butyl), 2-butyl (s-butyl), 1-pentyl, 2-pentyl, 3-pentyl, 2-methyl-1-butyl, 3-methyl-1-butyl, 2-methyl-2-butyl, 3-methyl-2-butyl, 2,2-dimethyl-1-propyl, 1-hexyl, 2-hexyl, 3-hexyl, 2-methyl-1-pentyl, 3-methyl-1-pentyl, 4-methyl-1-pentyl, 2-methyl-2-pentyl, 3-methyl-2-pentyl, 4-methyl-2-pentyl, 2-methyl-3-pentyl, 3-methyl-3-pentyl, 2,3-dimethyl-1-butyl, 3,3-dimethyl-1-butyl, 2,2-dimethyl-1-butyl, 2-ethyl-1-butyl, 3,3-dimethyl-2-butyl, and 2,3-dimethyl-2-butyl.

The "C₂₋₆ alkenyl" represents an alkenyl of straight or branched chain having one double bond and a carbon number of 2 to 6, and includes, for specific example, ethenyl (vinyl), 1-propenyl, 2-propenyl (allyl), 1-butenyl, 2-butenyl, 3-butenyl, pentenyl, and hexenyl.

The "C₃₋₆ alkenyl" represents an alkenyl of straight or branched chain having one double bond and a carbon number of 3 to 6, and includes, for specific example, 2-propenyl (allyl), 2-butenyl, 3-butenyl, pentenyl, and hexenyl.

The "C₂₋₆ alkynyl" represents an alkynyl of straight or branched chain having one triple bond and a carbon number of 2 to 6, and includes, for specific example, ethynyl, 1-propynyl, 2-propynyl, 1-butynyl, 2-butynyl, 3-butynyl, pentynyl, and hexynyl.

The "C₃₋₆ alkynyl" represents an alkynyl of straight or branched chain having one triple bond and a carbon number of 3 to 6, and includes, for specific example, 2-propynyl, 2-butynyl, 3-butynyl, pentynyl, and hexynyl.

The "C₁₋₆ alkylene" represents a divalent group derived by eliminating further any one hydrogen from the "C₁₋₆ alkyl" defined above, and includes, for specific example, methylene, 1,2-ethylene, 1,1-ethylene, 1,3-propylene, tetramethylene, pentamethylene, and hexamethylene.

The "C₃₋₁₀ cycloalkyl" represents a mono- or di-cyclic saturated aliphatic hydrocarbon group having a carbon number of 3 to 10, and includes, for specific example, cyclopropyl, cyclobutyl, cyclopentyl, cyclohexyl, cycloheptyl, cyclooctyl, cyclononyl, cyclodecyl, bicyclo[2.1.0]pentyl, bicyclo[3.1.0]hexyl, bicyclo[2.1.1]hexyl, bicyclo[4.1.0]heptyl, bicyclo[2.2.1]heptyl (norbornyl), bicyclo[3.3.0]octyl, bicyclo[3.2.1]octyl, bicyclo[2.2.2]octyl, bicyclo[4.3.0]nonyl, bicyclo[3.3.1]nonyl, bicyclo[4.4.0]decyl (decalyl), and bicyclo[3.3.2]decyl.

The "C₆₋₁₀ aryl" represents an aromatic hydrocarbon ring group having a carbon number of 6 to 10, and includes, for specific example, phenyl, 1-naphthyl, 2-naphthyl, indenyl, azulenyl, and heptalenyl.

The "heteroatom" represents nitrogen, oxygen, or sulfur.

The "5- to 10-membered heteroaryl" represents an aromatic ring group having 5 to 10 atoms forming the ring and containing 1 to 5 heteroatoms, and includes, for specific example, furyl, thienyl, pyrrolyl, imidazolyl, triazolyl, tetrazolyl, thiazolyl, pyrazolyl, oxazolyl, isoxazolyl, isothiazolyl, furazanyl, thiadiazolyl, oxadiazolyl, pyridyl, pyrazinyl, pyridazinyl, pyrimidinyl, triazinyl, purinyl, pteridinyl, quinolyl, isoquinolyl, naphthylidinyl, quinoxalinyl, cinnolinyl, quinazolinyl, phthalazinyl, imidazopyridyl, imidazothiazolyl, imidazoxazolyl, benzothiazolyl, benzoxazolyl, benzimidazolyl, indolyl, isoindolyl, indazolyl, pyrrolopyridyl, thienopyridyl, furopyridyl, benzothiadiazolyl, benzoxadiazolyl, pyridopyrimidinyl, benzofuryl, benzothienyl, and thienofuryl.

The preferable example of the "5- to 10-membered heteroaryl" includes furyl, thienyl, pyrrolyl, imidazolyl, thiazolyl, pyrazolyl, oxazolyl, isoxazolyl, isothiazolyl, pyridyl, and pyrimidinyl.

The "3- to 10-membered non-aromatic heterocyclic group" represents
(1) a monocyclic or a bicyclic non-aromatic heterocyclic group
(2) having 3 to 10 atoms in the ring,
(3) containing 1 to 2 heteroatoms among the atoms of the ring,
(4) optionally containing 1 to 2 double bonds in the ring,
(5) optionally containing 1 to 3 carbonyl, sulfinyl, or sulfonyl in the ring.
   If the group contains nitrogen in the ring, the nitrogen may have a bond not participating in the formation of the ring. The group includes, for specific example, aziridinyl, azetidinyl, pyrrolidinyl, piperidinyl, azepanyl, azocanyl, piperazinyl, diazepanyl, diazocanyl, diazabicyclo[2.2.1]heptyl, morpholinyl, thiomorpholinyl, 1,1-dioxothiomorpholinyl, oxiranyl, oxetanyl, tetrahydrofuryl, tetrahydropyranyl, dioxanyl, tetrahydrothienyl, tetrahydrothiopyranyl, oxazolidinyl, and thiazolidinyl.

The preferable example of the "3- to 10-membered non-aromatic heterocyclic group" includes aziridinyl, azetidinyl, pyrrolidinyl, piperidinyl, azepanyl, piperazinyl, diazepanyl, morpholinyl, thiomorpholinyl, 1,1-dioxothiomorpholinyl, tetrahydrofuryl, and tetrahydropyranyl.

The "4- to 10-membered non-aromatic heterocyclic group" represents
(1) a monocyclic or a bicyclic non-aromatic heterocyclic group
(2) having 4 to 10 atoms in the ring,
(3) containing 1 to 2 heteroatoms among the atoms of the ring,
(4) optionally containing 1 to 2 double bonds in the ring,
(5) optionally containing 1 to 3 carbonyl, sulfinyl, or sulfonyl in the ring.
   If the group contains nitrogen in the ring, the nitrogen may have a bond not participating in the formation of the ring. The group includes, for specific example, azetidinyl, pyrrolidinyl, piperidinyl, azepanyl, azocanyl, piperazinyl, diazepanyl, diazocanyl, diazabicyclo[2.2.1]heptyl, morpholinyl, thiomorpholinyl, 1,1-dioxothiomorpholinyl, oxetanyl, tetrahydrofuryl, tetrahydropyranyl, dioxanyl, tetrahydrothienyl, tetrahydrothiopyranyl, oxazolidinyl, and thiazolidinyl.

The preferable example of the "4- to 10-membered non-aromatic heterocyclic group" includes azetidinyl, pyrrolidinyl, piperidinyl, azepanyl, piperazinyl, diazepanyl, morpholinyl, thiomorpholinyl, 1,1-dioxothiomorpholinyl, tetrahydrofuryl, and tetrahydropyranyl.

The "C₃₋₁₀ cycloalkyl-C₁₋₆ alkyl" represents a group obtained by substituting any one hydrogen of the above defined "C₁₋₆ alkyl" with the above defined "C₃₋₁₀ cycloalkyl", and includes, for specific example, cyclopropylmethyl, cyclobutylmethyl, cyclopentylmethyl, cyclohexylmethyl, cycloheptylmethyl, cyclooctylmethyl, cyclononylmethyl, cyclodecylmethyl, bicyclo[2.2.1]heptylmethyl (norbornylmethyl), and bicyclo[4.4.0]decylmethyl (decarylmethyl).

The "C₆₋₁₀ aryl-C₁₋₆ alkyl" represents a group obtained by substituting any one hydrogen of the above defined "C₁₋₆ alkyl" with the above defined "C₆₋₁₀ aryl", and includes, for specific example, benzyl, 1-naphthylmethyl, 2-naphthylmethyl, phenethyl, 1-naphthylethyl, and 2-naphthylethyl.

The "5- to 10-membered heteroaryl-C₁₋₆ alkyl" represents a group obtained by substituting any one hydrogen of the above defined "C₁₋₆ alkyl" with the above defined "5- to 10-membered heteroaryl", and includes, for specific example, furylmethyl, thienylmethyl, pyrrolylmethyl, imidazolylmethyl, triazolylmethyl, tetrazolylmethyl, thiazolylmethyl, pyrazolylmethyl, oxazolylmethyl, isoxazolylmethyl, isothiazolylmethyl, furazanylmethyl, thiadiazolylmethyl, oxadiazolylmethyl, pyridylmethyl, pyrazinylmethyl, pyridazinylmethyl, pyrimidinylmethyl, triazinylmethyl, furylethyl, thienylethyl, pyrrolylethyl, imidazolylethyl, triazolylethyl, tetrazolylethyl, thiazolylethyl, pyrazolylethyl, oxazolylethyl, isoxazolylethyl, isothiazolylethyl, furazanylethyl, thiadiazolylethyl, oxadiazolylethyl, pyridylethyl, pyrazinylethyl, pyridazinylethyl, pyrimidinylethyl, and triazinylethyl.

The preferable example of the "5- to 10-membered heteroaryl C₁₋₆ alkyl" includes furylmethyl, thienylmethyl, pyrrolylmethyl, imidazolylmethyl, thiazolylmethyl, pyrazolylmethyl, oxazolylmethyl, isoxazolylmethyl, isothiazolylmethyl, pyridylmethyl, pyrimidinylmethyl, furylethyl, thienylethyl, pyrrolylethyl, imidazolyl ethyl, thiazolylethyl, pyrazolylethyl, oxazolylethyl, isoxazolylethyl, isothiazolylethyl, pyridylethyl, and pyrimidinylethyl.

The "3- to 10-membered non-aromatic heterocyclic-C₁₋₆ alkyl" represents a group obtained by substituting any one hydrogen of the above defined "C₁₋₆ alkyl" with the above defined "3- to 10-membered heterocyclic group", and includes, for specific example, aziridinylmethyl, azetidinylmethyl, pyrrolidinylmethyl, piperidinylmethyl, azepanylmethyl, azocanylmethyl, piperazinylmethyl, diazepanylmethyl, diazocanylmethyl, morpholinylmethyl, thiomorpholinylmethyl, 1,1-dioxothiomorpholinylmethyl, oxiranylmethyl, oxetanylmethyl, tetrahydrofurylmethyl, tetrahydropyranylmethyl, dioxanylmethyl, tetrahydrothienylmethyl, tetrahydrothiopyranylmethyl, oxazolidinylmethyl, thiazolidinylmethyl, aziridinylethyl, azetidinylethyl, pyrrolidinylethyl, piperidinylethyl, azepanylethyl, azocanylethyl, piperazinylethyl, diazepanylethyl, diazocanylethyl, morpholinylethyl, thiomorpholinylethyl, 1,1-dioxothiomorpholinylethyl, oxiranylethyl, oxetanylethyl, tetrahydrofurylethyl, tetrahydropyranylethyl, dioxanylethyl, tetrahydrothienylethyl, tetrahydrothiopyranylethyl, oxazolidinylethyl, and thiazolidinylethyl.

The preferable example of the "3- to 10-membered non-aromatic heterocyclic-C₁₋₆ alkyl" includes azetidinylmethyl, pyrrolidinylmethyl, piperidinylmethyl, azepanylmethyl, piperazinylmethyl, diazepanylmethyl, morpholinylmethyl, thiomorpholinylmethyl, tetrahydrofurylmethyl, azetidinylethyl, pyrrolidinylethyl, piperidinylethyl, azepanylethyl, piperazinylethyl, diazepanylethyl, morpholinylethyl, thiomorpholinylethyl, and tetrahydrofurylethyl.

The "C₁₋₆ alkoxy" represents a group obtained by adding oxygen to the terminal of the above defined "C₁₋₆ alkyl", and includes, for specific example, methoxy, ethoxy, 1-propoxy (n-propoxy), 2-propoxy (i-propoxy), 2-methyl-1-propoxy (i-butoxy), 2-methyl-2-propoxy (t-butoxy), 1-butoxy (n-butoxy), 2-butoxy (s-butoxy), 1-pentyloxy, 2-pentyloxy, 3-pentyloxy, 2-methyl-1-butoxy, 3-methyl-1-butoxy, 2-methyl-2-butoxy, 3-methyl-2-butoxy, 2,2-dimethyl-1-propoxy, 1-hexyloxy, 2-hexyloxy, 3-hexyloxy, 2-methyl-1-pentyloxy, 3-methyl-1-pentyloxy, 4-methyl-1-pentyloxy, 2-methyl-2-pentyloxy, 3-methyl-2-pentyloxy, 4-methyl-2-pentyloxy, 2-methyl-3-pentyloxy, 3-methyl-3-pentyloxy, 2,3-dimethyl-1-butoxy, 3,3-dimethyl-1-butoxy, 2,2-dimethyl-1-butoxy, 2-ethyl-1-butoxy, 3,3-dimethyl-2-butoxy, and 2,3-dimethyl-2-butoxy.

The "C₁₋₆ alkylthio" represents a group obtained by adding sulfur to the terminal of the above defined "C₁₋₆ alkyl", and includes, for specific example, methylthio, ethylthio, 1-propylthio (n-propylthio), 2-propylthio (i-propylthio), 2-methyl-1-propylthio (i-butylthio), 2-methyl-2-propylthio (t-butylthio), 1-butylthio (n-butylthio), 2-butylthio (s-butylthio), 1-pentylthio, 2-pentylthio, 3-pentylthio, 2-methyl-1-butylthio, 3-methyl-1-butylthio, 2-methyl-2-butylthio, 3-methyl-2-butylthio, 2,2-dimethyl-1-propylthio, 1-hexylthio, 2-hexylthio, 3-hexylthio, 2-methyl-1-pentylthio, 3-methyl-1-pentylthio, 4-methyl-1-pentylthio, 2-methyl-2-pentylthio, 3-methyl-2-pentylthio, 4-methyl-2-pentylthio, 2-methyl-3-pentylthio, 3-methyl-3-pentylthio, 2,3-dimethyl-1-butylthio, 3,3-dimethyl-1-butylthio, 2,2-dimethyl-1-butylthio, 2-ethyl-1-butylthio, 3,3-dimethyl-2-butylthio, and 2,3-dimethyl-2-butylthio.

The "C₃₋₆ alkenyloxy" represents a group obtained by adding oxygen to the terminal of the above defined "C₃₋₆ alkenyl", and includes, for specific example, 2-propenyloxy (allyloxy), 2-butenyloxy, 3-butenyloxy, pentenyloxy, and hexenyloxy.

The "C₃₋₆ alkenylthio" represents a group obtained by adding sulfur to the terminal of the above defined "C₃₋₆ alkenyl", and includes, for specific example, 2-propenylthio (allylthio), 2-butenylthio, 3-butenylthio, pentenylthio, and hexenylthio.

The "C₃₋₆ alkynyloxy" represents a group obtained by adding oxygen to the terminal of the above defined "C₃₋₆ alkynyl", and includes, for specific example, 2-propynyloxy, 2-butynyloxy, 3-butynyloxy, pentynyloxy, and hexynyloxy.

The "C₃₋₆ alkynylthio" represents a group obtained by adding sulfur to the terminal of the above defined "C₃₋₆ alkynyl", and includes, for specific example, 2-propynylthio, 2-butynylthio, 3-butynylthio, pentynylthio, and hexynylthio.

The "C₃₋₁₀ cycloalkoxy" represents a group obtained by adding oxygen to the terminal of the above defined "C₃₋₁₀ cycloalkyl", and includes, for specific example, cyclopropoxy, cyclobutoxy, cyclopentyloxy, cyclohexyloxy, cycloheptyloxy, and cyclooctyloxy.

The "C₃₋₁₀ cycloalkylthio" represents a group obtained by adding sulfur to the terminal of the above defined "C₃₋₁₀ cycloalkyl", and includes, for specific example, cyclopropylthio, cyclobutylthio, cyclopentylthio, cyclohexylthio, cycloheptylthio, and cyclooctylthio.

The "C₆₋₁₀ aryloxy" represents a group obtained by adding oxygen to the terminal of the above defined "C₆₋₁₀ aryl", and includes, for specific example, phenoxy, 1-naphthoxy, 2-naphthoxy, indenyloxy, azulenyloxy, and heptalenyloxy.

The "C₆₋₁₀ arylthio" represents a group obtained by adding sulfur to the terminal of the above defined "C₆₋₁₀ aryl", and includes, for specific example, phenylthio, 1-naphthylthio, 2-naphthylthio, indenylthio, azulenylthio, and heptalenylthio.

The "5- to 10-membered heteroaryloxy" represents a group obtained by adding oxygen to the terminal of the above defined "5- to 10-membered heteroaryl", and includes, for specific example, furyloxy, thienyloxy, pyrrolyloxy, imidazolyloxy, triazolyloxy, thiazolyloxy, pyrazolyloxy, oxazolyloxy, isoxazolyloxy, isothiazolyloxy, furazanyloxy, thiadiazolyloxy, oxadiazolyloxy, pyridyloxy, pyrazinyloxy, pyridazinyloxy, pyrimidinyloxy, and triazinyloxy.

The "5- to 10-membered heteroarylthio" represents a group obtained by adding sulfur to the terminal of the above defined "5- to 10-membered heteroaryl", and includes, for specific example, furylthio, thienylthio, pyrrolylthio, imidazolylthio, triazolylthio, thiazolylthio, pyrazolylthio, oxazolylthio, isoxazolylthio, isothiazolylthio, furazanylthio, thiadiazolylthio, oxadiazolylthio, pyridylthio, pyrazinylthio, pyridazinylthio, pyrimidinylthio, and triazinylthio.

The "4- to 10-membered non-aromatic heterocyclicoxy group" represents a group obtained by adding oxygen to the terminal of the above defined "4- to 10-membered non-aromatic heterocyclic group", and includes, for specific example, azetidinyloxy, pyrrolidinyloxy, piperidinyloxy, azepanyloxy, azocanyloxy, piperazinyloxy, diazepanyloxy, diazocanyloxy, morpholinyloxy, thiomorpholinyloxy, 1,1-dioxothiomorpholinyloxy, oxetanyloxy, tetrahydrofuryloxy, tetrahydropyranyloxy, tetrahydrothienyloxy, and tetrahydrothiopyranyloxy.

The "4- to 10-membered non-aromatic heterocyclicthio group" represents a group obtained by adding sulfur to the terminal of the above defined "4- to 10-membered non-aromatic heterocyclic group", and includes, for specific example, azetidinylthio, pyrrolidinylthio, piperidinylthio, azepanylthio, azocanylthio, piperazinylthio, diazepanylthio, diazocanylthio, oxetanylthio, tetrahydrofurylthio, tetrahydropyranylthio, tetrahydrothienylthio, and tetrahydrothiopyranylthio.

The "mono-C₁₋₆ alkylamino" represents a group obtained by substituting one hydrogen of amino with the above defined "C₁₋₆ alkyl", and includes, for specific example, methylamino, ethylamino, 1-propylamino (n-propylamino), 2-propylamino (i-propylamino), 2-methyl-1-propylamino (i-butylamino), 2-methyl-2-propylamino (t-butylamino), 1-butylamino (n-butylamino), 2-butylamino (s-butylamino), 1-pentylamino, 2-pentylamino, 3-pentylamino, 2-methyl-1-butylamino, 3-methyl-1-butylamino, 2-methyl-2-butylamino, 3-methyl-2-butylamino, 2,2-dimethyl-1-propylamino, 1-hexylamino, 2-hexylamino, 3-hexylamino, 2-methyl-1-pentylamino, 3-methyl-1-pentylamino, 4-methyl-1-pentylamino, 2-methyl-2-pentylamino, 3-methyl-2-pentylamino, 4-methyl-2-pentylamino, 2-methyl-3-pentylamino, 3-methyl-3-pentylamino, 2,3-diethyl-1-butylamino, 3,3-dimethyl-1-butylamino, 2,2-dimethyl-1-butylamino, 2-ethyl-1-butylamino, 3,3-dimethyl-2-butylamino, and 2,3-dimethyl-2-butylamino.

The "mono-C₃₋₁₀ cycloalkylamino" represents a group obtained by substituting one hydrogen of amino with the above defined "C₃₋₁₀ cycloalkyl", and includes, for specific example, cyclopropylamino, cyclobutyl amino, cyclopentylamino, cyclohexylamino, cycloheptylamino, and cyclooctylamino.

The "mono-C₆₋₁₀ arylamino" represents a group obtained by substituting one hydrogen of amino with the above defined "C₆₋₁₀ aryl", and includes, for specific example, phenylamino, 1-naphthylamino, 2-naphthylamino, indenylamino, azulenylamino, and heptalenylamino.

The "mono-5- to 10-membered heteroarylamino" represents a group obtained by substituting one hydrogen of amino with the above defined "5- to 10-membered heteroaryl", and includes, for specific example, furylamino, thienylamino, pyrrolylamino, imidazolylamino, triazolylamino, tetrazolylamino, thiazolylamino, pyrazolylamino, oxazolylamino, isoxazolylamino, isothiazolylamino, furazanylamino, thiadiazolylamino, oxadiazolylamino, pyridylamino, pyrazinylamino, pyridazinylamino, pyrimidinylamino, and triazinylamino.

The preferable example of the "mono-5- to 10-membered heteroarylamino" includes furylamino, thienylamino, pyrrolylamino, imidazolylamino, thiazolylamino, pyrazolylamino, oxazolylamino, isoxazolylamino, isothiazolylamino, pyridylamino, and pyrimidinylamino.

The "mono-4- to 10-membered non-aromatic heterocyclic amino" represents a group obtained by substituting one hydrogen of amino with the above defined "4- to 10-membered non-aromatic heterocyclic group", and includes, for specific example, azetidinylammo, pyrrolidinylamino, piperidinylamino, azepanylamino, azocanylamino, piperazinylamino, diazepanylamino, diazocanylamino, morpholinylamino, thiomorpholinylamino, 1,1-dioxothiomorpholinylamino, oxetanylamino, tetrahydrofurylamino, tetrahydropyranylamino, tetrahydrothienylamino, and tetrahydrothiopyranylamino.

The preferable example of the "mono-4- to 10-membered non-aromatic heterocyclic amino" includes pyrrolidinylamino, piperidinylamino, azepanylamino, piperazinylamino, diazepanylamino, morpholinylamino, thiomorpholinylamino, and tetrahydrofurylamino.

The "di-C₁₋₆ alkylamino" represents a group obtained by substituting two hydrogen of amino with the same or different groups of the above defined "C₁₋₆ alkyl", and includes, for specific example, N,N-dimethylamino, N,N-diethylamino, N,N-di-n-propylamino, N,N-di-i-propylamino, N,N-di-n-butylamino, N,N-di-i-butylamino, N,N-di-s-butylamino, N,N-di-t-butylamino, N-ethyl-N-methylamino, N-n-propyl-N-methylamino, N-i-propyl-N-methylamino, N-n-butyl-N-methylamino, N-i-butyl-N-methylamino, N-s-butyl-N-methylamino, and N-t-butyl-N-methylamino.

Each of the substituents in the pyridine of pyrimidine derivative of the present invention represented by the above formula (I) will be described below.

### (Meaning of R¹)

R¹ represents a 3- to 10-membered non-aromatic heterocyclic group
wherein the group is limited to a group having nitrogen as a ring constituent atom and the nitrogen having a bonding hand, or a group represented by the formula - NR^{11a}R^{11b}, wherein R^{11a} and R^{11b} may be the same or different and each represents hydrogen, C₁₋₆ alkyl, C₃₋₆ alkenyl, C₃₋₆ alkynyl, C₃₋₁₀ cycloalkyl, C₆₋₁₀ aryl, 5- to 10-membered heteroaryl or a 4- to 10-membered non-aromatic heterocyclic group, and R^{11a} and R^{11b} may be substituted with a substituent selected from Substituent Group A or Substituent Group B.
R¹ may be substituted with a substituent selected from Substituent Group A or Substituent Group B.
The preferable example of R¹ includes a group represented by the formula (II): 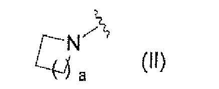 wherein a represents an integer of 1 to 4;
a group represented by the formula (III): 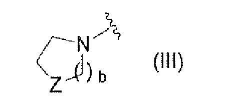 wherein b represents an integer of 1 to 3, and Z represents oxygen, sulfur, carbonyl, sulfonyl, or a group represented by the formula -NR^{Z}-, wherein R^{Z} represents hydrogen or C₁₋₆ alkyl, and the groups represented by the formula (II) or (III) may be substituted with a substituent selected from Substituent Group A or Substituent Group B; or
a group represented by the formula -NR^{11c}R^{11d}, wherein R^{11c} represents hydrogen or C₁₋₆ alkyl, and R^{11d} represents C₁₋₆ alkyl or a group represented by the formula (IV): 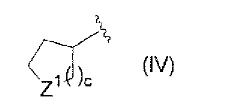 wherein c represents an integer of 1 to 3, and Z¹ represents oxygen, sulfur, carbonyl, sulfonyl or a group represented by the formula -NR^{Z1}-, wherein R^{Z1} represents hydrogen or C₁₋₆ alkyl, and R^{11d} may be substituted with a substituent selected from Substituent Group A or Substituent Group B.
The more preferable example of R¹ includes azetidin-1-yl, pyrrolidin-1-yl, piperidin-1-yl, azepan-1-yl, piperazin-1-yl, diazepan-1-yl, morpholin-4-yl, thiomorpholin-4-yl, 1,1-dioxothiomorpholin-4-yl, or a group represented by the formula -NR^{11e}R^{11f}, wherein R^{11e} represents hydrogen or C₁₋₆ alkyl, R^{11f} represents C₁₋₆ alkyl, pyrrolidin-3-yl, piperidin-3-yl, piperidin-4-yl or tetrahydropyran-4-yl, and R^{11f} may be substituted with a substituent selected from Substituent Group D, and each of the above substituents may be substituted with a substituent selected from Substituent Group D.
The even more preferable example of R¹ includes azetidin-1-yl, pyrrolidin-1-yl, piperidin-1-yl, piperazin-1-yl, diazepan-1-yl, morpholin-4-yl, and each of the above substituents may be substituted with a substituent selected from Substituent Group E, or a group represented by the formula -NR^{11g}R^{11h}, wherein R^{11g} represents hydrogen or methyl, R^{11h} represents n-propyl, n-butyl, pyrrolidin-3-yl, piperidin-3-yl, piperidin-4-yl or tetrahydropyran-4-yl, and R^{11h} may be substituted with a substituent selected from Substituent Group F.
The especially preferable example of R¹ includes azetidin-1-yl, pyrrolidin-1-yl, piperidin-1-yl or piperazin-1-yl, wherein azetidin-1-yl may be substituted with a substituent selected from Substituent Group G and pyrrolidin-1-yl, piperidin-1-yl and piperazin-1-yl are substituted with a substituent selected from Substituent Group G, or a group represented by the formula -N(CH₃)R¹¹ⁱ wherein R¹¹ⁱ represents n-propyl, n-butyl, pyrrolidin-3-yl or piperidin-4-yl, and R¹¹ⁱ is substituted with a substituent selected from Substituent Group H.
The most preferable example of R¹ includes azetidin-1-yl, pyrrolidin-1-yl, piperidin-1-yl or piperazin-1-yl, wherein azetidin-1-yl may be substituted with a substituent selected from Substituent Group G-1 and pyrrolidin-1-yl, piperidin-1-yl and piperazin-1-yl are substituted with a substituent selected from Substituent Group G-1, or azetidin-1-yl having dimethylamino, pyrrolidin-1-yl having dimethylamino or piperidin-1-yl having dimethylamino, a group represented by the formula -N(CH₃)R^{11j} wherein R^{11j} represents 1-methylpiperidin-4-yl or 1-ethylpiperidin-4-yl, azetidin-1-yl optionally substituted with a substituent selected from Substituent Group G-2, pyrrolidin-1-yl substituted with a substituent selected from Substituent Group G-2, piperidin-1-yl substituted with a substituent selected from Substituent Group G-2 or a group represented by the formula -N(CH₃)^{11k},
wherein R^{11k} represents 3-(dimethylamino)propyl or 1-[2-(dmiethylamino)ethyl]piperidin-4-yl.
The most preferable example of R¹ also includes [2-(dimethylamino)ethyl]piperazin-1-yl, 4-pyrrolidin-1-ylpiperidin-1-yl, 4-[(dimethylamino)methyl]piperidin-1-yl, 4-azetidin-1-ylpiperidin-1-yl, 4-[3-(dimethylamino)azetidin-1-yl]piperidin-1-yl, 4-(4-methylpiperazin-1-yl)piperidin-1-yl, 4-(1-methylpiperidin-4-yl)piperazin-1-yl, 4-(1-methylazetidin-3-yl)piperazin-1-yl, 4-(dimethylamino)piperidin-1-yl, 4-(azetidin-1-ylmethyl)piperidin-1-yl, 4-(pyrrolidin-1-ylmethyl)piperidin-1-yl, (3S)-3-(dimethylamino)pyrrolidin-1-yl, (3R)-3-(dimethylamino)pyrrolidin-1-yl, azetidin-1-yl, pyrrolidin-1-yl, morpholin-4-yl, 4-methylpiperazin-1-yl, 3-hydroxyazetidin-1-yl, 1,3'-biazetidin-1'-yl, 3-(hydroxymethyl)azetidin-1-yl, 3-(dimethylamino)azetidin-1-yl, 3-[(dimethylamino)methyl]azetidin-1-yl, 4-hydroxypiperidin-1-yl, 4-(hydroxymethyl)piperidin-1-yl, (3R)-3-hydroxypyrrolidin-1-yl, (3S)-3-hydroxypyrrolidin-1-yl, 3-(azetidin-1-ylmethyl)azetidin-1-yl, 3-(2-dimethylaminoacetoxy)azetidin-1-yl, methyl(1-methylpiperidin-4-yl)amino, (1-ethylpiperidin-4-yl)(methyl)amino, [3-(dimethylamino)propyl](methyl)amino or {1-[2-(dimethylamino)ethyl]piperidin-4-yl}(methyl)amino.

### (Meaning of Substituent Group A)

The Substituent Group A represents a group consisting of halogen, hydroxyl, mercapto, nitro, cyano and oxo.

### (Meaning of Substituent Group B)

The Substituent Group B represents a group consisting of C₁₋₆ alkyl, C₂₋₆ alkenyl, C₂₋₆ alkynyl, C₃₋₁₀ cycloalkyl, C₆₋₁₀ aryl, 5- to 10-membered heteroaryl, a 3- to 10-membered non-aromatic heterocyclic group, C₁₋₆ alkoxy, C₃₋₆ alkenyloxy, C₃₋₆ alkynyloxy, C₃₋₁₀ cycloalkoxy, C₆₋₁₀ aryloxy, 5- to 10-membered heteroaryloxy, 4- to 10-membered non-aromatic heterocyclicoxy, C₁₋₆ alkylthio, C₃₋₆ alkenylthio, C₃₋₆ alkynylthio, C₃₋₁₀ cycloalkylthio, C₆₋₁₀ arylthio, 5- to 10-membered heteroarylthio, 4- to 10-membered non-aromatic heterocyclicthio and a group represented by the formula T¹-T²-T³, wherein T¹ represents a direct bond or C₁₋₆ alkylene, T² represents carbonyl, sulfinyl, sulfonyl, a group represented by the formula -C(=O)-O-, a group represented by the formula -O-C(=O)-, a group represented by the formula -SO₂-O-, a group represented by the formula -O-SO₂-, a group represented by the formula -NR^{T1}-, a group represented by the formula - C(=O)-NR^{T1}-, a group represented by the formula -NR^{T1}-C(=O)-, a group represented by the formula -SO₂-NR^{T1}- or a group represented by the formula - NR^{T1}-SO₂-, T³ represents hydrogen, C₁₋₆ alkyl, C₃₋₆ alkenyl, C₃₋₆ alkynyl, C₃₋₁₀ cycloalkyl, C₆₋₁₀ aryl, 5- to 10-membered heteroaryl or a 4- to 10-membered non-aromatic heterocyclic group, and R^{T1} represents hydrogen or C₁₋₆ alkyl.
Each group included in Substituent Group B may be substituted with a substituent selected from Substituent Group C.

### (Meaning of Substituent Group C)

The Substituent Group C represents a group consisting of halogen, hydroxyl, mercapto, nitro, cyano, oxo, C₁₋₆ alkyl, C₂₋₆ alkenyl, C₂₋₆ alkynyl, C₃₋₁₀ cycloalkyl, C₆₋₁₀ aryl, 5- to 10-membered heteroaryl, a 3- to 10-membered non-aromatic heterocyclic group, C₁₋₆ alkoxy, C₁₋₆ alkylthio, mono-C₁₋₆ alkylamino and di-C₁₋₆ alkylamino.

### (Meaning of Substituent Group D)

The Substituent Group D represents a group consisting of halogen, hydroxyl, mercapto, cyano, formyl, oxo, C₁₋₆ alkyl, C₃₋₁₀ cycloalkyl, C₁₋₆ alkoxy, amino, mono-C₁₋₆ alkylamino, di-C₁₋₆ alkylamino, azetidinyl, pyrrolidinyl, piperidinyl, piperazinyl, diazepanyl and a group represented by -T⁴-T⁵, wherein T⁴ represents carbonyl or sulfonyl, and T⁵ represents C₁₋₆ alkyl, C₃₋₁₀ cycloalkyl, azetidinyl, pyrrolidinyl, piperidinyl, hydroxyl, C₁₋₆ alkoxy, amino, mono-C₁₋₆ alkylamino or di-C₁₋₆ alkylamino.
Each group included in Substituent Group D may be substituted with hydroxyl, C₁₋₆ alkyl, di-C₁₋₆ alkylamino, azetidinyl or pyrrolidinyl.

### (Meaning of Substituent Group E)

The Substituent Group E represents a group consisting of methyl, ethyl, dimethylamino, azetidinyl, pyrrolidinyl, piperidinyl and piperazinyl.
Each group included in Substituent Group E may be substituted with hydroxyl, methyl, dimethylamino, azetidinyl, pyrrolidinyl or piperidinyl.

### (Meaning of Substituent Group F)

The Substituent Group F represents a group consisting of methyl, ethyl, n-propyl, acetyl, dimethylamino, diethylamino, azetidinyl, pyrrolidinyl and piperazinyl.
Each group included in Substituent Group F may be substituted with methyl or dimethylamino.

### (Meaning of Substituent Group G)

The Substituent Group G represents a group consisting of dimethylamino, azetidinyl, pyrrolidinyl, piperidinyl, piperazinyl, dimethylaminomethyl, dimethylaminoethyl, azetidin-1-ylmethyl, pyrrolidin-1-ylmethyl and piperidin-1-ylmethyl.
Each group included in Substituent Group G may be substituted with methyl or dimethylamino.

### (Meaning of Substituent Group G-1)

The Substituent Group G-1 represents a group consisting of azetidinyl, pyrrolidinyl, piperidinyl, piperazinyl, dimethylaminomethyl, dimethylaminoethyl, azetidin-1-ylmethyl, pyrrolidin-1-ylmethyl and piperidin-1-ylmethyl.
Each group included in Substituent Group G-1 may be substituted with methyl or dimethylamino.

### (Meaning of Substituent Group G-2)

The Substituent Group G-2 represents a group consisting of hydroxyl, methoxy, hydroxymethyl and dimethylaminoacetoxy.

### (Meaning of Substituent Group H)

The Substituent Group H represents a group consisting of dimethylamino, diethylamino, dimethylaminoethyl, dimethylaminopropyl and 1-methylazetidin-3-yl.

### (Meaning of R² and R³)

R² and R³ represent hydrogen.

### (Meaning of R⁴, R⁵, R⁶ and R⁷)

R⁴, R⁵, R⁶ and R⁷ may be the same or different and each represents hydrogen, halogen, hydroxyl, cyano, trifluoromethyl, C₁₋₆ alkyl, C₂₋₆ alkenyl, C₂₋₆ alkynyl, C₁₋₆ alkoxy, amino, mono-C₁₋₆ alkylamino, di-C₁₋₆ alkylamino or a group represented by the formula -CO-R¹², wherein R¹² represents hydrogen, hydroxyl, C₁₋₆ alkyl, C₁₋₆ alkoxy, amino, mono-C₁₋₆ alkylamino or di-C₁₋₆ alkylamino.
The preferable example of R⁴, R⁵, R⁶ and R⁷ includes hydrogen, halogen, C₁₋₆ alkyl, C₁₋₆ alkoxy and trifluoromethyl.
The more preferable example of R⁴, R⁵, R⁶ and R⁷ includes hydrogen, halogen and C₁₋₆ alkyl.
The even more preferable example of R⁴, R⁵, R⁶ and R⁷ includes hydrogen, fluorine, chlorine and methyl.
R⁴, R⁵, R⁶ and R⁷ may be in any one of the following cases: (1) all of them represent hydrogen, (2) all of them represent substituents other than hydrogen, and (3) some of them represent hydrogen and the others represent substituents other than hydrogen. Preferably, 2 to 4 of R⁴, R⁵, R⁶ and R⁷ represent hydrogen.
Preferable example for a group represented by the formula: 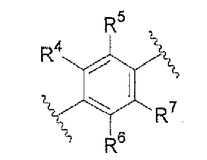 includes groups represented by the formulas: 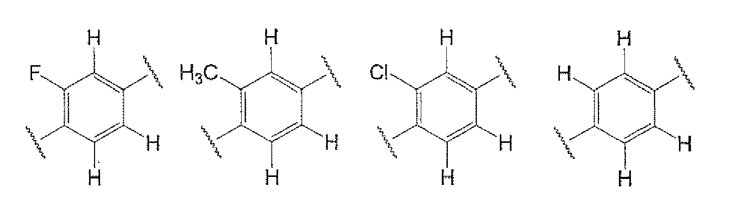 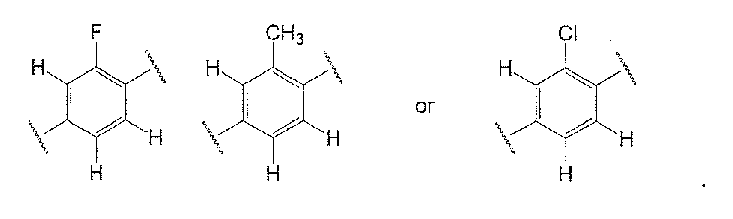 or a group represented by the formula:

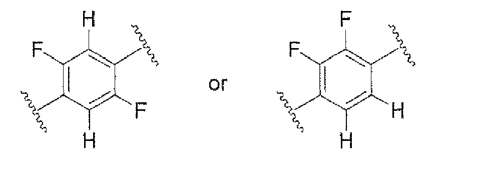

### (Meaning of R⁸)

R⁸ represents hydrogen or C₁₋₆ alkyl.
The preferable example of R⁸ includes hydrogen.

### (Meaning of R⁹)

R⁹ represents a 3- to 10-membered non-aromatic heterocyclic group
wherein the group is limited to a group having nitrogen as a ring constituent atom and the nitrogen having a bonding hand, or a group represented by the formula - NR^{11a}R^{11b}, wherein R^{11a} and R^{11b} represent the same meaning as described above.
R⁹ may be substituted with a substituent selected from Substituent Group A or Substituent Group B.
The preferable example of R⁹ includes mono-C₁₋₆ alkylamino, mono-C₃₋₁₀ cycloalkylamino, mono-C₆₋₁₀ arylamino, mono-5- to 10-membered heteroarylamino or mono-4- to 10-membered non-aromatic heterocyclic amino,
wherein R⁹ may be substituted with a substituent selected from Substituent Group A or Substituent Group B.
The more preferable example of R⁹ includes mono-C₃₋₁₀ cycloalkylamino or mono-C₆₋₁₀ arylamino, wherein R⁹ may be substituted with a substituent selected from Substituent Group A or Substituent Group B.
The even more preferable example of R⁹ includes mono-C₃₋₁₀ cycloalkylamino or mono-C₆₋₁₀ arylamino, wherein R⁹ may be substituted with a substituent selected from Substituent Group I.
The Substituent Group I represents a group consisting of halogen, trifluoromethyl, cyano, C₁₋₆ alkyl and C₁₋₆ alkoxy.
The especially preferable example of R⁹ includes cyclopentylamino, cyclohexylamino, cycloheptylamino and phenylamino, wherein R⁹ may be substituted with a substituent selected from Substituent Group I.
The most preferable example of R⁹ includes phenylamino optionally substituted with a substituent selected from the above Substituent Group I.

### (Meaning of n)

n represents an integer of 1 or 2.
The preferable example of n includes 1.

### (Meaning of X)

X represents a group represented by the formula -C(R¹⁰)= or nitrogen,
wherein R¹⁰ represents hydrogen, halogen, cyano, C₁₋₆ alkyl, C₂₋₆ alkenyl, C₂₋₆ alkynyl or a group represented by the formula -CO-R¹², wherein R¹² represents the same meaning as described above.
The preferable example of X includes a group represented by the formula - C(R^{10a})= or nitrogen, wherein R^{10a} represents hydrogen, halogen or cyano.
The more preferable example of X includes a group represented by the formula -CH= or nitrogen.

The preferable compound of the formula (I) includes a compound obtained by selecting respective aspects of R¹, R², R³, R⁴, R⁵, R⁶, R⁷, R⁸, R⁹, X and n in the compound and combining them arbitrarily.

The preferable compound of the formula (I) includes, other than the compounds described in Examples, the compounds illustrated below; but the present invention is not limited to the compounds described in Examples and the compounds illustrated below.
(1) N-(4-{[2-({[(1-ethylpiperidin-4-yl)(methyl)amino]carbonyl}amino)pyridin-4-yl]oxy}-2-fluorophenyl)-N'-(4-fluorophenyl)cyclopropane-1,1-dicarboxamide,
(2) N-(4-{[2-({[(1-ethylpiperidin-4-yl)(methyl)amino]carbonyl}amino)pyridin-4-yl]oxy}phenyl)-N'-(4-fluorophenyl)cyclopropane-1,1-dicarboxamide,
(3) N-{2-fluoro-4-[(2-{[(4-methyl-1,4-diazepan-1-yl)carbonyl]amino}pyridin-4-yl)oxy]phenyl}-N'-(4-fluorophenyl)cyclopropane-1,1-dicarboxamide,
(4) N-(4-fluorophenyl)-N'-{2-fluoro-4-[(2-{[(3-pyrrolidin-1-ylazetidin-1-yl)carbonyl]amino}pyridin-4-yl)oxy]phenyl}cycopropane-1,1-dicarboxamide,
(5) N-{2-fluoro-4-[(2-{[(4-methylpiperazin-1-yl)carbonyl]amino}pyridin-4-yl)oxy]phenyl}-N'-(4-fluorophenyl)cyclopropane-1,1-dicarboxamide,
(6) N-[4-({2-[({4-[2-(dimethylamino)ethyl]-1,4-diazepan-1-yl}carbonyl)ammo]pyridin-4-yl}oxy)-2-fluorophenyl]-N'-phenylcyclopropane-1,1-dicarboxamide,
(7) N-(4-{[2-({[3-(dimethylamino)azetidin-1yl]carbonyl}amino)pyridin-4-yl]oxy}-2-fluorophenyl)-N'-(4-fluorophenyl)cyclopropane-1,1-dicarboxamide,
(8) N-(4-{[2-({[3-(dimethylamino)azetidin-1-yl]carbonyl}amino)pyridin-4-yl]oxy}phenyl)-N'-(4-fluorophenyl)cycopropane-1,1-dicarboxamide,
(9) N-(4-{[2-({[3-(dimethylamino)azetidin-1-yl]carbonyl}amino)pyridin-4-yl]oxy}-2-fluorophenyl)-N'-phenylcycopropane-1,1-dicarboxamide,
(10) N-[2-fluoro-4-({2-[({methyl[1-(1-methylazetidin-3-yl)piperidin-4-yl]amino}carbonyl)amino]pyridin-4-yl}oxy)phenyl]-N'-phenylcyclopropane-1,1-dicarboxamide,
(11) N-(2-fluoro-4-{[2-({[4-(1-methylazetidin-3-yl)piperazin-1-yl]carbonyl}amino)pyridin-4-yl]oxy}phenyl)-N'-phenylcyclopropane-1,1-dicarboxamide,
(12) N-(4-fluorophenyl)-N'-(4-{[2-({[4-(1-methylazetidin-3-yl)piperazin-1-yl]carbonyl}amino)pyridin-4-yl]oxy}phenyl)cyclopropane-1,1-dicarboxamide,
(13) N-(2-fluoro-4-{[2-({[(1-methylpiperidin-4-yl)amino]carbonyl}amino)pyridin-4-yl]oxy}phenyl)-N'-(4-fluorophenyl)cycloropane-1,1-dicarboxamide,
(14) N-{2-fluoro-4-[(2-{[(4-hydroxy-1,4'-bipiperidin-1'-yl)carbonyl]amino}pyridin-4-yl)oxy]phenyl}-N'-phenylcyclopropane-1,1-dicarboxamide,
(15) N-(4-{[2-({[{1-[3-(dimethylamino)propyl]piperidin-4-yl}(methyl)amino]carbonyl}amino)pyridin-4-yl]oxy}-2-fluorophenyl)-N'-phenylcyclopropane-1,1-dicarboxamide,
(16) N-(4-{[2-({[(3-azetidin-1-ylpropyl)(methyl)amino]carbonyl}amino)pyridin-4-yl]oxy}-2-fluorophenyl)-N'-(4-fluorophenyl)cyclopropane-1,1-dicarboxamide,
(17) N-(2-fluoro-4-{[2-({[methyl(3-pyrrolidin-1-ylpropyl)amino]carbonyl}amino)pyridin-4-yl]oxy}phenyl)-N'-(4-fluorophenyl)cyclopropane-1,1-dicarboxamide,
(18) N-(4-{[2-({[[3-(dimethylamino)propyl](methyl)amino]carbonyl}amino)pyridin-4-yl]oxy}-2-fluorophenyl)-N'-(4-fluorophenyl)cyclopropane-1,1-dicarboxamide,
(19) N-(2-fluoro-4-{[2-({[methyl(4-pyrrolidin-1-ylbutyl)amino]carbonyl}amino)pyridin-4-yl]oxy}phenyl)-N'-phenylcyclopropane-1,1-dicarboxamide,
(20) N-[2-fluoro-4-({2-[(morpholin-4-ylcarbonyl)amino]pyridin-4-yl}oxy)phenyl]-N'-(4-fluorophenyl)cycopropane-1,1-dicarboxamide,
(21) N-[4-({2-[(azetidin-1-ylcarbonyl)amino]pyridin-4-yl}oxy)-2-fluorophenyl]-N'-(4-fluorophenyl)cyclopropane-1,1-dicarboxamide,
(22) N-(2-fluoro-4-{[2-({[methyl(3-morpholin-4-ylpropyl)amino]carbonyl}amino)pyridin-4-yl]oxy}phenyl)-N'-(4-fluorophenyl)cyclopropane-1,1-dicarboxamide,
(23) N-[2-fluoro-4-({2-[({methyl[3-(4-methylpiperazin-1-yl)propyl]amino}carbonyl)amino]pyridin-4-yl}oxy)phenyl]-N'-(4-fluorophenyl)cyclopropane-1,1-dicarboxamide,
(24) N-(4-fluorophenyl)-N'-[2-fluoro-4-({2-[(pyrrolidin-1-ylcarbonyl)amino]pyridin-4-yl}oxy)phenyl]cyclopropane-1,1-dicarboxamide,
(25) N-(2-fluoro-4-{[2-({[methyl(1-methylpiperidin-4-yl)amino]carbonyl}amino)pyridin-4-yl]oxy}phenyl)-N'-2-thienylcyclopropane-1,1-dicarboxamide,
(26) N-(2-fluoro-4-{[2-({[methyl(1-methylpiperidin-4-yl)amino]carbonyl}amino)pyridin-4-yl]oxy}phenyl)-N'-1,3-thiazol-2-ylcyclopropane-1,1-dicarboxamide,
(27) N-(2-fluoro-4-{[2-({[methyl(1-methylpiperidin-4-yl)amino]carbonyl}amino)pyridin-4-yl]oxy}phenyl)-N'-(5-methylisoxazol-3-yl)cyclopropane-1,1-dicarboxamide,
(28) N-(2-fluoro-4-{[2-({[methyl(1-methylpiperidin-4-yl)amino]carbonyl}amino)pyridin-4-yl]oxy}phenyl)-N'-(3-methylisoxazol-5-y)cyclopropane-1,1-dicarboxamide,
(29) N-{2-fluoro-4-[(2-{[(4-hydroxypiperidin-1-yl)carbonyl]amino}pyridin-4-yl)oxy]phenyl}-N'-(4-fluorophenyl)cyclopropane-1,1-dicarboxamide,
(30) N-{2-fluoro-4-[(2-{[(4-methoxypiperidin-1-yl)carbonyl]ammo}pyridin-4-yl)oxy]phenyl}-N'-(4-fluorophenyl)cyclopropane-1,1-dicarboxamide,
(31) N-{2-fluoro-4-[(2-{[(3-hydroxyazetidin-1-yl)carbonyl]amino}pyridin-4-yl)oxy]phenyl}-N'-(4-fluorophenyl)cyclopropane-1,1-dicarboxamide,
(32) N-{2-fluoro-4-[(2-{[(3-methoxyaxetidin-1-yl)carbonyl]amino}pyridin-4-yl)oxy]phenyl}-N'-(4-fluorophenyl)cyclopropane-1,1-dicarboxamide,
(33) N-(2-fluoro-4-{[2-({[(2-methoxyethyl)(methyl)amino]carbonyl}amino)pyridin-4-yl]oxy}phenyl)-N'-(4-fluorophenyl)cyclopropane-1,1-dicarboxamide,
(34) N-(2-fluoro-4-{[2-({[4-(3-hydroxyazetidin-1-yl)piperidin-1-yl]carbonyl}amino)pyridin-4-yl]oxy}phenyl)-N'-(4-fluorophenyl)cyclopropane-1,1-dicarboxamide,
(35) N-(2-fluoro-4-{[2-({[methyl(tetrahydro-2H-pyran-4-yl)amino]carbonyl}amino)pyridin-4-yl]oxy}phenyl)-N'-(4-fluorophenyl)cyclopropane-1,1-dicarboxamide,
(36) N-(2-fluoro-4-{[2-({[methyl(1-methylpiperidin-3-yl)amino]carbonyl}amino)pyridin-4-yl]oxy}phenyl)-N'-(4-fluorophenyl)cyclopropane-1,1-dicarboxamide,
(37) N-[4-({2-[({3-[(dimethylamino)methyl]piperidin-1-yl}carbonyl)amino]pyridin-4-yl}oxy)phenyl]-N'-(4-fluorophenyl)cycopropane-1,1-dicarboxamide,
(38) N-[4-({2-[({3-[(dimethylamino)methyl]pyrrolidin-1-yl}carbonyl)amino]pyridin-4-y}oxy)-2-fluorophenyl]-N'-(4-fluorophenyl)cyclopropane-1,1-dicarboxamide,
(39) N-(2-fluoro-4-{[2-({[methyl(1-methylpyrrolidin-3-yl)amino]carbonyl}amino)pyridin-4-yl]oxy}phenyl)-N'-(4-fluorophenyl)cyclopropane-1,1-dicarboxamide,
(40) N-{2-fluoro-4-[(2-{[(3-hydroxypyrrolidin-1-yl)carbonyl]amino}pyridin-4-yl)oxy]phenyl}-N'-(4-fluorophenyl)cyclopropane-1,1-dicarboxamide,
(41) N-{2-fluoro-4-[(2-{[(3-methoxypyrrolidin-1-yl)carbonyl]amino}pyridin-4-yl)oxy]phenyl}-N'-(4-fluorophenyl)cyclopropane-1,1-dicarboxamide,
(42) N-{4-[(2-{[(3,4-dihydroxypyrrolidin-1-yl)carbonyl]amino}pyridin-4-yl)oxy]-2-fluorophenyl}-N'-(4-fluorophenyl)cyclopropane-1,1-dicarboxamide,
(43) N-{2-fluoro-4-[(2-{[(3-hydroxy-4-methoxypyrrolidin-1-yl)carbonyl]amino}pyridin-4-yl)oxy]phenyl}-N'-(4-fluorophenyl)cyclopropane-1,1-dicarboxamide,
(44) N-{4-[(2-{[(3,4-dimethoxypyrrolidin-1-yl)carbonyl]amino}pyridin-4-yl)oxy]-2-fluorophenyl}-N'-(4-fluorophenyl)cyclopropane-1,1-dicarboxamide,
(45) N-{2-fluoro-4-[(2-{[(3-hydroxypiperidin-1-yl)carbonyl]amino} pyridin-4-yl)oxy]phenyl}-N'-(4-fluorophenyl)cyclopropane-1,1-dicarboxamide,
(46) N-{2-fluoro-4-[(2-{[(3-methoxypiperidin-1-yl)carbonyl]amino}pyridin-4-yl)oxy]phenyl}-N'-(4-fluorophenyl)cyclopropane-1,1-dicarboxamide,
(47) N-(4-{[2-({[3-(dimethylamino)piperidin-I-yl]carbonyl}amino)pyridin-4-yl]oxy}-2-fluorophenyl)-N'-(4-fluorophenyl)cyclopropane-1,1-dicarboxamide

The more preferable compound of the formula (I) includes the compounds illustrated below;
(1) N-[4-({2-[({4-[2-(Dimethylamino)ethyl]piperazin-1-yl}carbonyl)amino]pyridin-4-yl}oxy)-2-fluorophenyl]-N'-(4-fluorophenyl)cyclopropane-1,1-dicarboxamide,
(2) N-(2-Fluoro-4-{[2-({[methyl(1-methylpiperidin-4-yl)amino]carbonyl}amino)pyridin-4-yl]oxy}phenyl)-N'-(4-fluorophenyl)cyclopropane-1,1-dicarboxamide,
(3) N-(4-Fluorophenyl)-N'-{2-fluoro-4-[(2-{[(4-pyrrolidin-1-ylpiperidin-1-y1)carbonyl]amino}pyridin-4-yl)oxy]phenyl}cyclopropane-1,1-dicarboxamide,
(4) N-[4-({2-[({4-[(Dimethylamino)methyl]piperidin-1-yl}carbonyl)amino]pyridin-4-yl}oxy)-2-fluorophenyl]-N'-(4-fluorophenyl)cyclopropane-1,1-dicarboxamide,
(5) N-{4-[(2-{[(4-Azetidin-1-ylpiperidin-1-yl)carbonyl]amino}pyridin-4-yl)oxy]-2-fluoropheny}-N'-(4-fluorophenyl)cyclopropane-1,1-dicarboxamide,
(6) N-[4-({2-[({4-[3-(Dimethylamino)azetidin-1-yl]piperidin-1-yl}carbonyl)amino]pyridin-4-yl}oxy)-2-fluorophenyl]-N'-(4-fluorophenyl)cyclopropane-1,1-dicarboxamide,
(7) N-(2-Fluoro-4-{[2-({[4-(4-methylpiperazin-1-yl)piperidin-1-yl]carbonyl}amino)pyridin-4-yl]oxy}phenyl)-N'-(4-fluorophenyl)cyclopropane-1,1-dicarboxamide,
(8) N-(2-Fluoro-4-{[2-({[4-(1-methylpiperidin-4-yl)piperazin-1-yl]carbonyl}amino)pyridin-4-yl]oxy}phenyl)-N'-(4-fluorophenyl)cyclopropane-1,1-dicarboxamide,
(9) N-(2-Fluoro-4-{[2-({[4-(1-methylazetidin-3-yl)piperazin-1-yl]carbonyl}amino)pyridin-4-yl]oxy}phenyl)-N'-(4-fluorophenyl)cyclopropane-1,1-dicarboxamide,
(10) N-(4-{[2-({[4-(Dimethylamino)piperidin-1-yl]carbonyl}amino)pyridin-4-yl]oxy}-2-fluorophenyl)-N'-(4-fluorophenyl)cyclopropane-1,1-dicarboxamide,
(11) N-(4-{[2-({[4-(Azetidin-1-ylmethyl)piperidin-1-yl]carbonyl}amino)pyridin-4-yl]oxy}-2-fluorophenyl)-N'-(4-fluorophenyl)cycopropane-1,1-dicarboxamide,
(12) N-(4-Fluorophenyl)-N'-(2-fluoro-4-{[2-({[4-(pyrrolidin-1-ylmethyl)piperidin-1-yl]carbonyl}amino)pyridin-4-yl]oxy}phenyl)cyclopropane-1,1-dicarboxamide,
(13) N-(4-{[2-({[(3S)-3-(Dimethylamino)pyrrolidin-1-yl]carbonyl}amino)pyridin-4-yl]oxy}-2-fluorophenyl)-N'-(4-fluorophenyl)cycopropane-1,1-dicarboxamide,
(14) N-(4-{[2-({[(3R)-3-(Dimethylamino)pyrrolidin-1-yl]carbonyl}amino)pyridin-4-yl]oxy}-2-fluorophenyl)-N'-(4-fluorophenyl)cyclopropane-1,1-dicarboxamide,
(15) N-(2-Fluoro-4-{[2-({[methyl(1-methylpiperidin-4-yl)amino]carbonyl}amino)pyridin-4-yl]oxy}phenyl)-N'-phenylcyclopropane-1,1-dicarboxamide,
(16) N-(2-Fluoro-4-{[2-({[4-(4-methylpiperazin-1-yl)piperidin-1-yl]carbonyl}amino)pyridin-4-yl]oxy}phenyl)-N'-phenylcyclopropane-1,1-dicarboxamide,
(17) N-[4-({2-[({4-[3-(Dimethylamino)azetidin-1-yl]piperidin-1-yl}carbonyl)amino]pyridin-4-yl}oxy)-2-fluorophenyl]-N'-phenylcyclopropane-1,1-dicarboxamide,
(18) N-(4-{[2-({[(1-Ethylpiperidin-4-yl)(methyl)amino]carbonyl}amino)pyridin-4-yl]oxy}-2-fluorophenyl)-N'-phenylcyclopropane-1,1-dicarboxamide,
(19) N-[4-({2-[(Azetidin-1-ylcarbonyl)amino]-4-yl}oxy)-2-fluorophenyl]-N'-(4-fluorophenyl)cyclopropane-1,1-dicarboxamide,
(20) N-(4-Fluorophenyl)-N'-[2-fluoro-4-({2-[(pyrrolidin-1-ylcarbonyl)amino]pyridin-4-yl}oxy)phenyl]cyclopropane-1,1-dicarboxamide,
(21) N-{2-Fluoro-4-[(2-{[(3-hydroxyazetidin-1-yl)carbonyl]amino}pyridin-4-yl)oxy]phenyl}-N'-(4-fluorophenyl)cyclopropane-1,1-dicarboxamide,
(22) N-[4-({2-[(1,3'-Biazetidin-1'-ylcarbonyl)amino]pyridin-4-yl}oxy)-2-fluorophenyl]-N'-(4-fluorophenyl)cyclopropane-1,1-dicarboxamide,
(23) N-(2-Fluoro-4-{[2-({[3-(hydroxymethyl)azetidin-1-yl]carbonyl}amino)pyridin-4-yl]oxy}phenyl)-N'-(4-fluorophenyl)cyclopropane-1,1-dicarboxamide,
(24) N-(4-{[2-({[3(Dimethylamino)azetidin-1-yl]carbonyl}amino)pyridin-4-yl]oxy}-2-fluorophenyl)-N'-(4-fluorophenyl)cyclopropane-1,1-dicarboxamide,
(25) N-[4-({2-[({3-[(Dimethylamino)methyl]azetidin-1-yl}carbonyl)amino]pyridin-4-yl}oxy)-2-fluorophenyl]-N'-(4-fluorophenyl)cyclopropane-1,1-dicarboxamide,
(26) N-{2-Fluoro-4-[(2-{[(4-hydroxypiperidin-1-yl)carbonyl]amino}pyridin-4-yl)oxy]phenyl}-N'-(4-fluorophenyl)cyclopropane-1,1-dicarboxamide,
(27) N-(2-Fluoro-4-{[2-({[4-(hydroxymethyl)piperidin-1-y1]carbonyl}amino)pyridin-4-yl]oxy}phenyl)-N'-(4-fluorophenyl)cyclopropane-1, 1-dicarboxamide,
(28) N-(2-Fluoro-4-{[2-({[(3R)-3-hydroxypyrrolidin-1-yl]carbonyl}amino)pyridin-4-yl]oxy}phenyl)-N'-(4-fluorophenyl)cyclopropane-1,1-dicarboxamide,
(29) N-(2-Fluoro-4-{[2-({[(3S)-3-hydroxypyrrolidin-1-yl]carbonyl}amino)pyridin-4-yl]oxy}phenyl)-N'-(4-fluorophenyl)cyclopropane-1,1-dicarbxamide,
(30) N-[4-({2-[(Azetidin-1-ylcarbonyl)amino]pyridin-4-yl}oxy)-2,5-difluorophenyl]-N'-(4-fluorophenyl)cyclopropane-1,1-dicarboxmide,
(31) N-{2,5-Difluoro-4-[(2-{[(3-hydroxyazetidin-1-yl)carbonyl]amino}pyridin-4-yl)oxy]phenyl}-N'-(4-fluorophenyl)cyclopropane-1,1-dicarboxamide,
(32) N-(2,5-Difluoro-4-{[2-({[4-(4-methylpiperazin-1-yl)piperidin-1-yl]carbonyl}amino)pyridin-4-yl]oxy}phenyl)-N'-(4-fluorophenyl)cyclopropane-1,1-dicarboxamide,
(33) N-[2,5-Difluoro-4-({2-[({3-[(dimethylamino)methyl]azetidin-1-yl}carbonyl)amino]pyridin-4-yl}oxy)phenyl]-N'-(4-fluorophenyl)cycopropane-1,1-dicarboxamide,
(34) N-(2,5-Difluoro-4-{[2-({[methyl(1-methylpiperidin-4-yl)amino]carbonyl}amino)pyridin-4-yl]oxy}phenyl)-N'-(4-fluorophenyl)cyclopropane-1,1-dicarboxamide,
(35) N-{4-[(2-{[3-(Azetidin-1-ylmethyl)azetidin-1-ylcarbonyl]amino}pyridin-4-yl)oxy]-2,5-difluorophenyl}-N'-(4-fluorophenyl)cyclopropane-1,1-dicarboxamide,
(36) N-(2,5-Difluoro-4-{[2-({[3-(hydroxymethyl)azetidin-1-yl]carbonyl}amino)pyridin-4-yl]oxy}phenyl)-N'-(4-fluorophenyl)cyclopropane-1,1-dicarboxamide,
(37) N-{2,5-Difluoro-4-[(4-{[(3-hydroxyazetidin-1-yl)carbonyl]amino}pyrimidin-6-yl)oxy]phenyl}-N'-(4-fluorophenyl)cycopropane-1,1-dicarboxamide,
(38) N-[4-({4[({3[(Dimethylamino)methyl]azetidin-1-yl}carbonyl)amino]pyrimidin-6-yl}oxy)-2,5-difuorophenyl]-N'-(4-fluorophenyl)cyclopropane-1,1-dicarboxamide,
(39) N-(2,5-Difluoro-4-{[4-({[3-(hydroxymethyl)azetidin-1-yl]carbonyl}amino)pyrimidin-6-yl]oxy}phenyl)-N'-(4-fluorophenyl)cyclopropane-1,1-dicarboxamide,
(40) N-(2,5-Difluoro-4-{[4-({[methyl(1-methylpiperidin-4-yl)amino]carbonyl}amino)pyrimidin-6-yl]oxy}phenyl)-N'-(4-fluorophenyl)cyclopropane-1,1-dicarboxamide,
(41) N-(2,5-Difluoro-4-{[4-({[4-(4-methylpiperazin-1-yl)piperidin-1-yl]carbonyl}amino)pyrimidin-6-yl]oxy}phenyl)-N'-(4-fluorophenyl)cyclopropane-1,1-dicarboxamide,
(42) N-(4-{[2-({[4-(Dimethylamino)piperidin-1-yl]carbonyl}amino)pyridin-4-yl]oxy}-2,5-difluorophenyl)-N'-(4-fluorophenyl)cyclopropane-1,-dicarboxamide,
(43) N-{2,5-Difluoro-4-[(2-{[(4-methylpiperazin-1-yl)carbonyl]amino}pyridin-4-yl]oxy}phenyl)-N'-(4-fluorophenyl)cyclopropane-1,1-dicarboxamide,
(44) N-{2,5-Difluoro-4-[(2-{[(4-hydroxypiperidin-1-yl)carbonyl]amino}pyridin-4-yl)oxy]phenyl}-N'-(4-fluorophenyl)cyclopropane-1,1-dicarboxamide,
(45) N-{4-[(2-{[(4-Azetidin-1-ylpiperidin-1-yl)carbonyl]amino}pyridin-4-yl)oxy]oxy}-2,5-difluorophenyl}-N'-(4-fluorophenyl)cyclopropane-1,1-dicarboxamide,
(46) N-(2,5-Difluoro-4-{[2-({[3-(2-dimethylaminoacetoxy)azetidin-1-yl]carbonyl}amino)pyridin-4-yl]oxy}phenyl)-N'-(4-fluorophenyl)cyclopropane-1,1-dicarboxamide,
(47) N-(2,5-Difluoro-4-{[2-{[(3S)-3-hydroxypyrrolidin-1-yl]carbonyl}amino)pyridin-4-yl]oxy}phenyl)-N'-(4-fluorophenyl)cyclopropane-1,1-dicarboxamide,
(48) N-(2,5-Difluoro-4-{[2-({[(3R)-3-hydroxypyrrolidin-1-yl]carbonyl}amino)pyridin-4-yl]oxy}phenyl)-N'-(4-fluorophenyl)cyclopropane-1,1-dicarboxamide or
(49) N-(3-Fluoro-4-{[6-({[methyl(1-methylpiperidin-4-yl)amino]carbonyl}amino)pprimidin-4-yl]oxy}phenyl)-N'-(4-fluorophenyl)cyclopropane-1,1-dicarboxamide

The phrase "may be substituted with a substituent selected from Substituent Group" or "optionally substituted with a substituent selected from Substituent Group" means "may be substituted with 1 to 3 substituents selected arbitrarily from the substituents described in the Substituent Group."

### (General production method)

The pyridine or pyrimidine derivative of the present invention can be produced by methods described below. But the method for producing the pyridine or pyrimidine derivative of the present invention is not limited to these methods.

[Production method 1] A method for producing intermediates (1m) and (In) [Production method 1-A] A method for producing intermediates (1m) and (In) via coupling of a derivative of 2-aminopyridine or 6-aminopyrimidine with phenol 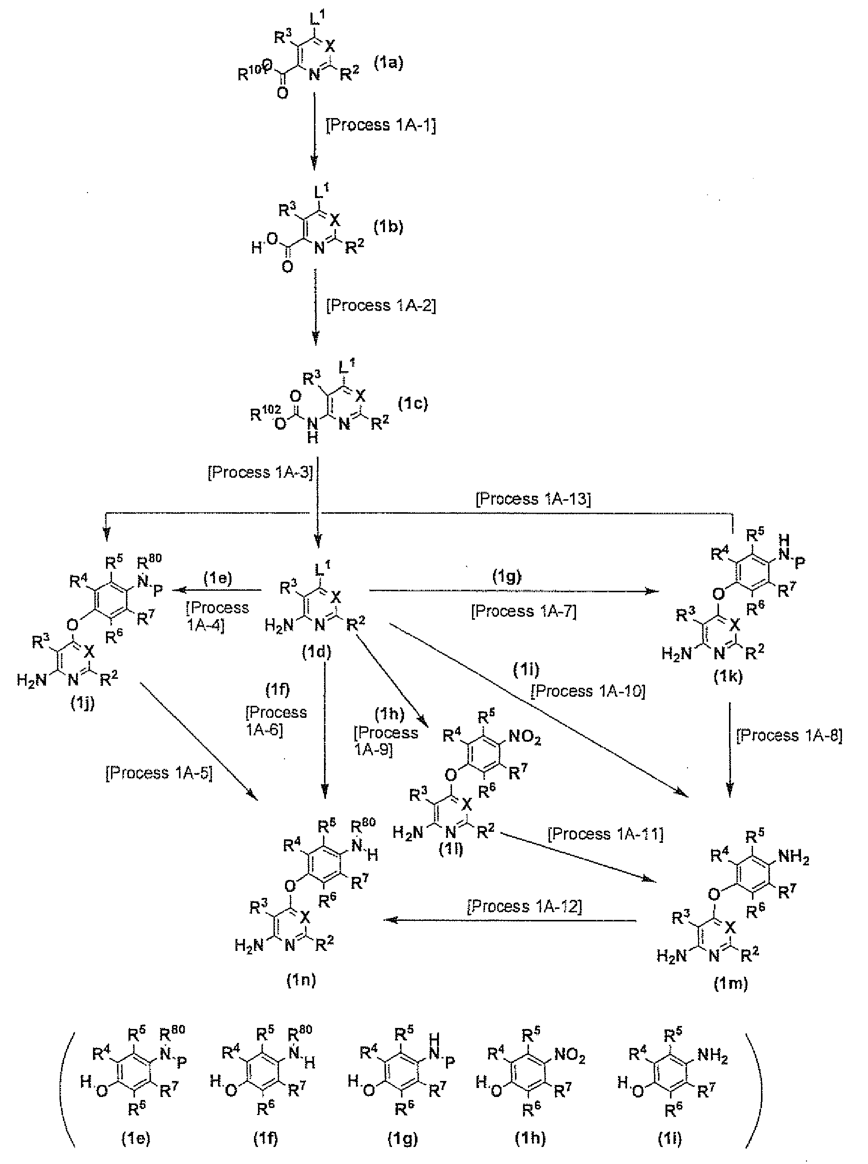 In the scheme, L¹ represents a leaving group; R¹⁰¹ represents C₁₋₆ alkyl or benzyl; R¹⁰² represents C₁₋₆ alkyl, benzyl or 2-(trimethylsilyl)ethyl; R⁸⁰ represents C₁₋₆ alkyl; P represents a protecting group for amino; and the other symbols represent the same meaning as defined above.
The compound (1a) includes, for example, 4-nitropicolinic acid ester, 4-chloropicolinic acid ester, 6-chloropyrimidine-4-carboxylic acid ester. 4-nitropicolinic acid ester and 4-chloropicolinic acid ester can be obtained by the esterification of 4-nitropicolinic acid and 4-chloropicolinic acid, both of which are commercially available. Among 6-chloropyrimidine-4-carboxylic acid ester, methyl 6-chloropyrimidine-4-carboxylate is described in Ukr. Kihm. Zh., 1982, Vol.48, p 67 (CAS No. 6627-22-1). 6-chloropyrimidine-4-carboxylic acid ester also can be produced according to a method described in J. Heterocycl. Chem., 1, 130 (1964).
The compound (1d) includes, for example, commercially available compounds such as 2-amino-4-chloropyridine and 4-amino-6-chloropyrimidine. The compound (1d) also can be produced via <Process 1A-1>, <Process 1A-2> and <Process 1A-3> described below, using the compound (1a) as a starting material.
The compound (1f) includes, for example, commercially available compounds such as p-methylaminophenol sulfate.
The compound (1e) can be obtained by protecting a group represented by the formula R⁸⁰NH- of the compound (1f). The general reaction for protecting amino can be used. For example, the compound (1e) can be obtained by a reaction of the compound (1f) with ethyl chloroformate, methyl chloroformate, benzyl chloroformate, di-t-butyl dicarbonate or trifluoroacetic anhydride.
The compound (1g) includes, for example, commercially available compounds such as 4-acetoamidophenol, N-(4-hydroxyphenyl)formamide, 4-(N-t-butoxycarbonylamino)phenol and 4-trifluoroacetoamidophenol.
The compound (1h) includes, for example, commercially available compounds such as 4-nitrophenol, 2-chloro-4-nitrophenol, 2-fluoro-4-nitrophenol, 3-fluoro-4-nitrophenol and 3-methyl-4-nitrophenol.
The compound (1i) includes, for example, commercially available compounds such as 4-aminophenol, 4-amino-3-chlorophenol hydrochloride, 4-amino-2,5-dimethylphenol, 4-amino-2,6-dichlorophenol and 5-amino-2-hydroxybenzonitrile.
The above compounds can also be produced from commercially available compounds by a known method.

### <Process 1A-1>

The process is a process for producing the compound (1b) from the compound (1a). For example, hydrolysis using a base can be used. As the base, an inorganic base such as sodium hydroxide, potassium hydroxide and lithium hydroxide can be used. As the solvent, methanol, ethanol, water or the like can be used. The reaction temperature is between 0 °C and a reflux temperature. The reaction time is between 10 minutes and 30 hours.

### <Process 1A-2>

The process is a process for rearrangement of the compound (1b) to the compound (1c). The compound (1c) can be obtained by a reaction of the compound (1b) with an alcohol represented by the formula R¹⁰²-OH in the presence of diphenylphosphoryl azide and triethylamine. The preferable example of R¹⁰² includes t-butyl, benzyl and 2-(trimethylsilyl)ethyl. As the solvent, N,N-dimethylformamide, N-methylpyrrolidone, toluene or the like can be used as well as t-butanol or benzylalcohol. The reaction temperature is between room temperature and a reflux temperature. The reaction time is between 10 minutes and 30 hours.

### <Process 1A-3>

The process is a process for producing the compound (1d) from the compound (1c) by deprotection of carbamate. For the reaction, general deprotection for amino can be used and specific examples are deprotection using an acid such as hydrochloric acid and trifluoroacetic acid, deprotection using an inorganic base such as sodium hydroxide and potassium hydroxide, and deprotection using tetrabutylammonium fluoride. As the solvent, methanol, ethanol, water, tetrahydrofuran, N,N-dimethylformamide or the like can be used. The reaction temperature is between room temperature and a reflux temperature. The reaction time is between 10 minutes and 30 hours.

### <Process 1A-4> <Process 1A-6> <Process 1A-7> <Process 1A-9> <Process 1A-10>

These processes are processes for coupling the compound (1d) with the compounds (1e), (1f), (1g), (1h) or (1i) to produce the compounds (1j), (In), (1k), (1l) or (1m), respectively. As the solvent, N-methylpyrrolidone, N,N-dimethylformamide, dimethyl sulfoxide, 2-ethoxyethanol, chlorobenzene or the like can be used. A base or an acid may be added in the reaction system, and specifically an organic base such as triethylamine and diisopropylethylamine, an inorganic base such as potassium carbonate, cesium carbonate and sodium hydride, or an acid such as pyridine hydrochloride and hydrochloric acid can be used. The reaction temperature is between room temperature and a reflux temperature. The reaction time is between 10 minutes and 30 hours.

### <Process 1A-5>

The process is a process for deprotecting the compound (1j) to produce the compound (In). For the reaction, general deprotection for amino can be applied, for specific example, deprotection using an acid such as hydrochloric acid and trifluoroacetic acid, deprotection using an inorganic base such as sodium hydroxide and potassium hydroxide, and deprotection using tetrabutylammonium fluoride. When a protecting group is benzyloxycarbonyl and R⁴, R⁵, R⁶, R⁷ and R¹⁰ are not any of chlorine, bromine and iodine, deprotection by catalytic hydrogenation using palladium-carbon or palladium hydroxide as a catalyst can also be used. As the solvent, methanol, ethanol, water, tetrahydrofuran, N,N-dimethylformamide or the like can be used. The reaction temperature is between room temperature and a reflux temperature. The reaction time is between 10 minutes and 30 hours.

### <Process 1A-8>

The process is a process for deprotecting the compound (1k) to produce the compound (1m). The conditions similar to those in <Process 1A-5> can be used.

### <Process 1A-11>

The process is a process for reducing nitro of the compound (1l) to produce the compound (1m). Generally used conditions for reduction from nitro to amino can be applied, for specific example, reduction using iron-ammonium chloride, or iron-acetic acid. When R⁴, R⁵, R⁶, R⁷ and R¹⁰ are not any of chlorine, bromine and iodine, catalytic hydrogenation using palladium hydroxide or palladium-carbon as a catalyst also can be used. As the solvent, methanol, ethanol, water, N,N-dimethylformamide, ethyl acetate, tetrahydrofuran or the like can be used. The reaction temperature is between room temperature and a reflux temperature. The reaction time is between 10 minutes and 30 hours.

### <Process 1A-12>

The process is a process for alkylating the compound (1m) to produce the compound (In). Reductive amination of aldehyde or ketone can convert hydrogen to C₁₋₆ alkyl. As the reducing agent, sodium cyanoborohydride and sodium triacetoxyborohydride can be used. As the solvent, methanol, tetrahydrofuran, dichloromethane, dichloroethane or the like can be used.
A method for reducing a benzotriazole derivative with sodium borohydride can also be used, as described in Tetrahedron, 47(16), 2683(1991). Specifically for example, the compound (In) wherein R⁸⁰ is methyl can be obtained by reduction with sodium borohydride, a benzotriazol-1-ylmethylaniline derivative obtained by a reaction of the compound (1m) with 1-(hydroxymethyl)1H-benzotriazale. In the process for producing a benzotriazol-1-ylmethylaniline derivative, an alcohol such as methanol or ethanol, or a mixed solvent of an alcohol with N,N-dimethylformamide, acetic acid or water can be used for the solvent. The reaction temperature is between -5 °C and a reflux temperature. The reaction time is between 10 minutes and 30 hours. In the process of reduction with sodium borohydride, tetrahydrofuran, dioxane, an alcohol such as methanol or ethanol, or a mixed solvent of an alcohol with N,N-dimethylformamide or the like can be used as the solvent. The reaction temperature is between -5 °C and a reflux temperature. The reaction time is between 10 minutes and 30 hours.

### <Process 1A-13>

The process is an alternative method for producing the compound (1j) by alkylating the compound (1k) to produce the compound (1j). The compound (1j) can be obtained by a reaction with alkyl halide in the presence of a base such as potassium carbonate or sodium hydride. As the solvent, tetrahydrofuran, N,N-dimethylformamide or the like can be used. The reaction temperature is between 0 °C and a reflux temperature. The reaction time is between 10 minutes and 30 hours.

[Production method 1-B] A method for producing an intermediate (1x) via coupling of pyridine-2-carboxylic acid ester or pyrimidine-6-carboxylic acid ester with a derivative of phenol 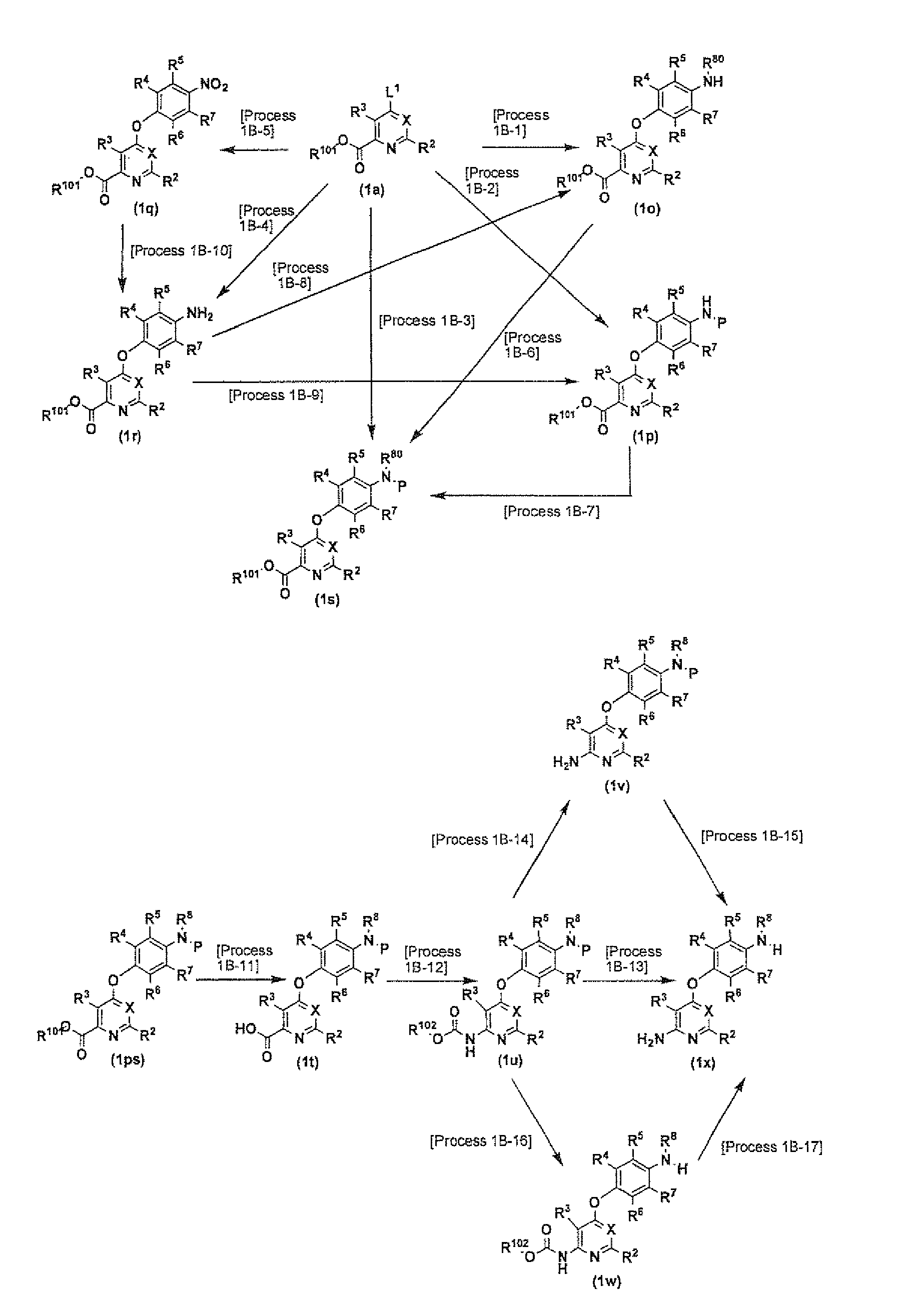 In the scheme, the symbols represent the same weaning as defined above. <Process 1B-1> <Process 1B-2> <Process 1B-3> <Process 1B-4> <Process 1B-5>
These processes are processes for coupling the compound (1a) with the compound (1f), (1g), (1e), (1i) or (1h) to produce the compound (1o), (1p), (1s), (1r) or (1q), respectively. The methods similar to those in <Process 1A-4> can be used.

### <Process 1B-6>

The process is a process for protecting amino of the compound (1o) to produce the compound (1s). A general reaction for protecting amino can be used. Specifically for example, a reaction with ethyl chloroformate, methyl chloroformate, benzyl chloroformate, di-t-butyl dicarbonate and trifluoroacetic anhydride can be used. A base may be added in the reaction system, and an organic base such as pyridine, triethylamine and diisopropylethylamine, and an inorganic base such as sodium carbonate, potassium carbonate and sodium hydrogencarbonate can be used. As the solvent, tetrahydrofuran, acetone, water, dioxane or the like can be used. The reaction temperature is between room temperature and a reflux temperature. The reaction time is between 10 minutes and 30 hours.

### <Process 1B-7>

The process is a process for alkylating the compound (1p) to produce the compound (1s). The methods similar to those in <Process 1A-13> can be used.

### <Process 1B-8>

The process is a process for alkylating the compound (1r) to produce the compound (1o). The methods similar to those in <Process 1A-12> can be used.

### <Process 1B-9>

The process is a process for protecting amino of the compound (1r) to produce the compound (1p). The methods similar to those in <Process 1B-6> can be used.

### <Process 1B-10>

The process is a process for reducing nitro of the compound (1q) to produce the compound (1r). The methods similar to those in <Process 1A-11> can be used.

### <Process 1B-11>

The process is a process for producing the compound (1t) from the compound (1ps) (the compound (1ps) represents the compound (1p) and the compound (1s) described in [Production method 1-B]). The methods similar to those in <Process 1A-1> can be used.

### <Process 1B-12>

The process is a process for producing the compound (1u) from the compound (It). The methods similar to those in <Process 1A-2> can be used.

### <Process 1B-13>

The process is a process for deprotecting the two protecting groups "R¹⁰²-O-C(=O)-" and "P" of the compound (1u) to produce the compound (1x). Depending on the kind of the protecting groups, deprotection using an acid such as hydrochloric acid and trifluoroacetic acid, deprotection using an inorganic base such as sodium hydroxide and potassium hydroxide, deprotection using tetrabutylammonium fluoride, and deprotection by catalytic hydrogenation using palladium-carbon or palladium hydroxide as a catalyst can be appropriately combined to produce the compound (1x).

### <Production 1B-14> <Production 1B-16>

These processes are processes for deprotecting only one of the two protecting groups "R¹⁰²-O-C(=O)-" and "P" of the compound (1u) to produce the compound (1v) or the compound (1w), respectively. The process is applicable only when the two protecting groups "R¹⁰²-O-C(-O)-" and "P" are different. Specifically, for example, when a group represented by the formula R¹⁰²-O-C(=O)-is 2-(trimethylsilyl)ethoxycarbonyl and P is benzyloxycarbonyl, deprotection using tetrabutylammonium fluoride or deprotection by catalytic hydrogenation can be applied to deprotect selectively only one of the two protecting groups.

### <Process 1B-15>

The process is a process for deprotecting the compound (1v) to produce the compound (1x). The method described in <Process 1A-5> can be used.

### <Process 1B-17>

The process is a process for deprotecting the compound (1w) to produce the compound (1x). The method described in <Process 1A-5> can be used.

[Production method 2] An alternative production method of intermediates (1l), (1m), (1k), (1j) and (1n) from a pyridine or pyrimidine derivative (2a) having leaving groups L¹ at the 4-position and L² at the 2-position or 6-position 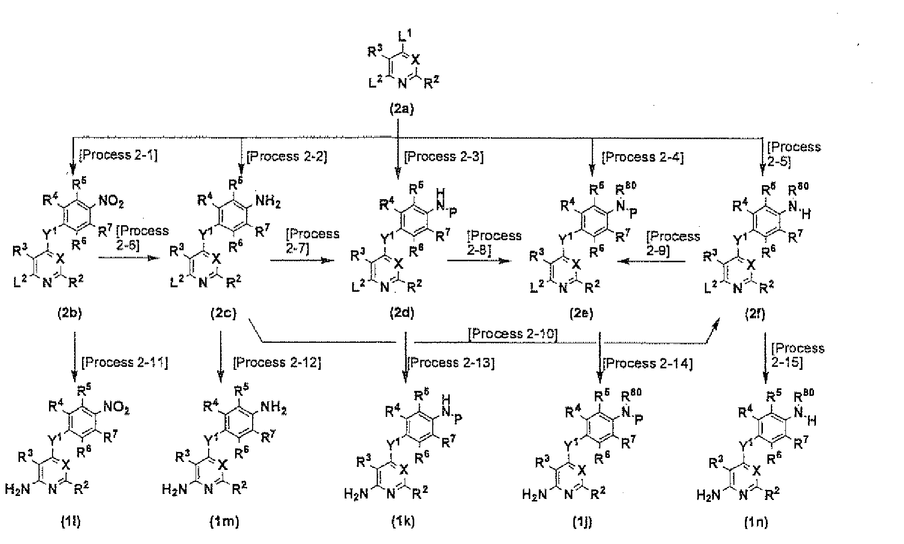 In the scheme, L² represents a leaving group. The other symbols represent the same meanings as defined above.
The compound (2a) includes, for example, commercially available compounds such as 4,6-dichloropyrimidine, 2-chloro-4-nitropyridine, and 2,4-dichloropyridine. The compound (2a) also can be produced from commercially available compounds by a known method.

### <Process 2-1> <Process 2-2> <Process 2-3> <Process 2-4> <Process 2-5>

These processes are processes for coupling the compound (2a) with the compound (1h), (1i), (1g), (1e) or (1f) to produce the compound (2b), (2c), (2d), (2e) or (2f), respectively. Preferably, in (2a), L¹ is a reactive group having higher reactivity than L². In a specific combination, for example, L¹ is nitro and L² is chlorine. The methods similar to those in <Process 1A-4> can be used for these processes.

### <Process 2-6>

The process is a process for reducing nitro of the compound (2b) to produce the compound (2c). Generally used conditions of reduction from nitro to amino can be used. Specifically, for example a reduction using iron-ammonium chloride or iron-acetic acid can be used. As the solvent, methanol, ethanol, water, N,N-dimethylformamide, tetrahydrofuran or the like can be used. The reaction temperature is between room temperature and a reflux temperature. The reaction time is between 10 minutes and 30 hours.

### <Process 2-7>

The process is a process for protecting amino of the compound (2c) to produce the compound (2d). The methods similar to those in <Process 1B-6> can be used.

### <Process 2-8>

The process is a process for alkylating the compound (2d) to produce the compound (2e). The methods similar to those in <Process 1A-13> can be used.

### <Process 2-9>

The process is a process for protecting amino of the compound (2f) to produce the compound (2e). The methods similar to those in <Process 1B-6> can be used.

### <Process 2-10>

The process is a process for alkylating the compound (2c) to produce the compound (2f). The methods similar to those in <Process 1A-12> can be used.

### <Process 2-11> <Process 2-12> <Process 2-13> <Process 2-14> <Process 2-15>

These process are processes for converting the leaving group L² of the compound (2b), (2c), (2d), (2e) or (2f) to amino to produce the compound (1l), (1m), (1k), (1j) or (1n), respectively. The process can be carried out using, for example, an ammonia-ethanol solution in a sealed tube. The reaction temperature is a reflux temperature. The reaction time is between 10 minutes and 100 hours.

[Production method 3] A method for producing an intermediate represented by the formula (XI) 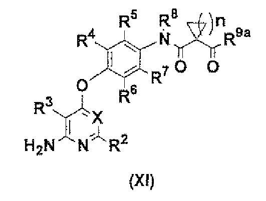 In the formula, R^{9a} represents a 3- to 10-membered non-aromatic heterocyclic group wherein the group is limited to a group having nitrogen as a ring constituent atom and the nitrogen having a bonding hand, or a group represented by the formula -NR^{11a}R^{11b}, wherein R^{11a} and R^{11b} represent the same meaning as described above. R^{9a} may be substituted with a substituent selected from Substituent Group A or Substituent Group B. Where R^{9a} has hydroxyl, primary amino or secondary amino as a substituent group, the group may be protected by a suitable protecting group. The other symbols represent the same meanings as defined above. 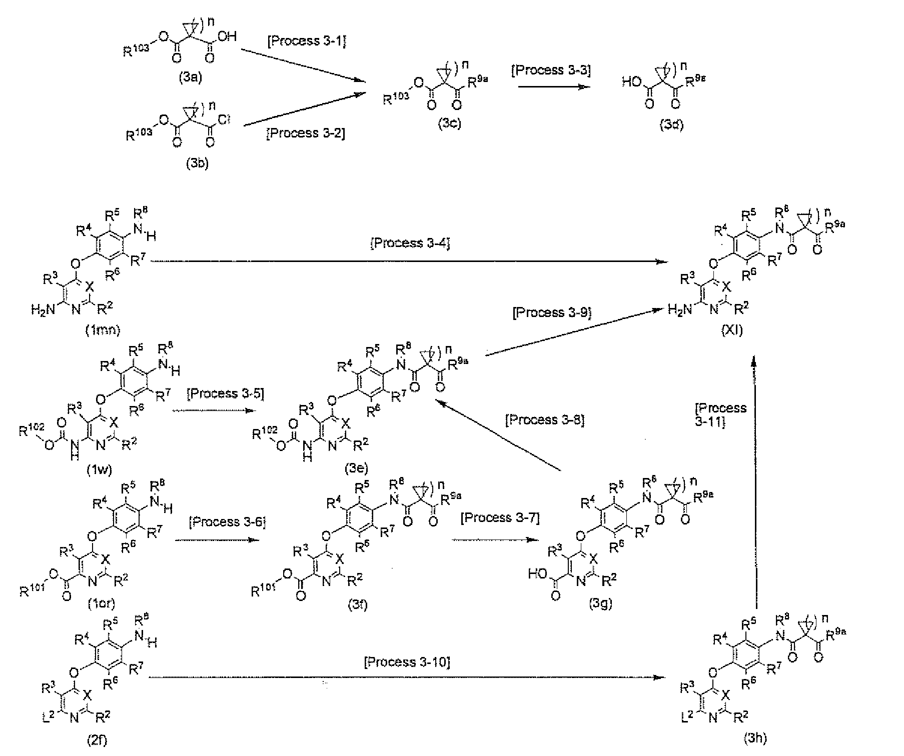 In the formula, R¹⁰³ represents C₁₋₆ alkyl or benzyl. The other symbols represent the same meanings as defined above.
The compound (3a) includes, for example, 1-ethoxycarbonylcyclopropanecarboxylic acid, 1-methoxycarbonylcyclopropanecarboxylic acid, 1-benzyloxycarbonylcyclobutanecarboxylic acid and 1-ethoxycarbonylcyclobutanecarboxylic acid.
The compound (3b) includes, for example, 1-chlorocarbonylcyclopropanecarboxylic acid ethyl ester and 1-chlorocarbonylcyclobutanecarboxylic acid ethyl ester.
The above compounds can also be produced from commercially available compounds by a known method.

### <Process 3-1>

The process is a process for condensing the compound (3a) with an amine represented by the formula R^{9a}-H or a salt thereof to produce the compound (3c). For the process, a general condensation of a carboxylic acid with an amine can be used. For specific example, as the solvent, N,N-dimethylformamide and tetrahydrofuran can be used, and for the condensing agent, carbonyldiimidazole, dicyclohexylcarbodiimide, 1-ethyl-3-(3-dimethylaminopropyl)carbodiimide hydrochloride, and (1H-1,2,3-benzotriazol-1-yloxy)(tri(dimethylamino))phosphonium hexafluorophosphate can be used. An organic base such as triethylamine also can be appropriately used. The reaction temperature is between 0 °C and a reflux temperature. The reaction time is between 10 minutes and 30 hours.

### <Process 3-2>

The process is a process for condensing the compound (3b) with an amine represented by the formula R^{9a}-H or a salt thereof to produce the compound (3c). As the solvent, N,N-dimethylformamide, tetrahydrofuran, dichloromethane or the like can be used. An organic base such as triethylamine also can be appropriately used. The reaction temperature is between 0 °C and a reflux temperature. The reaction time is between 10 minutes and 30 hours.

### <Process 3-3>

The process is a process for producing the compound (3d) from the compound (3c). For the process, hydrolysis using a base can be used. For the base, lithium hydroxide or the like can be used. If R¹⁰³ is benzyl and R^{9a} does not have chlorine, bromine and iodine as a substituent group, catalytic hydrogenation using palladium-carbon or palladium hydroxide as a catalyst also can be used. As the solvent, methanol, ethanol, water, N,N-dimethylformamide, tetrahydrofuran, ethyl acetate or the like can be used. The reaction temperature is between 0 °C and a reflux temperature. The reaction time is between 10 minutes and 30 hours.

### <Process 3-4>

The process is a process for condensing the compound (1mn) (the compound (1mn) represents the compounds (1m) and (1n) described in [Production method 1-A]) with the compound (3d) to produce the compound (XI). For the condensing agent, 1-ethyl-3-(3-dimethylaminopropyl)carbodimide hydrochloride, (1H-1,2,3-benzotriazol-1-yloxy)(tri(dimethylamino))phosphonium hexafluorophosphate or the like can be used. An organic base such as triethylamine also can be appropriately used. As the solvent, tetrahydrofuran, N,N-dimethylformamide or the like can be used. The reaction temperature is between 0 °C and a reflux temperature. The reaction time is between 10 minutes and 30 hours. <Process 3-5> <Process 3-6> <Process 3-10>

These processes are processes for producing the compounds (3e), (3f) or (3h) from the compound (1w), (1or) (the compound (1or) represents the compounds (1o) and (1r) described in [Production method 1-B], the same applies hereinafter), or (2f), respectively. The methods similar to those in <Process 3-4> can be used.

### <Process 3-7>

The process is a process for producing the compound (3g) from the compound (3f). The methods similar to those in <Process 1A-1> can be used.

### <Process 3-8>

The process is a process for rearrangement of the compound (3g) to the compound (3e). The methods similar to those in <Process 1A-2> can be used.

### <Process 3-9>

The process is a process for deprotecting the compound (3e) to produce the compound (XI). The methods similar to those in <Process 1A-5> can be used.

### <Process 3-11>

The process is a process for converting the leaving group L² of the compound (3h) to amino to produce the compound (XI). The methods similar to those in <Process 2-11> can be used.

[Production method 4] An alternative method for synthesizing various intermediates in [Production method 3] 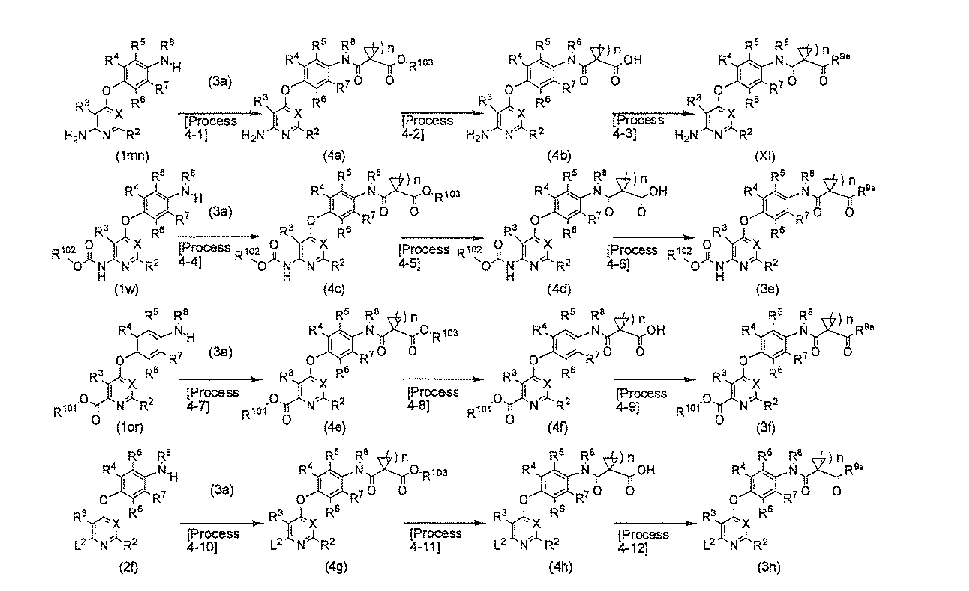 In the scheme, the symbols represent the same meanings as defined above.

### <Process 4-1> <Process 4-4> <Process 4-7> <Process 4-10>

These processes are processes for condensing the compound (1mn), (1w), (1or) or (2f) with the compound (3a) to produce the compound (4a), (4c), (4e) or (4g), respectively. The method similar to those in <Process 3-4> can be used.

### <Process 4-2> <Process 4-5> <Process 4-8> <Process 4-11>

These processes are processes for producing the compound (4b), (4d), (4f) or (4h) from the compound (4a), (4c), (4e) or (4g), respectively. The methods similar to those in <Process 1A-1> can be used. But in <Process 4-5> and <Process 4-8> deprotection is carried out under such a condition that the protecting group of amino or carboxyl at 2-position of pyridine or 4-position of pyrimidine may not be deprotected. Specifically, for example, if R¹⁰¹ or R¹⁰² is C₁₋₆ alkyl or 2-(trimethylsilyl)ethyl and R¹⁰³ is benzyl, then catalytic hydrogenation can be carried out to produce the compound (4d) or (4f).

### <Process 4-3> <Process 4-6> <Process 4-9> <Process 4-12>

These processes are processes for condensing the compound (4b), (4d), (4f) or (4h) with an amine represented by the formula R^{9a}-H or a salt thereof to produce the compound (XI), (3e), (3f) or (3h), respectively. The method similar to those in <Process 3-1> can be used.

[Production method 5] An alternative method (2) for synthesizing various intermediates in [Production method 3] 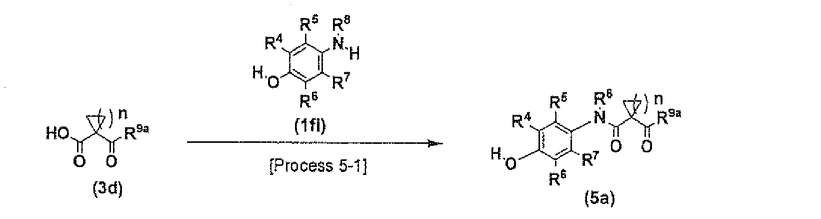 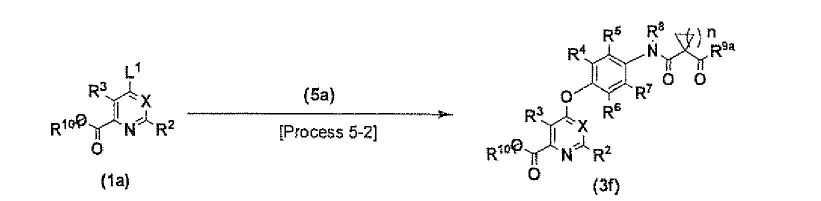 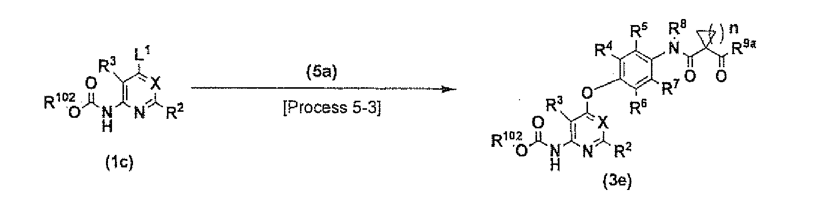 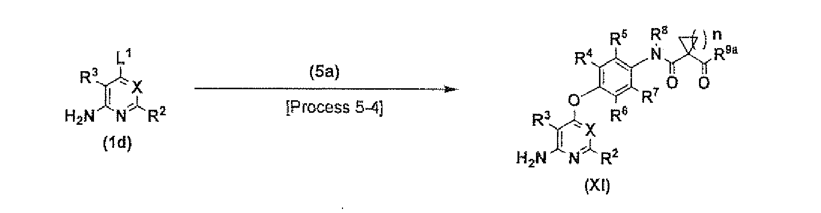 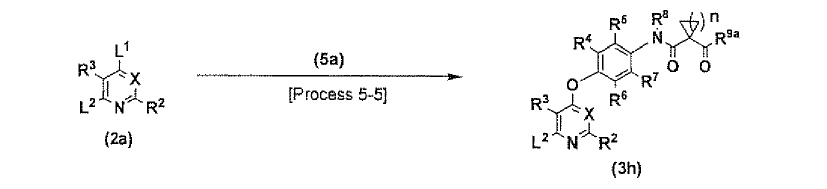 In the scheme, the symbols represent the same meanings as defined above.

### <Process 5-1>

The process is a process for condensing the compound (3d) with the compound (1fi) (the compound (1fi) represents the compounds (1f) and (1i) described in [Production method 1-A]) to produce the compound (5a). The method similar to those in <Process 3-4> can be used.

### <Process 5-2> <Process 5-3> <Process 5-4> <Process 5-5>

These processes are processes for coupling the compound (1a), (1c), (1d) or (2a) with the compound (5a) to produce the compound (3f), (3e), (XI) or (3h), respectively. The methods similar to those in <Process 1A-4> can be used.

[Production method 6] A method for producing an intermediate represented by the formula (XII) 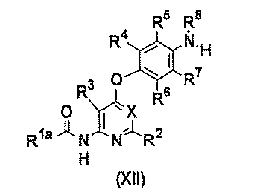 In the formula, R^{1a} represents a 3- to 10-membered non-aromatic heterocyclic group wherein the group is limited to a group having nitrogen as a ring constituent atom and the nitrogen having a bonding hand, or a group represented by the formula -NR^{11a}R^{11b}, wherein R^{11a} and R^{11b} represent the same meaning as described above. R^{1a} may be substituted with a substituent selected from Substituent Group A or Substituent Group B. Where R^{1a} has hydroxyl, primary amino or secondary amino as a substituent group, the group may be protected by a suitable protecting group. The other symbols represent the same meanings as defined above. 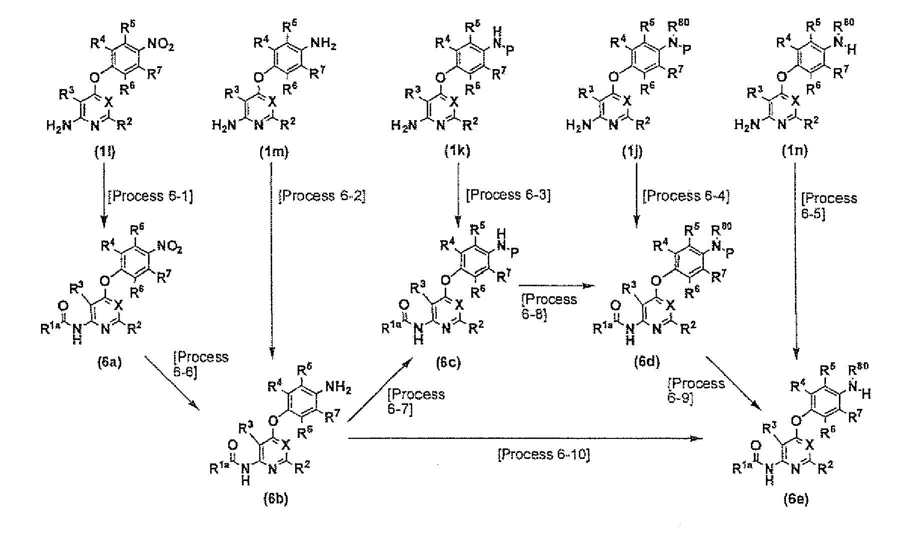 In the scheme, the symbols represent the same meanings as defined above.

### <Process 6-1> <Process 6-2> <Process 6-3> <Process 6-4> <Process 6-5>

These processes are processes for producing the compound (6a), (6b), (6c), (6d) or (6e) from the compound (11), (1m), (1k), (1j) or (In), respectively. For example, a method wherein the compound (11), (1m), (1k), (1j) or (In) is converted to a carbamic acid ester derivative using a compound represented by the formula Ar-OC(=O)-Cl, wherein Ar represents a phenyl group optionally substituted with one or two substituent(s) selected from halogen, methyl, methoxy and nitro, followed by reacting with an amine can be used. Alternatively, the compound (11), (1m), (1k), (1j) or (In) can be reacted with a carbamate derivative, an isocyanate derivative to convert to a corresponding urea derivative. As the solvent, chloroform, toluene, N-methylpyrrolidone, N,N-dimethylformamide, dimethylsulfoxide, chlorobenzene or the like can be used. A mixed solvent of the above solvent and water also can be used. A base also can be used. Specifically, an organic base such as pyridine, triethylamine and diisopropylethylamine, and an inorganic base such as potassium carbonate, cesium carbonate, sodium hydride and sodium hydroxide can be used. The reaction temperature is between 0 °C and a reflux temperature. The reaction time is between 10 minutes and 30 hours.
After the process, in order to convert substituent groups on R^{1a}, generally used reactions such as oxidation, reduction, esterification, amidation, introduction of protecting groups, deprotection and hydrolysis can also be carried out in a suitable succeeding process. Specifically, for example, the method includes a method wherein the compound (11), (1k) or (1j) is reacted with a ketone or aldehyde-containing amine, followed by reductive amination with an amine to introduce an amine side chain on R^{1a}. As the reducing agent, sodium cyanoborohydride and sodium triacetoxyborohydride or the like can be used. As the solvent, methanol, tetrahydrofuran, dichloromethane, dichloroethane or the like can be used. Furthermore, the compound (11), (1k) or (1j) can be reacted with an ester-containing amine to produce a compound, an ester portion of which is then hydrolyzed with a base such as lithium hydroxide, sodium hydroxide and potassium hydroxide in hydrous ethanol, followed by converting with a condensing agent to an amide derivative. As the solvent, N,N-dimethylformamide, tetrahydrofuran or the like can be used. As the condensing agent, 1-ethyl-3-(3-dimethylaminopropyl)carbodiimide hydrochloride and (1H-1,2,3-benzotriazol-1-yloxy)(tri(dimethylamino))phosphonium hexafluorophosphate can be used. The reaction temperature is between 0 °C and a reflux temperature. The reaction time is between 10 minutes and 30 hours.

### <Process 6-6>

The process is a process for reducing the compound (6a) to produce the compound (6b). The methods similar to those in <Process 1A-11> can be used.

### <Process 6-7>

The process is a process for protecting amino of the compound (6b) to produce the compound (6c). The methods similar to those in <Process 1B-6> can be used.

### <Process 6-8>

The process is a process for alkylating the compound (6c) to produce the compound (6d). The methods similar to those in <Process 1A-13> can be used.

### <Process 6-9>

The process is a process for deprotecting the compound (6d) to produce the compound (6e). The methods similar to those in <Process 1A-5> can be used.

### <Process 6-10>

The process is a process for alkylating the compound (6b) to produce the compound (6e). The methods similar to those in <Process 1A-12> can be used.

[Production method 7] A method for producing the compound of the present invention represented by the formula (I) 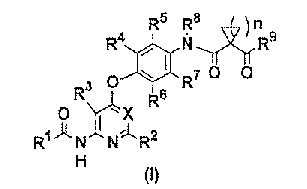 In the formula, the symbols represent the same meanings as defined above. 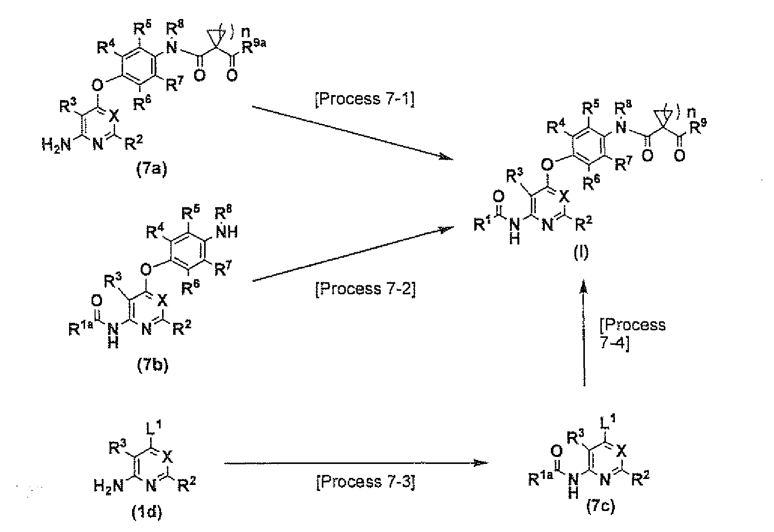 In the scheme, the symbols represent the same meanings as defined above.

### <Process 7-1>

The process is a process for producing the compound (I) of the present invention from the compound (7a), that is, the above intermediate (XI).
(1) When R^{1a} or R^{9a} does not contain hydroxyl, primary amino or secondary amino: Using a compound represented by the formula Ar-OC(=O)-Cl, wherein Ar represents the same meaning as defined above, the compound (7a) can be converted to a carbamic acid ester derivative, which is then reacted with an amine to produce the compound (I) of the present invention. Alternatively, the compound (7a) can be reacted with a carbamate derivative, an isocyanate derivative to convert to the compound (I) of the present invention. As the solvent, chloroform, toluene, N-methylpyrrolidone, N,N-dimethylformamide, dimethyl sulfoxide, chlorobenzene or the like can be used. A mixed solvent of the above solvent and water also can be used. A base also can be used, and specifically, an organic base such as pyridine, triethylamine and diisopropylethylamine, and an inorganic base such as potassium carbonate, cesium carbonate, sodium hydride and sodium hydroxide can be used. The reaction temperature is between 0 °C and a reflux temperature. The reaction time is between 10 minutes and 30 hours.
(2) When R^{1a} or R^{9a} contains hydroxyl, primary amino or secondary amino:
   After these substituents are suitably protected, the above reaction can be carried out followed by deprotecting suitably to produce the compound (I) of the present invention.
(3) After the process, in order to convert substituent groups on R^{1a} or R^{9a}, generally used reactions such as oxidation, reduction, esterification, amidation, protection, deprotection and hydrolysis can also be carried out in a suitable succeeding process, as described in <Process 6-1> of the above production method 6].

### <Process 7-2>

The process is a process for producing the compound (I) of the present invention from the compound (7b), that is, the above intermediate (XII).
(1) When R^{1a} or R^{9a} does not contain hydroxyl, primary amino or secondary amino:
   (Method 1)
      The compound (7b) can be condensed with the compound (3d) to produce the compound (I) of the present invention. As a condensing agent, 1-ethyl-3-(3-dimethylaminopropyl)carbodiimide hydrochloride, (1H-1,2,3-benzotriazol-1-yloxy)(tri(dimethylamino))phosphonium hexafluorophosphate or the like can be used. An organic base such as triethylamine also can be used. As the solvent, tetrahydrofuran, N,N-dimethylformamide or the like can be used. The reaction temperature is between 0 °C and a reflux temperature. The reaction time is between 10 minutes and 30 hours.
   (Method 2) When R^{1a}, R^{9a} or R¹⁰ does not contain alkoxycarbonyl:
      The compound (7b) can be condensed with the compound (3a), R¹⁰³ of the resultant compound is then deprotected, followed by condensing with an amine or a salt thereof to produce the compound (I) of the present invention.
      In condensation of the compound (7b) with the compound (3a), as the condensing agent, 1-ethyl-3-(3-dimethylaminopropyl)carbodiimide hydrochloride, (1H-1,2,3-benzotriazol-1-yloxy)(tri(dimethylamino))phosphonium hexafluorophosphate or the like can be used. A base such as triethylamine can also be suitably used. As the solvent, tetrahydrofuran, N,N-dimethylformamide or the like can be used. The reaction temperature is between 0 °C and a reflux temperature. The reaction time is between 10 minutes and 30 hours.
      For the deprotection of R¹⁰³, hydrolysis using a base or the like can be used.
      In condensation with an amine or a salt thereof, general condensation of a carboxylic acid with an amine can be used. Specifically for example, as the solvent, N,N-dimethylformamide and tetrahydrofuran can be used, and as the condensing agent, carbonyl diimidazole, dicyclohexyl carbodiimide, 1-ethyl-3-(3-dimethylaminopropyl)carbodiimide hydrochloride and (1H-1,2,3-benzotriazol-1-yloxy)(tri(dimethylamino))phosphonium hexafluorophosphate can be used. A base such as triethylamine can also be suitably used. The reaction temperature is between 0 °C and a reflux temperature. The reaction time is between 10 minutes and 30 hours.
(2) When R^{1a} or R^{9a} contains hydroxyl, primary amino or secondary amino:
   After the substituent is protected if necessary, the above reaction can be carried out, followed by deprotecting suitably to produce the compound (I) of the present invention.
(3) After the process, in order to convert substituent groups on R^{1a} or R^{9a}, generally used reactions such as oxidation, reduction, esterification, amidation, protection, deprotection and hydrolysis can also be carried out, as described in <Process 6-1> of the above [Production method 6].

### <Process 7-3>

The process is a process for producing the compound (7c) from the compound (1d). The methods similar to those in <Process 6-1> can be used, for example, a method wherein the compound (1d) is converted to a carbamic acid ester derivative using a compound represented by the formula Ar-OC(=O)-Cl,
wherein Ar represents the same meaning as defined above, followed by reacting with an amine can be used. Alternatively, the compound (1d) can be reacted with a carbamate derivative, an isocyanate derivative to convert to a corresponding urea derivative. As the solvent, chloroform, toluene, N-methylpyrrolidone, N,N-dimethylformamide, dimethylsulfoxide, chlorobenzene or the like can be used. A mixed solvent of the above solvent and water also can be used. A base also can be used. Specifically, an organic base such as pyridine, triethylamine and diisopropylethylamine, and an inorganic base such as potassium carbonate, cesium carbonate, sodium hydride and sodium hydroxide can be used. The reaction temperature is between 0 °C and a reflux temperature. The reaction time is between 10 minutes and 30 hours.

### <Process 7-4>

The process is a process for producing the compound (I) of the present invention from the compound (7c).
(1) When R^{1a} or R^{9a} does not contain hydroxyl, primary amino or secondary amino:
   The compound (I) of the present invention can be obtained by a coupling reaction of the compound (7c) and the compound (5a). The methods similar to those in <Process 1A-4> can be used. As the solvent, N-methylpyrrolidone, N,N-dimethylformamide, dimethyl sulfoxide, 2-ethoxyethanol, chlorobenzene or the like can be used. A base or an acid may be added in the reaction system, and specifically an organic base such as triethylamine and diisopropylethylamine, an inorganic base such as potassium carbonate, cesium carbonate and sodium hydride, or an acid such as pyridine hydrochloride and hydrochloric acid can be used. The reaction temperature is between room temperature and a reflux temperature. The reaction time is between 10 minutes and 30 hours.
(2) When R^{1a} or R^{9a} contains hydroxyl, primary amino or secondary amino:
   After these substituents are suitably protected, the above reaction can be carried out followed by deprotecting suitably to produce the compound (I) of the present invention.
(3) After the process, in order to convert substituent groups on R^{1a} or R^{9a}, generally used reactions such as oxidation, reduction, esterification, amidation, protection, deprotection and hydrolysis can also be carried out in a suitable succeeding process, as described in <Process 6-1> of the above [Production method 6].

[0097] [Production Method 8] A method for producing an intermediate (1d),
wherein X is a group represented by the formula -C(R^{10b})=

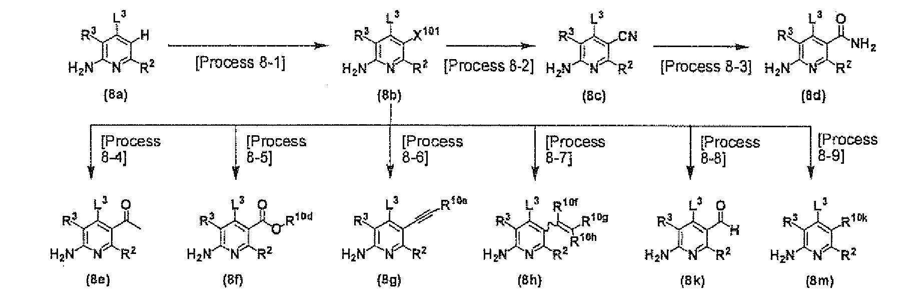

In the scheme, L³ represents chlorine or bromine; X¹⁰¹ represents chlorine, bromine or iodine; R^{10b} represents halogen, cyano, C₁₋₆ alkyl, C₂₋₆ alkenyl, C₂₋₆ alkynyl or a group represented by the formula -CO-R¹², wherein R¹² represents the same meaning as defined above; R^{10d} represents C₁₋₆ alkyl; R^{10e} represents hydrogen or C₁₋₄ alkyl; R^{10f}, R^{10g} and R^{10h} may be the same or different and each represents hydrogen or C₁₋₄ alkyl, with the proviso that the total carbon number of R^{10f}, R^{10g} and R^{10h} is 0 or more to 4 or less; R^{10k} represents C₁₋₆ alkyl; and the other symbols represent the same meanings as defined above.

### <Process 8-1>

The process is a process for chlorinating, brominating or iodinating the 5-position of the compound (8a) to produce the compound (8b). For example, a halogenating agent such as iodine, N-iodosuccinimide, bromine, N-bromosuccinimide and N-chlorosuccinimide can be used. As the solvent, for example, N,N-dimethylformamide, dimethyl sulfoxide, dichloromethane and acetonitrile can be used. The reaction temperature is between 0 °C and a reflux temperature. The reaction time is between 10 minutes and 48 hours.

### <Process 8-2>

The process is a process for converting X¹⁰¹ of the compound (8b) to cyano to produce the compound (8c). Concerning the combination of L³ and X¹⁰¹ upon cyanation, X¹⁰¹ is preferably iodine or bromine when L³ is chloride, and X¹⁰¹ is preferably iodine when L³ is bromine. For example, in the presence of a palladium catalyst such as tetrakis(triphenylphosphine)palladiwn(0) and dichlorobis(triphenylphosphine)palladium(II), 0.5-0.6 equivalent of zinc cyanide is used relative to the compound (8b), or 1.0-1.2 equivalent of potassium cyanide or trimethylsilyl cyanide is used relative to the compound (8b). As the solvent, for example, N,N-dimethylformamide, dioxane or tetrahydrofuran can be used. The reaction temperature is between room temperature and a reflux temperature. The reaction time is between 10 minutes and 10 hours.

### <Process 8-3>

The process is a process for producing the compound (8d) from the compound (8c). Hydrolysis using an inorganic base such as potassium carbonate and a hydrogen peroxide can be used. As the solvent, dimethyl sulfoxide or the like can be used. The reaction temperature is between 0 °C and a reflux temperature. The reaction time is between 10 minutes and 10 hours. A method of heating under reflux in a solvent such as toluene and tetrahydrofuran in the presence of potassium trimethylsilanolate, as described in Tetrahedron Lett., 41, 3747 (2000), also can be used. The reaction time is between 10 minutes and 60 hours.

### <Process 8-4>

The process is a process for producing the compound (8e) from the compound (8b). A method of reacting with (1-ethoxyvinyl)tributyltin in the presence of a palladium catalyst such as dichlorobis(triphenylphosphine)palladium(II) and tetrakis(triphenylphosphine)palladium(0) can be used. In the reaction system, a salt such as lithium chloride may be added. As the solvent, tetrahydrofuran, N,N-dimethylformamide, N-methylpyrrolidone or the like can be used. The reaction temperature is between room temperature and a reflux temperature. The reaction time is between 10 minutes and 60 hours.
As for a document that complements the above method, Tetrahedron, 53 (14), 5159 (1997) can be mentioned.

### <process 8-5>

The process is a process for producing the compound (8f) from the compound (8b). A method of reacting an alcohol represented by the formula R^{10d}-OH with carbon monoxide in the presence of a palladium catalyst such as dichlorobis(triphenylphosphine)palladium(II) can be used. In the reaction system, a base such as triethylamine and diisopropylethylamine may be added. As the solvent, an alcohol represented by the formula R^{10d}-OH, tetrahydrofuran, N,N-dimethylformamide, N-methylpyrrolidone, dimethyl sulfoxide or the like can be used. The reaction temperature is between room temperature and a reflux temperature. The reaction time is between 10 minutes and 60 hours.
As for a document that complements the above method, Tetrahedron Lett., 25 (51), 5939 (1984) can be mentioned.

### <Process 8-6>

The process is a process for producing the compound (8g) from the compound (8b). The compound (8b) can be reacted with an acetylene derivative in the presence of a palladium catalyst such as dichlorobis(triphenylphosphine)palladium(II) to produce the compound (8g). In the reaction system, an organic base such as triethylamine or an inorganic base such as potassium carbonate and sodium hydroxide may be added. A monovalent copper halide may coexist. As the solvent, tetrahydrofuran, N,N-dimethylformamide, N-methylpyrrolidone, dioxane, 1.2-dimethoxyethane, toluene, benzene, acetonitrile or the like can be used. The reaction temperature is between room temperature and a reflux temperature. The reaction time is between 10 minutes and 60 hours.

### <Process 8-7>

The process is a process for producing the compound (8h) from the compound (8b). The compound (8b) can be reacted with a trialkylvinyltin derivative in the presence of a palladium catalyst such as dichlorobis(triphenylphosphine)palladium(II) to produce the compound (8h). In the reaction system, hexamethylphosphoramide or the like may be added. As the solvent, tetrahydrofuran, N,N-dimethylformamide, N-methylpyrrolidone, dimethyl sulfoxide or the like can be used. The reaction temperature is between room temperature and a reflux temperature. The reaction time is between 10 minutes and 60 hours.
As for a document that complements the above method, Tetrahedron, 53 (14), 5159 (1997) can be mentioned.

### <Process 8-8>

The process is a process for producing the compound (8k) from the compound (8b). A method of reacting with carbon monoxide in the presence of a palladium catalyst such as dichlorobis(triphenylphosphine)palladium(II), and sodium formate, as described in Bull. Chem. Soc. Jpn., 67 (8), 2329 (1994), can be used. As the solvent, tetrahydrofuran, N,N-dimethylformamide, N-methylpyrrolidone, dimethyl sulfoxide or the like can be used. The reaction temperature is between room temperature and a reflux temperature. The reaction time is between 10 minutes and 60 hours.

### <Process 8-9>

The process is a process for producing the compound (8m) from the compound (8b). A method of reacting with a reagent prepared from alkyl magnesium halide and zinc(II)chloride in the presence of a palladium catalyst such as dichlorobis(triphenylphosphine)palladium(II), as described in J. Org. Chem., 2001, 66 (20), 605, can be used. As the solvent, tetrahydrofuran or the like can be used. The reaction temperature is between room temperature and a reflux temperature. The reaction time is between 10 minutes and 60 hours. Alternatively, a method of reacting with tetraalkyltin in the presence of a palladium catalyst such as dichlorobis(triphenylphosphine)palladium(II), as described in Tetrahedron Lett. 1996, 37 (14), 2409-2412, can be used. As the solvent, toluene or the like can be used. The reaction temperature is between room temperature and a reflux temperature. The reaction time is between 10 minutes and 60 hours.
The reactions similar to described in the processes of <Process 8-1> to <Process 8-9> can be applied to the conversion of the substituent at the 5-position (R¹⁰) of the pyridine ring of various intermediates described in [Production Method 1] to [Production Method 7].

The "leaving group" may be any group generally known as a leaving group in organic synthesis, and is not particularly limited. Specifically for example, it includes halogen such as chlorine, bromine and iodine; nitro; alkylsulfonyloxy such as methanesulfonyloxy, trifluoromethanesulfonyloxy and ethanesulfonyloxy; arylsulfonyloxy such as benzenesulfonyloxy and p-toluenesulfonyloxy; and alkanoyloxy such as acetoxy and trifluoroacetoxy.

The amino-protecting group may be any group generally known as an amino-protecting group in organic synthesis, and is not particularly limited. Specifically for example, it includes substituted or unsubstituted acyl such as formyl, acetyl, chloroacetyl, dichloroacetyl, propionyl, phenylacetyl, phenoxyacetyl and thienylacetyl; alkoxycarbonyl such as t-butoxycarbonyl; substituted or unsubstituted benzyloxycarbonyl such as benzyloxycarbonyl and 4-nitrobenzyloxycarbonyl; substituted or unsubstituted alkyl such as methyl, t-butyl and 2,2,2-trichloroethyl; substituted benzyl such as trityl, 4-methoxybenzyl, 4-nitrobenzyl and diphenylmethyl; alkylcarbonyloxyalkyl such as pivaloyloxymethyl; alkylsilyl such as trimethylsilyl and t-butyldimethylsilyl; and alkylsilylalkoxyalkyl such as trimethylsilylmethoxymethyl, trimethylsilylethoxymethyl, t-butyldimethylsilylmethoxymethyl, t-butyldimethylsilylethoxymethyl.

These protecting groups can be deprotected by a conventional method such as hydrolysis and reduction depending on the kind of the protecting group used.

The hydroxyl-protecting group may be any group generally known as a hydroxyl-protecting group in organic synthesis, and is not particularly limited. Specifically for example, it includes alkylsilyl such as trimethylsilyl and t-butyldimethylsilyl; alkoxymethyl such as methoxymethyl and 2-methoxyethoxymethyl; tetrahydropyranyl; substituted or unsubstituted benzyl such as benzyl, 4-methoxybenzyl, 2,4-dimethoxybenzyl, 2-nitrobenzyl, 4-nitrobenzyl and trityl; alkenyl such as allyl; and acyl such as formyl and acetyl.

These protecting groups can be deprotected by a conventional method such as hydrolysis and reduction depending on the kind of the protecting group used.

The carboxyl-protecting group may be any group generally known as a carboxyl-protecting group in organic synthesis, and is not particularly limited. For example, it includes substituted or unsubstituted alkyl such as methyl, ethyl, i-propyl, t-butyl, 2-iodoethyl and 2,2,2-trichloroethyl; alkoxymethyl such as methoxymethyl, ethoxymethyl and i-butoxymethyl; acyloxymethyl such as butylyloxymethyl and pivaloyloxymethyl; alkoxycarbonyloxyethyl such as 1-methoxycarbonyloxyethyl and 1-ethoxycarbonyloxyethyl; and substituted or unsubstituted benzyl such as benzyl, 4-methoxybenzyl, 2-nitrobenzyl and 4-nitrobenzyl.

These protecting groups can be deprotected by a conventional method such as hydrolysis and reduction depending on the kind of the protecting group used.

In addition to the above protecting groups, groups described in Greene et al., "Protective Groups in Organic Syntheses", 3rd Edition, JOHN WILEY & SONS, INC. can be used.

There have been described above the typical examples of a method for producing the pyridine or pyrimidine derivative (I) according to the present invention. Each of the starting materials and various reagents may be a salt, a hydrate or a solvate, varies depending on a starting material, a solvent and the like to be used, and is not limited to a particular one as long as it does not inhibit a reaction. A solvent to be used varies depending on a starting material, a reagent and the like, and is not limited to a particular one as long as it does not inhibit a reaction and can dissolve the starting material to some extent.

The pyridine or pyrimidine derivative (I) according to the present invention, if provided as a free form, can be converted to a form of a salt or a hydrate which the forgoing may form by a conventional method.

The pyridine or pyrimidine derivative (I) according to the present invention, if provided as the form of a salt or a hydrate of the compound (I), can be converted to a free form of the compound (I) by a conventional method.

The pyridine or pyrimidine derivative (I) according to the present invention and the various isomers (such as geometric isomers and optical isomers) of the pyridine or pyrimidine derivative (I) according to the present invention can be purified and isolated by a conventional separation means, including recrystallization, diastereomer salt method, enzyme separation method, and various chromatographies such as thin-layer chromatography, column chromatography and gas chromatography.

The pyridine or pyrimidine derivative (I) of the present invention is generally mixed with an appropriate additive and formulated to use as a medicament. But the compound of the present invention may be used alone without any additive.

The above additives include excipients, binders, lubricants, disintegrators, coloring agents, taste correctives, emulsifiers, surfactants, dissolving aids, suspending agents, isotonizing agents, buffering agents, antiseptics, antioxidants, stabilizers, absorption accelerators and the like. These also may be appropriately combined to use if desired.

The excipients include, for example, lactose, white soft sugar, glucose, corn starch, mannitol, sorbitol, starch, pregelatinized starch, dextrin, crystalline cellulose, soft silicic anhydride, aluminum silicate, calcium silicate, magnesium aluminometasilicate and calcium hydrogenphosphate.

The binders include, for example, polyvinyl alcohol, methylcellulose, ethylcellulose, gum arabic, tragacanth, gelatin, shellac, hydroxypropylmethylcellulose, hydroxypropylcellulose, carboxymethylcellulose sodium, polyvinylpyrrolidone and macrogol.
The lubricants includes magnesium stearate, calcium stearate, sodium stearyl fumarate, talc, polyethylene glycol and colloidal silica.
The disintegrators includes, for example, crystalline cellulose, agar, gelatin, calcium carbonate, sodium hydrogencarbonate, calcium citrate, dextrin, pectin, low-substituted hydroxypropylcellulose, carboxymethylcellulose, carboxymethylcellulose calcium, croscarmellose sodium, carboxymethyl starch and carboxymethyl starch sodium.
The coloring agents include, for example, those approved for addition to pharmaceuticals, such as iron sesquioxide, yellow iron sesquioxide, carmine, caramel, β-carotene, titanium oxide, talc, riboflavin sodium phosphate, yellow aluminum lake and the like.
The taste correctives include cocoa powder, menthol, aromatic powders, mentha oil, borneol, powdered cinnamon bark and the like.
The emulsifiers or surfactants include, for example, stearyl triethanolamine, sodium lauryl sulfate, lauryl aminopropionic acid, lecitin, glycerin monostearate, sucrose fatty acid esters and glycerin fatty acid esters.
The dissolving aids include, for example, polyethylene glycol, propylene glycol, benzyl benzoate, ethanol, cholesterol, triethanolamine, sodium carbonate, sodium citrate, polysorbate 80 and nicotinamide.
The suspending agents include, for example, hydrophilic polymers such as polyvinyl alcohol, polyvinylpyrrolidone, methylcellulose, hydroxymethylcellulose, hydroxyethylcellulose and hydroxypropylcellulose, in addition to the above surfactants.
The isotonizing agents include, for example, glucose, sodium chloride, mannitol and sorbitol.
The buffering agents include, for example, buffer solutions of phosphate, acetate, carbonate and citrate.
The antiseptics include, for example, methylparaben, propylparaben, chlorobutanol, benzyl alcohol, phenethyl alcohol, dehydroacetic acid and sorbic acid.
The antioxidants include, for example, sulfite, ascorbic acid and α-tocopherol.
The stabilizers include those commonly used in pharmaceuticals.
The absorption accelerators include those commonly used in pharmaceuticals.

The formulation may be in an oral form such as tablets, powders, granules, capsules, syrups, lozenges and inhalants; an external application form such as suppositories, ointment, eye salve, tape, eye drops, nose drops, ear drops, pap and lotion; and an injection.

An oral formulation may be formulated by combining appropriately the above additives, and may be coated on the surface if necessary.

An external application may be formulated by combining appropriately the above additives, particularly excipients, binders, taste correctives, emulsifiers, surfactants, dissolving aids, suspending agents, isotonizing agents, antiseptics, antioxidants, stabilizers and absorption accelerators.

An injection may be formulated by combining appropriately the above additives, particularly emulsifiers, surfactants, dissolving aids, suspending agents, isotonizing agents, buffering agents, antiseptics, antioxidants, stabilizers and absorption accelerators.

The dose of the pyridine or pyrimidine derivative according to the present invention for the pharmaceutical use varies depending on symptoms and age of the patients, but it will ordinary be 0.1 mg to 10 g (preferably 1 mg to 2 g) for an oral formulation, 0.01 mg to 10 g (preferably 0.1 mg to 2 g) for an external application, and 0.01 mg to 10 g (preferably 0.1 mg to 2 g) for an injection, which is administrated once or divided over two to four times a day.

The pyridine or pyrimidine derivative according to the present invention is useful as a pharmaceutical composition against tumors in which expression of hepatocyte growth factor receptor is enhanced, as a hepatocyte growth factor receptor inhibitor against tumors in which expression of hepatocyte growth factor receptor is enhanced, and as an anti-tumor agent against tumors in which expression of hepatocyte growth factor receptor is enhanced.

The method for predicting anti-tumor effect of a pyridine or pyrimidine derivative according to the present invention comprises the steps of:
assaying expression level of hepatocyte growth factor receptor in tumor cells; and
determining whether a pyridine or pyrimidine derivative is effective or not against the tumor cells by using the expression level of hepatocyte growth factor receptor as an index based on the assayed expression level,
wherein the pyridine or pyrimidine derivative is at least one compound, salt thereof or solvate of the foregoing selected from the compound represented by the above formula (I).

The expression level of hepatocyte growth factor receptor means the expression level of hepatocyte growth factor receptor protein and the expression level of hepatocyte growth factor receptor mRNA. In addition, the expression level of hepatocyte growth factor receptor is considered to be enhanced by gene amplification, thus the expression level includes hepatocyte growth factor receptor DNA copy number. The expression of hepatocyte growth factor receptor may be measured in terms of at least one of protein expression, mRNA expression and DNA copy number.

The expression level of hepatocyte growth factor receptor protein can be measured by a method known to those skilled in the art, e.g. an immunological method. The immunological method includes fluorescent antibody method, enzyme-linked immunosorbent assay (ELISA), radioimmunoassay (RIA), western blotting, immunostaining (immunohistochemistry) method, etc. and immunostaining method is particularly preferable.

The expression level of hepatocyte growth factor receptor mRNA can be measured by a method known to those skilled in the art, e.g. northern blotting, dot blotting, RT-PCR, etc.

Hepatocyte growth factor receptor DNA copy number can be measured by a method known to those skilled in the art, e.g. fluorescent *in situ* hybridization (FISH), quantitative PCR, southern blotting, etc, and FISH is particularly preferable. FISH is a technique where a fluorescent-labeled probe DNA against hepatocyte growth factor receptor is hybridized to a sample and the number of the resultant fluorescent signal is counted under a fluorescent microscope. It is possible to evaluate the copy number more precisely by labeling a probe against Chromosome 7 alphasatellite with a fluorescent dye different from that of the probe against hepatocyte growth factor receptor and performing hybridization at the same time.

When assaying the expression level of hepatocyte growth factor receptor, other than the tumor cells in which anti-tumor effect of the pyridine or pyrimidine derivative is to be predicted, tumor cells known to show high expression level of hepatocyte growth factor receptor may be used as a positive control and tumor cells known to show low expression level of hepatocyte growth factor receptor may be used as a negative control. The present inventors have confirmed that MKN-45 (human gastric cancer cells), SNU-5 (human gastric cancer cells) and EBC-1 (human lung cancer cells) show high expression level of hepatocyte growth factor receptor. The present inventors have confirmed that MKN-74 (human gastric cancer cells), SNU-1 (human gastric cancer cells) and A549 (human lung cancer cells) show low expression level of hepatocyte growth factor receptor.

Determining whether a pyridine or pyrimidine derivative is effective or not against the tumor cells by using the expression level of hepatocyte growth factor receptor as an index based on the assayed expression level may be performed as follows. The pyridine or pyrimidine derivative is expected to be effective against tumor cells showing high expression level of hepatocyte growth factor receptor. On the other hand, the pyridine or pyrimidine derivative is expected to be less effective against tumor cells showing low expression level of hepatocyte growth factor receptor compared to tumor cells showing high expression level of hepatocyte growth factor receptor.

With regard to measuring hepatocyte growth factor receptor DNA copy number by FISH, when the number of fluorescent signal of hepatocyte growth factor receptor is greater than that of Chromosome 7 alphasatellite, the tumor-cells are considered to show high expression level of hepatocyte growth factor receptor. When the number of fluorescent signal of hepatocyte growth factor receptor is nearly equal to that of Chromosome 7 alphasatellite, the tumor cells are considered to show low expression level of hepatocyte growth factor receptor.

It is possible to determine more precisely whether expression level of hepatocyte growth factor receptor is high or low by comparing with positive control and negative control.

It is possible to administer a pyridine or pyrimidine derivative only to tumor patients against whom the pyridine or pyrimidine derivative is effective by predicting anti-tumor effect of the pyridine or pyrimidine derivative.

The method for examining sensitivity of tumor cells to a pyridine or pyrimidine derivative according to the present invention comprises the steps of:
assaying expression levels of hepatocyte growth factor receptor in tumor cells extracted from a tumor patient before and after administration of a pyridine or pyrimidine derivative; and
determining that the tumor cells are sensitive to the pyridine or pyrimidine derivative if the expression level of hepatocyte growth factor receptor after administration of the pyridine or pyrimidine derivative is lower than the expression level of hepatocyte growth factor receptor before administration of the pyridine or pyrimidine derivative,
wherein the pyridine or pyrimidine derivative is at least one compound, salt thereof or solvate of the foregoing selected from the compound represented by the above formula (I).

In this method, tumor cells are extracted from a tumor patient before and after administration of a pyridine or pyrimidine derivative, and expression levels of hepatocyte growth factor receptor in the respective tumor cells are assayed according to the assay method described above. The tumor cells are considered to be sensitive to the pyridine or pyrimidine derivative if the expression level of hepatocyte growth factor receptor after administration of the pyridine or pyrimidine derivative is lower than the expression level of hepatocyte growth factor receptor before administration of the pyridine or pyrimidine derivative.

The method for administering a pyridine or pyrimidine derivative to a tumor patient according to the present invention comprises the steps of:
assaying expression level of hepatocyte growth factor receptor in tumor cells of a tumor patient;
determining whether a pyridine or pyrimidine derivative is effective or not against the tumor by using the expression level of hepatocyte growth factor receptor as an index based on the assayed expression level; and
administering the pyridine or pyrimidine derivative to the tumor patient in case of having determined effective,
wherein the pyridine or pyrimidine derivative is at least one compound, salt thereof or solvate of the foregoing selected from the compound represented by the above formula (I).

In this method, the pyridine or pyrimidine derivative is administered to a tumor patient in case of having determined the pyridine or pyrimidine derivative is effective against the tumor according to the assay method and criteria above.

The method for administering a pyridine or pyrimidine derivative to a tumor patient according to the present invention comprises the steps of:
assaying expression levels of hepatocyte growth factor receptor in tumor cells of a tumor patient and a non-tumor individual; and
administering a pyridine or pyrimidine derivative to the tumor patient if the expression level of hepatocyte growth factor receptor in tumor cells of the tumor patient is higher than the expression level of hepatocyte growth factor receptor in tumor cells of the non-tumor individual,
wherein the pyridine or pyrimidine derivative is at least one compound, salt thereof or solvate of the foregoing selected from the compound represented by the above formula (I).

Cells are extracted from a tumor patient and a non-tumor individual and expression levels of hepatocyte growth factor receptor in the respective cells are assayed according to the assay method described above. The pyridine or pyrimidine derivative is administered to the tumor patient in case that the expression level of hepatocyte growth factor receptor in the cells extracted from the tumor patient is higher compared to the cells extracted from the non-tumor individual. The cells extracted from the non-tumor individual are preferably tissues corresponding to the tumor tissue from which the tumor cells extracted.

### Examples

The pyridine or pyrimidine derivative according to the present invention can be produced, for example, by the methods described in the below Production Examples and Examples. But these Examples are for illustrative purposes, and the pyridine or pyrimidine derivative according to the present invention is not limited to the following specific Examples in any case.

In the Production Examples and Examples, YMC SIL-60-400/230W was used as silica gel for purification unless otherwise described.

### (Reference Example A-1) Ethyl 4-chloropyridine-2-carboxylate

A mixture of 4-chloropyridine-2-carboxylic acid (39.4g) and thionyl chloride (64 ml) was heated and stirred at 100 °C under a nitrogen atmosphere for 6 hr. The reaction mixture was allowed to cool down to room temperature. This was concentrated under reduced pressure and distilled azeotropically with toluene. The resultant residue was gradually added to ethanol while stirring in an ice bath. The reaction mixture was stirred at room temperature for 25.5 hr. The reaction mixture was concentrated under reduced pressure. To the residue was added a saturated aqueous solution of sodium hydrogencarbonate and extracted with ethyl acetate. The organic layer was washed with brine, dried over anhydrous sodium sulfate, and concentrated under reduced pressure to provide the titled compound as a brown oil (38.8 g, 83.6 %).
¹H-NMR Spectrum (CDCl₃) δ (ppm): 1.46 (3H, t, J = 7.2 Hz), 4.50 (2H, q, J = 7.2 Hz), 7.49 (1H, dd, J = 2.0, 5.2 Hz), 8.15 (1H, d, J = 2.0 Hz), 8.67 (1H, d, J = 5.2 Hz).

### Reference Example A-2 Ethyl 4-(3-fluoro-4-nitrophenoxy)pyridine-2-carboxylate

To ethyl 4-chloropyridine-2-carboxylate (19.4 g) were added 3-fluoro-4-nitrophenol (24.7 g) and chlorobenzene (7.0 ml), followed by heating and stirring under a nitrogen atmosphere at 120 °C for 4 hr. The reaction mixture was allowed to cool down to room temperature. Ethyl acetate (400 ml) and a saturated aqueous solution of sodium hydrogencarbonate (400 ml) were added thereto, followed by stirring at room temperature for 27 hr. Stirring was stopped and the aqueous layer was separated. To the organic layer was added again a saturated aqueous solution of sodium hydrogencarbonate, followed by stirring at room temperature for 2 days. Stirring was stopped and the aqueous layer was separated. The aqueous layer was extracted with ethyl acetate (300 ml). The organic layers were combined and washed with brine. The organic layer was dried over anhydrous sodium sulfate and concentrated under reduced pressure. The residue was purified by silica gel column chromatography (eluent; heptane:ethyl acetate = 2:1, 1:1, then ethyl acetate). Fractions containing the target compound were concentrated to provide the titled compound as a brown oil (12.9 g, 40.2 %).
¹H-NMR Spectrum (CDCl₃) δ (ppm): 1.45 (3H, t, J = 7.2 Hz), 4.49 (2H, q, J = 7.2 Hz), 6.97-7.01 (2H, m), 7.16 (1H, dd, J = 2.4, 5.6 Hz), 7.79 (1H, d, J = 2.4 Hz), 8.20 (1H, m), 8.76 (1H, d, J = 5.6 Hz).
ESI-MS (m/z): 329 [M+Na]⁺.

### (Reference Example A-3) 4-(4-Benzyloxycarbonylamino-3-fluorophenoxy)pyridine-2-carboxylic acid

To a solution of ethyl 4-(3-fluoro-4-nitrophenoxy)pyridine-2-carboxylate (8.56 g) in ethanol (150 ml) was added 20% palladium hydroxide on carbon (1.0 g), followed by stirring under a hydrogen atmosphere at room temperature for 9.5 hr. The catalyst was removed by filtration. To the filtrate was added a 4N solution of hydrochloric acid in ethyl acetate (14 ml) and concentrated. Concentration was stopped before dryness. Water (75 ml), acetone (150 ml) and sodium hydrogencarbonate (11.8 g) was added thereto. This was cooled in an ice bath, and benzyloxycarbonyl chloride (6.00 ml) was added. The reaction mixture was stirred at room temperature for 4 hr. The reaction mixture was concentrated under reduced pressure. The residue was extracted with ethyl acetate. The organic layer was washed with brine and dried over anhydrous sodium sulfate. This was concentrated under reduced pressure, and the residue was purified by silica gel column chromatography (eluent; heptane:ethyl acetate = 1:1, 1:2, then ethyl acetate). Fractions containing the target compound were concentrated under reduced pressure. The resultant solid was suspended in hexane and allowed to stand for a while, then the supernatant was removed by using a pipette. This residue was dried to provide ethyl 4-(4-benzyloxycarbonylamino-3-fluorophenoxy)pyridine-2-carboxylate as pale yellow solid (7.51 g, 65.4 %).
¹H-NMR Spectrum (CDCl₃) δ (ppm): 1.43 (3H, m), 4.45-4.52 (2H, m), 5.24 (2H, s), 6.87-6.92 (2H, m), 6.99 (1H, dd, J = 2.4, 5.6 Hz), 7.35-7.45 (6H, m), 7.65 (1H, d, J = 2.4 Hz), 8.19 (1H, m), 8.60 (1H, d, J = 5.6 Hz).
Ethyl 4-(4-benzyloxycarbonylamino-3-fluorophenyl)pyridine-2-carboxylate (7.95g) was dissolved in ethanol (120 ml), and water (25 ml) was added thereto. Lithium hydroxide (783 mg) was added thereto while stirring at room temperature, followed by stirring at room temperature for 1 hr. To the reaction mixture was added 1N hydrochloric acid (60 ml) and concentrated under reduced pressure. After concentration, precipitated crystals in the reaction mixture were collected by filtration and washed with water. The crystals were dissolved in ethyl acetate-tetrahydrofuran, and dried over anhydrous sodium sulfate. The solution after drying was concentrated under reduced pressure. The resultant crystals were suspended in hexane and collected by filtration. The crystals were dried to provide the target compound as pale yellow crystals (5.04 g, 72.0 %).
¹H-NMR Spectrum (DMSO-d₆) δ (ppm): 5.18 (2H, s), 7.08 (1H, m), 7.23 (1H, m), 7.24-7.46 (8H, m), 7.75 (1H, m), 8.59 (1H, d, J = 5.6 Hz), 9.59 (1H, s).

### (Reference Example A-4) tert-Butyl [4-(4-benzyloxycarbonylamino-3-fluorophenoxy)pyridin-2-yl]carbamate

To a suspension of 4-(4-benzyloxycarbonylamino-3-fluorophenoxy)pyridine-2-carboxylic acid (5.04 g) in *tert*-butanol (50 ml) was added triethylamine (4.6 ml) at room temperature, followed by stirring. Diphenylphosphoryl azide (3.13 ml) was added thereto at room temperature, followed by stirring under a nitrogen atmosphere at room temperature for 30 min. Then the reaction mixture was heated and stirred at 90 °C for 30 min and at 100 °C for 4 hr. The reaction mixture was allowed to cool down to room temperature. Ethyl acetate (25 ml) was added thereto, and the reaction mixture was stirred in an ice bath for 30 min. Precipitated crystals were collected by filtration and washed with diethyl ether. These crystals were dried under aeration at room temperature for 1 hr to provide the titled compound as colorless crystals (3.92 g, 65.5 %).
¹H-NMR Spectrum (DMSO-d₆) δ (ppm): 1.42 (9H, s), 5.17 (2H, s), 6.62 (1H, dd, J = 2.4, 5.6 Hz), 7.01 (1H, dd, J = 2.2, 8.8 Hz), 7.21 (1H, dd, J = 2.2, 11.2 Hz), 7.35-7.42 (6H, m), 7.70 (1H, m), 8.14 (1H, d, J = 5.6 Hz), 9.53 (1H, s), 9.83 (1H, s).

### (Reference Example A-5) Benzyl [4-(2-aminopyridin-4-yloxy)-2-fluorophenyl]carbamate

A 4N solution of hydrochloric acid in ethyl acetate (120 ml) was cooled in an ice bath. *tert*-Butyl [4-(4-benzyloxycarbonylamino-3-fluorophenoxy)pyridin-2-yl]carbamate (3.92 g) was added thereto while stirring, followed by stirring in an ice bath for 10 min. The reaction mixture was then stirred at room temperature for 3.5 hr. The reaction mixture was concentrated under reduced pressure. Ethyl acetate (150 ml) and a saturated aqueous solution of sodium hydrogencarbonate (70 ml) were added thereto, and liquid-liquid separation was carried out. The aqueous layer was extracted with ethyl acetate (50 ml). The combined organic layer was washed with brine and dried over anhydrous sodium sulfate. The organic layer after drying was concentrated under reduced pressure. The resultant crystals were suspended in a mixed solvent of hexane-ethyl acetate (5:1). The crystals were collected by filtration and washed with a mixed solvent of hexane-ethyl acetate (5:1). The crystals were sucked to dryness at room temperature to provide the titled compound as pale yellow crystals (2.93 g, 95.9 %).
¹H-NMR Spectrum (CDCl₃) δ (ppm): 4.49 (2H, m), 5.23 (2H, s), 5.95 (1H, d, J = 2.0 Hz), 6.26 (1H, dd, J = 2.0, 6.0 Hz), 6.84-6.90 (2H, m), 7.00 (1H, m), 7.34-7.42 (5H, m), 7.94 (1H, d, J = 6.0 Hz), 8.10 (1H, m).
ESI-MS (m/z): 354 [M+H]⁺.

### (Reference Example A-6) Phenyl [4-3-fluoro-4-{[1-(4-fluorophenylcarbamoyl)cyclopropanecarbonyl]amino}phenoxy)pyridin-2-yl]-N-phenoxycarbonylcarbamate

To a solution of benzyl [4-(2-aminopyridin-4-yloxy)-2-fluorophenyl]carbamate (1.25 g) in tetrahydrofuran (100 ml) were added triethylamine (1.48 ml) and phenyl chloroformate (1.11 ml), followed by stirring at room temperature for 1 hr. The reaction mixture was partitioned between ethyl acetate and a 1N aqueous solution of sodium hydroxide. The organic layer was washed with brine, dried over anhydrous sodium sulfate. The solvent was removed to provide a crude product of phenyl N-[4-(4-benzyloxycarbonylamino-3-fluorophenoxy)pyridin-2-yl]-N-phenoxycarbonylcarbamate as a brown oil (ESI-MS (m/z): 616 [M+Na]⁺). This was dissolved in tetrahydrofuran (200 ml), 20% palladium hydroxide (497 mg) was added thereto, and the mixture was stirred under a hydrogen atmosphere at room temperature for 4 hr. The catalyst was removed by filtration and washed with tetrahydrofuran. The filtrate was concentrated to 20 ml to provide a solution of phenyl N-[4-(4-amino-3-fluorophenoxy)pyridin-2-yl]-N-phenoxycarbonylcarbamate (ESI-MS (m/z): 482 [M+Na]⁺, 941 [2M+Na]⁺) in tetrahydrofuran. This was dissolved in N,N-dimethylformamide (50 ml). 1-(4-Fluorophenylcarbanoyl)cyclopropanecarboxylic acid (1.58 g), benzotriazol-1-yloxytris(dimethylamino)phosphonium hexafluorophosphate (3.13 g) and triethylamine (0.987 ml) were added thereto, followed by stirring at room temperature for 13.5 hr. The reaction mixture was partitioned between ethyl acetate and brine. The organic layer was washed with a 1N aqueous solution of sodium hydroxide and brine in this order, and dried over anhydrous sodium sulfate. This was concentrated, and the residue was purified by silica gel column chromatography (heptane:ethyl acetate = 3:2, 1:1 then 1:2) to provide the titled compound as colorless foam (940 mg, 40.0 %).
¹H-NMR Spectrum (CDCl₃) δ (ppm): 1.68-1.76 (4H, m), 6.90 (1H, dd, J = 2.4, 5.6 Hz), 6.95 (1H, m), 6.98 (1H, m), 7.03-7.07 (3H, m), 7.18 (4H, d, J = 8.4 Hz), 7.25 (2H, m), 7.38 (4H, m), 7.48 (2H, m), 8.27 (1H, m), 8.46 (1H, d, J = 5.6 Hz), 8.75 (1H, s), 9.40 (1H, s).
ESI-MS (m/z): 687 [M+Na]⁺.

### (Example 15) N-(2-Fluoro-4-{[2-({[4-(4-methylpiperazin-1-yl)piperidin-1-yl]carbonyl}amino)pyridin-4-yl]oxy}phenyl)-N'-(4-fluorophenyl)cyclopropane-1,1-dicarboxamide

To a solution of phenyl N-[4-(3-fluaro-4-{[1-(4-fluorophenylcarbamoyl)cyclopropanecarbonyl]amino}phenoxy)pyridin-2-yl]-N-phenoxycarbonylcarbamate (50.0 mg) in N,N-dimethylformamide (2.0 ml) was added 1-methyl-4-(piperidin-4-yl)piperazine (68.7 mg), followed by stirring at room temperature for 12 hr. The reaction mixture was partitioned between ethyl acetate and a 1N aqueous solution of sodium hydroxide. The organic layer was washed with brine, and dried over anhydrous sodium sulfate. The solvent was removed, and the residue was purified by silica gel column chromatography (Fuji Silysia NH, ethyl acetate, ethyl acetate:methanol = 20:1, then 10:1). Fractions containing the target compound were concentrated. To the residue was added diethyl ether:hexane =1:3, and the precipitate was collected by filtration. This was washed with hexane and dried under aeration to provide the titled compound as white powder (34.6 mg, 72.8 %).
¹H-NMR Spectrum (CDCl₃) δ (ppm): 1.44 (2H, m), 1.68 (2H, m), 1.75 (2H, m), 1.90 (2H, m), 2.32 (3H, s), 2.39-2.71 (9H, m), 2.90 (2H, m), 4.11 (2H, m), 6.55 (1H, dd, J = 2.0, 5.6 Hz), 6.92 (2H, m), 7.04 (2H, m), 7.26 (1H, covered by CDCl₃), 7.50 (2H, dd, J = 4.8, 9.2 Hz), 7.62 (1H, d, J = 2.0 Hz), 8.06 (1H, d, J = 5.6 Hz), 8.20 (1H, m), 8.84 (1H, s), 9.20 (1H, s).
ESI-MS (m/z): 634 [M+H]⁺, 656 [M+Na]⁺.

### (Reference Example B-1) N-(4-Fluorophenyl)-N'-(2-fluoro-4-hydroxyphenyl)cyclopropane-1,1-dicarboxamide

To a solution of 1-(4-fluorophenylcarbamoyl)cyclopropanecarboxylic acid (1.02 g) in N,N-dimethylformamide (5.0 ml) were added triethylamine (1.28 ml) and benzotriazol-1-yloxytris(dimethylamino)phosphonium hexafluorophosphate (2.03 g), followed by stirring at room temperature for 5 min. To this was added 4-amino-3-fluorophenol hydrochloride (500 mg), followed by stirring at room temperature for 3 days. The reaction mixture was partitioned between ethyl acetate and a 1N aqueous solution of sodium hydroxide. The organic layer was washed with a 1N aqueous solution of sodium hydroxide. To the aqueous layer was added 5N hydrochloric acid to make it acidic, this was extracted with ethyl acetate. The organic layer was washed with brine and dried over anhydrous sodium sulfate. The solvent was removed and the residue was purified by silica gel column chromatography (eluent; heptane:ethyl acetate = 2:3 to 1:2). Fractions containing the target compound were concentrated under reduced pressure to provide the titled compound as a pale red solid (3 95 mg, 39 %).
¹H-NMR Spectrum (CDCl₃) δ (ppm): 1.50-1.80 (4H, m), 4.99 (1H, brs), 6.60-6.70 (2H, m), 6.90-7.10 (2H, m), 7.45-7.55 (2H, m), 7.98 (1H, m), 8.23 (1H, brs), 9.58 (1H, brs).
ESI-MS (m/z): 355 [M+Na]⁺.

### (Reference Example B-2) 4-(4-Amino-3-fluorophenoxy)pyridine-2-carboxamide

4-Amino-3-fluorophenol (5.7 g) was dissolved in dimethyl sulfoxide (57 ml) under a nitrogen atmosphere, and potassium tert-butoxide (5.6 g) was added at room temperature, followed by stirring for 15 min. To the reaction mixture was added 4-chloropicolylamide (5.0 g), followed by stirring in an oil bath at an external temperature of 80 °C under a nitrogen atmosphere for 50 min. The reaction mixture was allowed to cool down to room temperature. To the reaction mixture was added a 1N aqueous solution of sodium hydroxide (85.5 ml), followed by stirring. The precipitated solid was collected by filtration, and washed with water. The solid was dried under aeration, then hot air-dried at 100 °C to provide the titled compound as pale brown powder (5.88 g, 74.3 %).
¹H-NMR Spectrum (DMSO-d₆) δ (ppm): 5.18-5.30 (2H, m), 6.80 (1H, dd, J = 2.4, 8.4 Hz), 6.81-6.90 (1H, m), 7.02 (1H, dd, J = 2.4, 11.6 Hz), 6.99-7.14 (1H, m), 7.32-7.39 (1H, m), 7.69 (1H, brs), 8.10 (1H, brs), 8.48(1H, m).

### (Reference Example B-3) 4-(3-Fluoro-4-{[1-(4-fluorophenylcarbamoyl)cyclopropanecarbonyl]amino}phenoxy)pridine-2-carboxamide

N-(4-Fluorophenyl)-N'-(2-fluoro-4-hydroxyphenyl)cyclopropane-1,1-dicarboxamide (665 mg) was dissolved in N-methylpyrrolidone (10 ml) under a nitrogen atmosphere, and potassium tert-butoxide (247 mg) was added at room temperature, followed by stirring for 1.5 hr. After 4-chloropicolylamide (313 mg) was added, the reaction mixture was stirred under a nitrogen atmosphere at 110 °C overnight, then at 120 °C for 8 hr. The reaction mixture was allowed to cool down to room temperature. The reaction mixture was partitioned between ethyl acetate and water. The organic layer was washed with a saturated aqueous solution of sodium hydrogencarbonate (twice) and brine, and dried over anhydrous sodium sulfate. The solvent was concentrated under reduced pressure, and the residue was purified by silica gel column chromatography (eluent; heptane:ethyl acetate = 1:2, 1:3, then 1:4). Fractions containing the target compound were concentrated under reduced pressure. After ethyl acetate (3 ml)-heptane (6 ml) was added, crystals were allowed to precipitate under sonication. The solvent was removed and the crystals were dried under reduced pressure to provide the titled compound as pale brown crystals (261 mg, 29 %).
¹H-NMR Spectrum (CDCl₃) δ (ppm): 1.40-1.80 (4H, m), 5.54 (1H, brs), 6.90-7.30 (7H, m), 7.71 (1H, m), 7.86 (1H, brs), 8.28 (1H, m), 8.45 (1H, d, J = 5.6 Hz), 8.94 (1H, brs), 9.14 (1H, brs).
ESI-MS (m/z): 475 [M+Na]⁺.

### Alternative Method for Synthesis of 4-(3-Fluoro-4-{[1-(4-fluorophenylcarbamoyl)cyclopropanecarbonyl]amino}phenoxy)pyridine-2-carboxamide

To a solution of 1-(4-fluorophenylaminocarbonyl)cyclopropanecarboxylic acid (1.45 g) in tetrahydrofuran (14.5 ml) was added dropwise triethylamine (1.13 ml) under a nitrogen atmosphere while cooling in an ice water bath, followed by stirring for 15 min. To the reaction mixture was added thionyl chloride (0.473 ml), followed by stirring at the same temperature for 1.5 hr. To the reaction mixture were added a solution of 4-(4-amino-3-fluorophenoxy)pyridine-2-carboxamide (1.0 g) in tetrahydrofuran (10.5 ml) and triethylamine (1.13 ml) in this order at the same temperature under a nitrogen atmosphere, followed by stirring. The reaction mixture was allowed to warm up to room temperature and stirred overnight. The reaction mixture was partitioned after addition of ethyl acetate (50 ml) and a 2N aqueous solution of sodium hydroxide (10 ml). The organic layer was washed with a 2N aqueous solution of sodium hydroxide (10 ml, twice), 1N hydrochloric acid (10 ml, three times) and a saturated aqueous solution of sodium hydrogencarbonate (30 ml), and dried over anhydrous sodium sulfate. The solvent was concentrated under reduced pressure, and the residue was filtered (eluent; ethyl acetate) through silica gel column (Fuji Silysia NH). The filtrate was concentrated under reduced pressure, and to the resultant residue (1.28 g) were added ethyl acetate (4 ml) and heptane (4 ml) to suspend. The solid was collected by filtration and dried under aeration to provide the titled compound as a pale pink solid (991.1 mg, 54.1 %). The residue obtained by concentrating the filtrate under reduced pressure was purified by silica gel column chromatography (Fuji Silysia NH, eluent; ethyl acetate:heptane = 3:1). Fractions containing the target compound were concentrated under reduced pressure to provide the titled compound as a white solid (24.3 mg, 1.33 %).

### (Reference Example B-4) N-{4-[(2-Aminopyridin-4-yl)oxy]-2-fluorophenyl}-N'-(4-fluorophenyl)cyclopropane-1,1-dicarboxamide

4-(3-Fluoro-4-{[1-(4-fluorophenylcarbamoyl)cyclopropanecarbonyl]amino}phenoxy)pyridine-2-carboxamide (101 mg) was dissolved in N,N-dimethylformamide (1.0 ml) under a nitrogen atmosphere, and water (0.01 ml), [bis(trifluoroacetoxy)iodo]benzene (109 mg) and pyridine (0.0541 ml) were added at room temperature in this order, followed by stirring overnight. Water (0.01 ml), [bis(trifluoroacetoxy)iodo]benzene (109 mg) and pyridine (0.0541 ml) were added at room temperature in this order, followed by further stirring for 24 hr. The reaction mixture was partitioned between ethyl acetate and a 1N aqueous solution of sodium hydroxide. The organic layer was separated, washed with brine, and dried over anhydrous sodium sulfate. The solvent was concentrated under reduced pressure, and the residue was purified by silica gel column chromatography (Fuji Silysia NH, eluent; ethyl acetate). Fractions containing the target compound were concentrated under reduced pressure, and the residue was dried under reduced pressure to provide the titled compound as white foam (62.2 mg, 66 %).
¹H-NMR Spectrum (CDCl₃) δ (ppm): 1.50-1.90 (4H, m), 4.90 (2H, brs), 5.98 (1H, d, J = 2.4 Hz), 6.33 (1H, dd, J = 2.4, 5.6 Hz), 6.85-7.55 (6H, m), 7.90 (1H, d, J = 5.6 Hz), 8.20 (1H, m), 8.84 (1H, brs), 9.26 (1H, brs).
ESI-MS (m/z): 447 [M+Na]⁺.

### (Example 61) N-[4-({2-[(Azetidin-1-ylcarbonyl)amino]pyridin-4-yl}oxy)-2-fluorophenyl]-N'-(4-fluorophenyl)cyclopropane-1,1-dicarboxamide

N-{4-[(2-Aminopyridin-4-yl)oxy]-2-fluorophenyl}-N'-(4-fluorophenyl)cyclopropane-1,1-dicarboxamide (1.5 g) was dissolved in tetrahydrofuran (15 ml) under a nitrogen atmosphere, and triethylamine (0.987 ml) and phenyl chloroformate (0.978 ml) were added dropwise at room temperature in this order, followed by stirring for 30 min. The reaction mixture was stirred after addition of ethyl acetate and a saturated aqueous solution of sodium hydrogencarbonate. The organic layer was separated, washed with brine, and dried over anhydrous sodium sulfate. The solvent was concentrated under reduced pressure. The residue was dissolved in N,N-dimethylformamide (7.5 ml). Triethylamine (4.92 ml) and azetidine hydrochloride (1.33 g) were added at room temperature, followed by stirring for 7.5 hr. The reaction mixture was partitioned between ethyl acetate and a saturated aqueous solution of sodium hydrogencarbonate. The organic layer was washed with water (three times) and brine in this order, and dried over anhydrous sodium sulfate. The solvent was concentrated under reduced pressure. To the resultant residue were added ethyl acetate (5 ml) and heptane (5 ml) to precipitate a solid. The solid was collected by filtration and dried under aeration to provide the titled compound as white powder (1.29 g, 72 %).
¹H-NMR Spectrum (CDCl₃) δ (ppm): 1.60-1.80 (4H, m), 2.31 (2H, m), 4.11 (4H, m), 6.60 (1H, dd, J = 2.4, 5.6 Hz), 6.91-7.52 (7H, m), 7.74 (1H, d, J = 2.4 Hz), 8.01 (1H, d, J = 5.6 Hz), 8.24 (1H, m), 8.96 (1H, brs), 9.12 (1H, brs).
ESI-MS (m/z): 530 [M+Na]⁺.

### (Reference Example C-1) 1-(Benzyloxy)-2,5-difluoro-4-nitrobenzene

To a solution of 2,4,5-trifluoronitrobenzene (9.48 g) and benzyl alcohol (5.54 ml) in N,N-dimethylformamide (40 ml) was added potassium carbonate (11.1 g), followed by stirring at room temperature for 60 hr. To the reaction mixture was added water (120 ml) at 0 °C, followed by stirring at 4 °C for 24 hr. The precipitated crystals were collected by filtration and washed with water. These crystals were dried under reduced pressure to provide the titled compound as pale yellow crystals (11.5g, 81 %).
¹H-NMR Spectrum (DMSO-d₆) δ (ppm): 5.35 (2H, s), 7.40-7.50 (5H, m), 7.64 (1H, dd, J = 7.2, 13.2 Hz), 8.20 (1H, dd, J = 7.2, 10.8 Hz).

### (Reference Example C-2) 4-Amino-2,5-difluorophenol

To a solution of 1-(benzyloxy)-2,5-difluoro-4-nitrobenzene (9.21 g) in methanol (300 ml) was added 10 % palladium on carbon (921 mg), followed by stirring under a hydrogen atmosphere at room temperature for 24 hr and 20 min. The atmosphere in the reaction vessel was replaced with nitrogen to stop the reaction, and the catalyst was filtered through Celite. The filtrate was removed under reduced pressure to provide the titled compound as a brown solid (4.96 g, 99 %).
¹H-NMR Spectrum (DMSO-d₆) δ (ppm): 4.67 (1H, s), 6.53-6.64 (1H, m), 9.03 (1H, s).

### (Reference Example C-3) 4-(4-Amino-2,5-difluorophenoxy)pyridine-2-carboxamide

4-Amino-2,5-difluorophenol (4.95 g) was dissolved in dimethyl sulfoxide (50 ml) under a nitrogen flow, and potassium tert-butoxide (4.05 g) was added at room temperature, followed by stirring for 25 min. 4-Chloropyridine-2-carboxamide (2.70 g) was added thereto, followed by stirring at 80 °C for 2.5 hr. The reaction mixture was allowed to cool down to room temperature, and a 1N aqueous solution of sodium hydroxide (74.25 ml) was added, followed by stirring for 10 hr. The precipitated solid was collected by filtration, and the resultant solid was washed with water. This solid was dried under hot air at 100 °C for 24 hr to provide the titled compound as purple powder (3.38 g, 74 %).
¹H-NMR Spectrum (DMSO-d₆) δ (ppm): 5.57 (2H, d, J = 6.0 Hz), 6.75-6.80 (1H, m), 7.17-7.20 (1H, m), 7.26 (1H, dd, J = 7.2, 10.8 Hz), 7.38 (1H, m), 7.73 (1H, s), 8.14 (1H, s), 8.52 (1H, d, J = 5.6 Hz).
ESI-MS (m/z): 288 [M+Na]⁺.

### (Reference Example C-4) N-(4-{[2-Aminocarbonyl)pyridin-4-yl]oxy}-2,5-difluorophenyl)-N'(4-fluorophenyl)cyclopropane1,1-carboxamide

1-(4-Fluorophenylaminocarbonyl)cyclopropanecarboxylic acid (1.35 g) was dissolved in tetrahydrofuran (25.0 ml) under a nitrogen atmosphere, and triethylamine (1.06 ml) was added dropwise while cooling in an ice water bath, followed by stirring for 15 min. Then thionyl chloride (0.439 ml) was added at the same temperature, followed by stirring for 1.5 hr. To the reaction mixture was added dropwise at the same temperature a mixture of 4-(4-amino-2,5-difluorophenoxy)pyridine-2-carboxamide (1.0 g), tetrahydrofuran (12 ml) and triethylamine (1.06 ml), followed by stirring at 0 °C for 24 hr and 45 min. The reaction mixture was partitioned between ethyl acetate (70 ml) and a 2N aqueous solution of sodium hydroxide (15 ml). The organic layer was washed with a 2N aqueous solution of sodium hydroxide (15 ml) twice, 1N hydrochloric acid (15 ml) three times and a saturated aqueous solution of sodium hydrogencarbonate (10 ml) in this order, and dried over anhydrous sodium sulfate. The solvent was removed under reduced pressure. The resultant residue was purified by silica gel column chromatography (Fuji Silysia NH, eluent; heptane:ethyl acetate = 1:1, 1:2, then ethyl acetate), and fractions containing the target compound were concentrated under reduced pressure. The residue was dried under reduced pressure to provide the titled compound as a white solid (372.8 mg, 21 %).
¹H-NMR Spectrum (DMSO-d₆) δ (ppm): 1.28-1.33 (4H, m), 7.12-7.22 (2H, m), 7.22-7.28 (1H, m), 7.41 (1H, d, J = 2.4 Hz), 7.59-7.67 (3H, m), 7.75 (1H, m), 8.10-8.17 (2H, m), 8.56 (1H, d, J = 5.6 Hz), 9.80 (1H, m), 11.02 (1H, m).

### (Reference Example C-5) N-(4-{[2-(Aminopyridin-4-yl)oxy]-2,5-difluorophenyl}-N'-(4-fluorophenyl)cyclopropane-1,1-dicarboxamide

N-(4-{[2-(Aminocarbonyl)pyridin-4-yl]oxy}-2,5-difluorophenyl)-N'-(4-fluorophenyl)cyclopropane-1,1-dicarboxamide (372.8 mg) was dissolved in N,N-dimethylformamide (5.0 ml). Water (0.0713 ml), [bis(trifluoroacetoxy)iodo]benzene (679 mg) and pyridine (0.384 ml) were added thereto at room temperature in this order, followed by stirring for 3 hr. The reaction mixture was partitioned between ethyl acetate (30 ml) and a 1N aqueous solution of sodium hydroxide (9 ml). The organic layer was separated, washed with brine, and dried over anhydrous sodium sulfate. The solvent was concentrated under reduced pressure, and the residue was purified by silica gel column chromatography (Fuji Silysia NH, eluent; heptane:ethyl acetate = 1:3, then ethyl acetate). Fractions containing the target compound were concentrated under reduced pressure, and the residue was dried under reduced pressure to provide the titled compound as white powder (301.0 mg, 86 %).
¹H-NMR Spectrum (DMSO-d₆) δ (ppm): 1.54-1.68 (4H, m), 5.83 (1H, d, J = 2.4 Hz), 5.99 (2H, d, J = 5.2 Hz), 6.17 (1H, dd, J = 2.4, 5.6 Hz), 7.16-7.20 (2H, m), 7.47-7.53 (1H, m), 7.57-7.62 (2H, m), 7.81 (1H, d, J = 5.6 Hz), 8.02-8.10 (1H, m), 9.77 (1H, m), 10.99 (1H, m).
ESI-MS (m/z): 443 [M+H]⁺.

### (Example 91) N-{2,5-Difluoro-4-[(2-{[(3-hydroxyazetidin-1-yl)carbonyl]amino}pyridin-4-yl)oxy]phenyl}-N'-(4-fluorophenyl)cyclopropane-1,1-dicarboxamide

N-{4-[(2-Aminopyridin-4-yl)oxy]-2,5-difluorophenyl}-N'-(4-fluorophenyl)cyclopropane-1,1-dicarboxamide (100.0 mg) was dissolved in tetrahydrofuran (1 ml) under a nitrogen atmosphere, and triethylamine (0.0630 ml) and phenyl chloroformate (0.0624 ml) were added dropwise at 0 °C in this order, followed by stirring for 30 min. The reaction mixture was stirred after addition of ethyl acetate (5 ml) and a saturated aqueous solution of sodium hydrogencarbonate (5 ml). The organic layer was separated, washed with brine, and dried over anhydrous sodium sulfate. The solvent was concentrated under reduced pressure. The residue was dissolved in N,N-dimethylformamide (1.0 ml). 3-Hydroxyazetidine hydrochloride (99.0 mg) and triethylamine (0.315 ml) were added at room temperature, followed by stirring for 22 hr and 5 min. The reaction mixture was partitioned between ethyl acetate (10 ml) and a saturated aqueous solution of sodium hydrogencarbonate (5 ml). The organic layer was washed with brine, and dried over anhydrous sodium sulfate. The solvent was concentrated under reduced pressure. To the resultant residue were added ethyl acetate (1 ml) and heptane (1 ml) to precipitate a solid. The solid was collected by filtration. The resultant solid was purified by silica gel column chromatography (Fuji Silysia NH, eluent; ethyl acetate, then ethyl acetate:methanol = 10:1), and fractions containing the target compound were concentrated under reduced pressure to provide the titled compound as white powder (71.1 mg, 58 %).
¹H-NMR Spectrum (DMSO-d₆) δ (ppm): 1.55-1.68 (4H, m), 3.68 (2H, dd, J = 4.4, 8.4 Hz), 4.10-4.14 (2H, m), 4.34-4.40 (1H, m), 5.60 (1H, d, J = 6.4 Hz), 6.64 (1H, dd, J = 2.4, 5.6 Hz), 7.15-7.20 (2H, m), 7.50 (1H, d, J = 2.4 Hz), 7.52-7.62 (3H, m), 8.05-8.14 (1H, m), 8.13 (1H, d, J = 5.6 Hz), 9.20 (1H, s), 9.81 (1H, m), 10.99 (1H, m).
ESI-MS (neg.) (m/z): 540 [M-H]⁻.

### (Example 92) N-(2,5-Difluoro-4-{[2-({[4-(4-methylpiperazin-1-yl)piperidin-1-yl]carbonyl}amino)pyridin-4-yl]oxy}phenyl)-N'-(4-fluorophenyl)cyclopropane-1,1-dicarboxamide

N-{4-[(2-Aminopyridin-4-yl)oxy]-2,5-difluorophenyl}-N'-(4-fluorophenyl)cyclopropane-1,1-dicarboxamide (104.0 mg) was dissolved in tetrahydrofuran (1 ml) under a nitrogen atmosphere, and triethylamine (0.0653 ml) and phenyl chloroformate (0.0646 ml) were added dropwise at 0 °C in this order, followed by stirring for 30 min. The reaction mixture was stirred after addition of ethyl acetate (5 ml) and a saturated aqueous solution of sodium hydrogencarbonate (5 ml). The organic layer was separated, washed with brine, and dried over anhydrous sodium sulfate. The solvent was concentrated under reduced pressure. The residue was dissolved in N,N-dimethylformamide (1.0 ml). 1-Methyl-4-(piperidin-4-yl)piperazine (172.0 mg) was added at room temperature, followed by stirring at 20 hr and 40 min. The reaction mixture was partitioned between ethyl acetate (10 ml) and a saturated aqueous solution of sodium hydrogencarbonate (5 ml). The organic layer was washed with brine, and dried over anhydrous sodium sulfate. The solvent was concentrated under reduced pressure. To the resultant residue were added ethyl acetate (5 ml) and heptane (5 ml) to precipitate a solid. The solid was collected by filtration. The resultant solid was washed with heptane:ethyl acetate = 1:1, and dried under aeration to provide the titled compound as white powder (89.2 mg, 59 %).
¹H-NMR Spectrum (DMSO-d₆) δ (ppm): 1.12-1.32 (2H, m), 1.55-1.67 (4H, m), 1.67-1.74 (2H, m), 2.12 (3H, s), 2.20-2.65 (7H, m), 2.65-2.80 (4H, m), 4.05-4.15 (2H, m), 6.63 (1H, dd, J = 2.4, 5.6 Hz), 7.18 (2H, m), 7.39 (1H, d, J = 2.4 Hz), 7.52-7.62 (3H, m), 8.05-8.15 (1H, m), 8.13 (1H, d, J = 5.6 Hz), 9.24 (1H, s), 9.80 (1H, m), 10.99 (1H, m).
ESI-MS (m/z): 652 [M+H]⁺.

### (Example 94) N-(2,5-Difluoro-4-{[2-({[methyl(1-methylpiperidin-4-yl)amino]carbonyl}amino)pyridin-4-yl]oxy}phenyl)-N'-(4-fluorophenyl)cyclopropane-1,1-dicarboxamide

N-{4-[(2-Aminopyridin-4-yl)oxy]-2,5-difluorophenyl}-N'-(4-fluorophenyl)cyclopropane-1,1-dicarboxamide (94.0 mg) was dissolved in tetrahydrofuran (1 ml) under a nitrogen atmosphere, and triethylamine (0.0593 ml) and phenyl chloroformate (0.0587 ml) were added dropwise at 0 °C in this order, followed by stirring for 25 min. The reaction mixture was stirred after addition of ethyl acetate (5 ml) and a saturated aqueous solution of sodium hydrogencarbonate (5 ml). The organic layer was separated, washed with brine, and dried over anhydrous sodium sulfate. The solvent was concentrated under reduced pressure. The residue was dissolved in N,N-dimethylformamide (1.0 ml). 1-Methyl-4-(methylamino)piperidine (0.123 ml) was added at room temperature, followed by stirring for 18 hr and 35 min. The reaction mixture was partitioned between ethyl acetate (10 ml) and a saturated aqueous solution of sodium hydrogencarbonate (5 ml). The organic layer was washed with water (10 ml) twice and brine in this order, and dried over anhydrous sodium sulfate. The solvent was concentrated under reduced pressure. The resultant residue was purified by silica gel column chromatography (Fuji Silysia NH, eluent; ethyl acetate, then ethyl acetate:methanol = 10:1), and fractions containing the target compound were concentrated under reduced pressure to provide the titled compound as white powder (96.8 mg, 75 %).
¹H-NMR Spectrum (CDCl₃) δ (ppm): 1.61-1.83 (8H, m), 2.03-2.10 (2H, m), 2.28 (3H, s), 2.88 (3H, s), 2.90-2.94 (2H, m), 4.10-4.20 (1H, m), 6.55 (1H, dd, J = 2.4, 5.6 Hz), 6.98-7.08 (3H, m), 7.15 (1H, s), 7.46-7.50 (2H, m), 7.67 (1H, d, J = 2.4 Hz), 8.08 (1H, d, J = 5.6 Hz), 8.29 (1H, dd, J = 7.2, 12.0 Hz), 8.57 (1H, s), 9.59 (1H, s).
ESI-MS (m/z): 597 [M+H]⁺.

### (Reference Example F-1) 1-Benzhydryl-3-(methanesulfonyloxy)azetidine

A suspension of 1-benzhydrylazetidin-3-ol (15.0 g) in pyridine (100 ml) was cooled to -20 °C under a nitrogen atmosphere, and methanesulfonyl chloride (6.33 ml) was added dropwise thereto. The reaction mixture was stirred under a nitrogen atmosphere at -20 °C for 1 hr, then in a water bath for 2.5 days. The reaction mixture was partitioned after addition of water and ethyl acetate. The organic layer was washed with a saturated aqueous solution of sodium hydrogencarbonate, water and brine, and dried over anhydrous sodium sulfate. The solvent was concentrated under reduced pressure. To the residue were added ethanol (10 ml) and hexane (50 ml) to suspend precipitated crystals. The crystals were collected by filtration and washed with hexane. This was dried under aeration at room temperature to provide the titled compound as pale yellow crystals (5.943 g, 44.8 %). The filtrate was concentrated, and the residue was purified by silica gel column chromatography (eluent; heptane:ethyl acetate = 2:1, 1:1, then heptane:ethyl acetate:methanol = 50:50:1, 40:60:1, then ethyl acetate:methanol = 100:1). Fractions containing the target compound were concentrated to provide the titled compound as pale yellow crystals (1.58 g, 11.9 %).
¹H-NMR Spectrum (CDCl₃) δ (ppm): 2.99 (3H, s), 3.18-3.21 (2H, m), 3.62-3.66 (2H, m), 4.40 (1H, s), 5.11 (1H, m), 7.18-7.22 (2H, m), 7.26-7.31 (4H, m), 7.39 (4H, d, J = 7.2 Hz).

### (Reference Example F-2) 1-Benzhydryl-3-cyanoazetidine

To a solution of 1-benzhydryl-3-(methanesulfonyloxy)azetidine (7.52 g) in N,N-dimethylformamide (60 ml) were added water (7.2 ml) and sodium cyanide (3.48 g), followed by stirring at 65 °C for 9 hr. To the reaction mixture were added water, sodium carbonate and ethyl acetate, and this was partitioned. The aqueous layer was extracted with ethyl acetate. The organic layer was combined, washed with brine, and dried over anhydrous sodium sulfate. This was concentrated under reduced pressure, and the resultant crystals were suspended by addition of diethyl ether (10 ml). The crystals were collected by filtration and washed with diethyl ether. This was dried under aeration to provide the titled compound as pale yellow crystals (5.43 g, 92.3 %).
¹H-NMR Spectrum (CDCl₃) δ (ppm): 3.20-3.31 (3H, m), 3.47 (2H, m), 4.36 (1H, s), 7.19-7.23 (2H, m), 7.26-7.30 (4H, m), 7.39 (4H, m).

### (Reference Example F-3) 1-Benzhydrylazetidine-3-carboxylic acid

To a solution of 1-benzhydryl-3-cyanoazetidine (5.43 g) in methoxyethanol (54 ml) were added potassium hydroxide (6.48 g) and water (3.25 ml), followed by stirring at 100 °C for 4 hr. The reaction mixture was allowed to cool down to room temperature. The reaction mixture was poured into ice. After adjusting this to pH 5 with 1N hydrochloric acid, sodium chloride was added thereto. This was extracted with a mixed solvent of ethyl acetate and tetrahydrofuran. The organic layer was washed with brine, and dried over anhydrous sodium sulfate. The organic layer after drying was concentrated under reduced pressure to provide a crude product of the titled compound as pale yellow crystals. The crystals were suspended by addition of diethyl ether (15 ml). The crystals were collected by filtration and washed with diethyl ether. This was dried under aeration to provide the titled compound as pale yellow crystals (4.20 g, 71.7 %).
¹H-NMR Spectrum (CDCl₃) δ (ppm): 3.00-3.90 (5H, m), 4.95 (1H, s), 7.25-7.28 (2H, m), 7.33 (4H, m), 7.53 (4H, m).

### (Reference Example F-4) 1-Benzhydryl-3-(hydroxymethyl)azetidine

1-Benzhydryl-3-azetidinecarboxylic acid (3.12 g) was suspended in tetrahydrofuran (60 ml) and cooled under a nitrogen atmosphere in an ice-ethanol bath. Triethylamine (1.96 ml) was added dropwise, and a solution of ethyl chlorocarbonate (1.34 ml) in tetrahydrofuran (5 ml) was added dropwise over 20 min. After the dropwise addition, stirring was carried out at the same temperature for 30 min. The reaction mixture was filtered and washed with tetrahydrofuran (30 ml). The filtrate was added dropwise over 15 min to an aqueous (15 ml) solution of sodium borohydride (1.33 g) cooled in an ice water bath. Upon completion of the dropwise addition, the reaction mixture was stirred at room temperature. To the reaction mixture was gradually added 1N hydrochloric acid (35 ml) to decompose excess sodium borohydride, and a 1N aqueous solution of sodium hydroxide (35 ml) was added. This was extracted with ethyl acetate (100 ml). The organic layer was washed with brine, and dried over anhydrous sodium sulfate. The solvent was concentrated, and the residue was dried under reduced pressure to provide the titled compound as a pale brown solid (1.59 g, 54 %).
¹H-NMR Spectrum (CDCl₃) δ (ppm): 2.57 (1H, m), 3.03 (2H, m), 3.24 (2H, m), 3.80 (2H, d, J = 5.2 Hz), 4.33 (1H, s), 7.15-7.45 (10H, m).
ESI-MS (m/z):254[M+H]⁺.

### (Reference Example F-5) 3-(Hydroxymethyl)azetidine hydrochloride

1-Benzhydryl-3-(hydroxymethyl)azetidine (1.59 g) was dissolved in methanol (30 ml), and palladium hydroxide on carbon (1.0 g) was added under a nitrogen atmosphere, followed by stirring under a pressurized hydrogen atmosphere (0.4 MPa). The atmosphere in the reaction vessel was replaced with nitrogen, and the catalyst was filtered and washed with methanol. After addition of a 4N solution of hydrochloric acid in ethyl acetate (2 ml), concentration under reduced pressure was carried out. To the residue was added heptane (15 ml), and the supernatant was removed. This operation was repeated. The residue was dried under reduced pressure overnight to provide a crude product of the titled compound as a pale yellow oil (832 mg).
ESI-MS (m/z): 88 [M+H]⁺.

### (Example 96) N-(2,5-Difluoro-4-{[2-({[3-(hydroxymethyl)azetidin-1-yl]carbonyl}amino)pyridin-4-yl]oxy}phenyl)-N'-(4-fluorophenyl)cyclopropane-1,1-dicarboxamide

N-{4-[(2-Aminopyridin-4-yl)oxy]-2,5-difluorophenyl}-N'-(4-fluorophenyl)cyclopropane-1,1-dicarboxamide (108.2 mg) was dissolved in tetrahydrofuran (2.5 ml) under a nitrogen atmosphere, and triethylamine (0.100 ml) and phenyl chloroformate (0.080 ml) were added dropwise at room temperature in this order, followed by stirring for 15 min. The reaction mixture was stirred after addition of ethyl acetate and water. The organic layer was separated, washed with a 1N aqueous solution of sodium hydroxide, water and brine, dried over anhydrous sodium sulfate. The solvent was concentrated under reduced pressure. The residue was dissolved in N,N-dimethylformamide (2.5 ml). Triethylamine (0.256 ml) and 3-(hydroxymethyl)azetidine hydrochloride (182 mg) were added at room temperature, followed by stirring overnight. The reaction mixture was partitioned between ethyl acetate and water. The organic layer was washed with a 1N aqueous solution of sodium hydroxide, water and brine in this order, and dried over anhydrous sodium sulfate. The solvent was concentrated under reduced pressure. The resultant residue was purified by silica gel column chromatography (eluent; ethyl acetate, then ethyl acetate:methanol = 95:5). Fractions containing the target compound were concentrated under reduced pressure. To the resultant residue was added tert-butyl methyl ether:heptane = 1:2 to precipitate a solid. The solid was collected by filtration and dried under aeration to provide the titled compound as white powder (38.1 mg, 28 %).
¹H-NMR Spectrum (CDCl₃) δ (ppm): 1.50-1.80 (4H, m), 2.83 (1H, m), 3.80 (2H, d, J = 6.0 Hz), 3.93 (2H, m), 4.18 (2H, m), 6.57 (1H, dd, J = 2.4, 5.6 Hz), 6.95-7.10 (4H, m), 7.40-7.55 (2H, m), 7.78 (1H, d, J = 2.4 Hz), 7.99 (1H, d, J = 5.6 Hz), 8.33 (1H, m), 8.48 (1H, brs), 9.79 (1H, brs).
ESI-MS (m/z): 578 [M+Na]⁺,

### (Reference Example G-1) 6-(2-Fluoro-4-nitrophenoxy)pyrimidin-4-ylamine

2-Fluoro-4-nitrophenol (1.736 g) was dissolved in dimethyl sulfoxide (10 ml), and sodium hydride (400 mg) was added thereto, followed by stirring for 20 min. Then, 4-Amino-6-chloropyrimidine (648 mg) was added thereto and stirred at 100 °C for 45 min. The reaction mixture was heated up to 120 °C and stirred for 1 hr 25 min. The reaction mixture was then heated up to 140 °C and stirred overnight. The reaction mixture was allowed to cool down to room temperature, a 1N aqueous solution of sodium hydroxide (10 ml) was added thereto and stirred, then extracted with ethyl acetate. The organic layer was washed with a 1N aqueous solution of sodium hydroxide, water and brine in this order, and dried over anhydrous sodium sulfate. The solvent was concentrated under reduced pressure to give residue, which was purified by silica gel column chromatography (eluent; hexane:ethyl acetate = 1:2). The solvent was concentrated under reduced pressure, the resultant residue was suspended in diethyl ether (7 ml)-hexane (3.5 ml). The solid was collected by filtration and dried under aeration to provide the titled compound as pale brown powder (201 mg, 16.0 %).
¹H-NMR Spectrum (DMSO-d₆) δ (ppm): 6.02 (1H, m), 7.06 (2H, brs), 7.60 (1H, dd, J = 8.0, 8.8 Hz), 8.04 (1H, m), 8.10-8.19 (1H, m), 8.30 (1H, dd, J = 2.0, 10.0 Hz).

### (Reference Example G-2) [6-(2-Fluoro-4-nitrophenoxy)pyrimidin-4-yl]carbamic acid phenyl ester

6-(2-Fluoro-4-nitrophenoxy)pyrimidin-4-ylamine (1 g) was dissolved in tetrahydrofuran (40 ml) under a nitrogen atmosphere, and triethylamine (1.67 ml) and phenyl chloroformate (1.51 ml) were added thereto in an ice water bath. The reaction mixture was allowed to warm up to room temperature, and stirred for 1 hr. The reaction mixture was partitioned between ethyl acetate (200 ml) and a saturated aqueous solution of sodium hydrogencarbonate (100 ml). The organic layer was washed with a saturated aqueous solution of sodium hydrogencarbonate (100 ml), water (100 ml) and brine (100 ml) in this order, and dried over anhydrous sodium sulfate. To the resultant residue was added tetrahydrofuran (40 ml), and a 1N aqueous solution of sodium hydroxide (4 ml) was added while stirring in an ice water bath, followed by stirring for 30 min. The reaction mixture was allowed to warm up to room temperature and stirred for 1 hr. After addition of 1N hydrochloric acid (4 ml), the reaction mixture was partitioned between tetrahydrofuran (100 ml) and a saturated aqueous solution of sodium hydrogencarbonate (50 ml). The organic layer was washed with water (50 ml) and brine (100 ml) in this order and dried over anhydrous sodium sulfate. The solvent was removed under reduced pressure to give residue (4.3 g), to which was added ethyl acetate (20 ml), and allowed to stand for 4 days. The precipitated solid was collected by filtration and dried under aeration to provide the titled compound as pale yellow powder (399 mg, 26.9 %).
¹H-NMR Spectrum (CDCl₃) δ (ppm): 7.16-7.25 (2H, m), 7.25-7.35 (1H, m), 7.36-7.50 (3H, m), 7.72 (1H, m), 8.04-8.18 (2H, m), 8.50 (1H, m), 9.18 (1H, brs). ESI-MS (neg.) (m/z): 369 [M-H]⁻

### (Reference Example G-3) [6-(4-Amino-2-fluorophenoxy)pyrimidin-4-yl]carbamic acid phenyl ester

To a solution of 6-(2-fluoro-4-nitrophenoxy)pyrimidin-4-yl]carbamic acid phenyl ester (394 mg) in tetrahydrofuran (20 ml) was added 20% palladium hydroxide on carbon (149 mg), followed by stirring under a hydrogen atmosphere at room temperature for 15 hr. The catalyst was removed by filtration and washed with tetrahydrofuran. The solvent was removed under reduced pressure to provide a crude product of the titled compound as a white solid (303 mg).
ESI-MS (m/z): 341 [M+H]⁺, 363 [M+Na]⁺

### (Reference Example G-4) [6-(2-Fluoro-4{[1-4-fluorophenylcarbamoyl)cyclopropanecarbonyl]amino}phenoxy)pyrimidin-4-yl]carbamic acid phenyl ester

A crude product of [6-(4-amino-2-fluorophenoxy)pyrimidin-4-yl]carbamic acid phenyl ester (303 mg) was dissolved in N,N-dimethylformamide (5 ml). 1-(4-Fluorophenylcarbamoyl)cyclopropanecarboxylic acid (497 mg), triethylamine (0.310 ml) and benzotriazol-1-yloxytris(dimethylamino)phosphonium hexafluorophosphate (984 mg) were added in this order under a nitrogen atmosphere at room temperature, followed by stirring for 5 hr. Liquid-liquid separation was carried out after addition of ethyl acetate and a saturated aqueous solution of sodium hydrogencarbonate to the reaction mixture. The resultant organic layer was washed with brine and dried over anhydrous sodium sulfate. The solvent was removed under reduced pressure to give residue, which was purified by silica gel column chromatography (eluent; heptane:ethyl acetate = 2:3 to 1:1). Fractions containing the target compound were concentrated under reduced pressure, the resultant residue was purified again by silica gel column chromatography (eluent; heptane:ethyl acetate = 2:3 to 1:1). Fractions containing the target compound were concentrated under reduced pressure to provide the titled compound as white powder (100.4 mg).
¹H-NMR Spectrum (CDCl₃) δ (ppm): 1.30-1.80 (4H, m), 7.00-7.10 (2H, m), 7.10-7.35 (5H, m), 7.35-7.52 (4H, m), 7.58 (1H, s), 7.70 (1H, dd, J = 1.6, 12.0 Hz), 8.38 (1H, brs), 8.49 (1H, s), 8.69 (1H, brs), 9.57 (1H, brs).
ESI-MS (m/z): 568 [M+Na]⁺.

### (Example 33) N-(3-Fluoro-4-{[6-({[methyl(1-methylpiperidin-4-yl)amino]carbonyl}amino)pyrimidin-4-yl]oxy}phenyl)-N'-(4-fluorophenyl)cyclopropane-1,1-dicarboxamide

[6-(2-Fluoro-4-{[1-(4-fluorophenylcarbamoyl)cyclopropanecarbonyl]amino}phenoxy)pyrimidin-4-yl]carbamic acid phenyl ester (40 mg) was dissolved in N,N-dimethylformamide (1.0 ml), and 1-methyl-4-(methylamino)piperidine (0.045 ml) was added thereto, followed by stirring for 3 hr. The reaction mixture was partitioned between ethyl acetate (50 ml) and a saturated aqueous solution of ammonium chloride (20 ml). The organic layer was washed with a saturated aqueous solution of ammonium chloride (20 ml), water (20 ml) and brine (20 ml) in this order, and dried over anhydrous sodium sulfate. The solvent was removed under reduced pressure, and the resultant residue was purified by silica gel column chromatography (Fuji Silysia NH, eluent; ethyl acetate, then ethyl acetate:methanol = 95:5). Fractions containing the target compound were concentrated under reduced pressure, and the resultant residue was suspended in diethyl ether (2 ml) and hexane (4 ml). The solid was collected by filtration and dried under aeration to provide the titled compound as white powder (33.7 mg, 79.3 %).
¹H-NMR Spectrum (CDCl₃) δ (ppm): 1.50-1.75 (6H, m), 1.75-1.90 (2H, m), 2.06-2.17 (2H, m), 2.30 (3H, s), 2.92 (3H, s), 2.96 (2H, m), 4.10-4.25 (1H, m), 7.05 (2H, m), 7.12-7.24 (2H, m), 7.31 (1H, brs), 7.40-7.50 (2H, m), 7.65 (1H, m), 7.68 (1H, dd, J = 2.0, 12.0 Hz), 8.34 (1H, m), 8.49 (1H, brs), 9.48 (1H, brs).
ESI-MS (m/z): 602 [M+Na]⁺.

### (Reference example H-1) Benzyl (2,5-difluoro-4-hydroxyphenyl)carbamate

1-(Benzyloxy)-2,5-difluoro-4-nitrobenzene (5.3 g) was dissolved in methanol (100 ml) - tetrahydrofuran (100 ml). 20 % palladium hydroxide on carbon (2.81 g) was added thereto, followed by stirring under a hydrogen atmosphere at room temperature for 8 hr. The catalyst was removed by filtration and washed with methanol. The filtrate was concentrated under reduced pressure. The resultant residue (3.06 g) was dissolved in acetone (100 ml) - water (50 ml). Sodium carbonate (2.02 g) and benzyl chloroformate (3.43 ml) were added thereto while stirring and cooling in an ice water bath, followed by stirring at room temperature for 1 hr. The reaction mixture was concentrated under reduced pressure. The residue was partitioned between ethyl acetate and brine. The organic layer was separated and concentrated under reduced pressure. The resultant residue was purified by silica gel column chromatography (eluent; heptane:ethyl acetate = 2:1). Fractions containing the target compound were concentrated under reduced pressure and the residue was dried under reduced pressure to provide the titled compound as a brown solid (4.90 g, 88 %).
ESI-MS (neg.) (m/z): 278 [M-H]⁻.

### (Reference example H-2) Benzyl [4-(4-chloropyrimidin-6-yloxy)-2,5-difluorophenyl]carbamate

Benzyl (2,5-difluoro-4-hydroxyphenyl)carbamate (4.90 g) was dissolved in N,N-dimethylformamide (30 ml), then 4,6-dichloropyrimidine (2.61 g) and potassium carbonate (3.63 g) were added thereto at room temperature, followed by stirring for 2 hr. Water (90 ml) was added to the reaction mixture to precipitate crystals. The crystals were collected by filtration and washed with water (30 ml, 6 times). The crystals were hot air-dried at 60 °C for 2 days to provide the titled compound as pale brown crystals (6.108 g, 89 %).
¹H-NMR Spectrum (CDCl₃) δ (ppm): 5.25 (2H, s), 6.95 (1H, brs), 7.01 (1H, m), 7.04 (1H, d, J = 0.8 Hz), 7.30-7.50 (5H, m), 8.16 (1H, m), 8.56 (1H, d, J = 0.8 Hz).
ESI-MS (neg.) (m/z): 390 [M-H]⁻.

### (Reference example H-3) Benzyl [4-(4-aminopyrimidin-6-yloxy)-2,5-difluorophenyl]carbamate

A mixture of benzyl [4-(4-chloropyrimidin-6-yloxy)-2,5-difluorophenyl]carbamate (3.92 g) and 2M ammonia - isopropanol (50 ml) was heated at 120 °C for 2 days in a sealed tube. The reaction mixture was allowed to cool to room temperature, then concentrated under reduced pressure. The resultant residue was partitioned between ethyl acetate and a 10 % aqueous solution of potassium bisulfate. The organic layer was washed with brine, dried over anhydrous sodium sulfate. The solvent was concentrated under reduced pressure and the resultant residue was purified by silica gel column chromatography (Fuji Silysia NH, eluent; heptane:ethyl acetate = 1:2). Fractions containing the target compound were concentrated under reduced pressure and the residue was dried under reduced pressure to provide the titled compound as pale yellow crystals (561 mg, 15 %).
¹H-NMR Spectrum (CDCl₃) δ (ppm): 4.94 (2H, br), 5.23 (2H, s), 5.97 (1H, d, J = 0.8 Hz), 6.91 (1H, brs), 6.99 (1H, m), 7.30-7.50 (5H, m), 8.10 (1H, m), 8.24 (1H, d, J = 0.8 Hz).
ESI-MS (m/z): 395 [M+Na]⁺.

### (Reference example H-4) Benzyl [4-(4-azidopyrimidin-6-yloxy)-2,5-difluorophenyl]carbamate

Benzyl [4-(4-chloropyrimidin-6-yloxy)-2,5-difluorophenyl]carbamate (1.96 g) was dissolved in N,N-dimethylformamide (20 ml). Sodium azide (650 mg) was added thereto, followed by stirring at 60 °C for 2 hr. The reaction mixture was allowed to cool to room temperature, then partitioned between ethyl acetate and water. The organic layer was washed with brine, dried over anhydrous sodium sulfate. The solvent was concentrated under reduced pressure and the resultant residue was purified by silica gel column chromatography (eluent; heptane:ethyl acetate = 3.1). Fractions containing the target compound were concentrated under reduced pressure and the residue was dried under reduced pressure to provide the titled compound as white crystals (685 mg, 34 %).
¹H-NMR Spectrum (CDCl₃) δ (ppm): 5.24 (2H, s), 6.40 (1H, d, J = 0.8 Hz), 6.93 (1H, brs), 6.99 (1H, dd, J = 7.2, 10.0 Hz), 7.30-7.50 (5H, m), 8.13 (1H, m), 8.51 (1H, d, J = 0.8 Hz).

### (Reference example H-5) 4-Amino-6-(4-amino-2,5-difluorophenoxy)pyrimidine

### Production method - 1

4-Amino-2,5-difluorophenol (2.15 g) was dissolved in dimethyl sulfoxide (12.5 ml) at room temperature under a nitrogen flow. Potassium tert-butoxide (1.66 g) was added thereto, followed by stirring at room temperature for 5 min. 4-Amino-6-chloropyrimidine (1.55 g) was added, and the resultant mixture was stirred at 100 °C for 18.5 hr under a nitrogen flow. The reaction mixture was allowed to cool to room temperature, then partitioned between ethyl acetate (100 ml) and a 1N aqueous solution of sodium hydroxide (50 ml). The organic layer was washed with a 2N aqueous solution of sodium hydroxide (50 ml, 3 times) and brine (50 ml). The solvent was concentrated under reduced pressure and the resultant residue was purified by silica gel column chromatography (Fuji Silysia NH, eluent; heptane:ethyl acetate = 1:2). Fractions containing the target compound were concentrated under reduced pressure and the residue was dried under reduced pressure to provide the titled compound as pale yellow powder (271 mg, 9.5 %).
¹H-NMR Spectrum (CDCl₃) δ (ppm): 3.76 (2H, br), 4.97 (2H, br), 5.94 (1H, d, J = 0.8 Hz), 6.60(1H, dd, J = 8.0, 11.2 Hz), 6.87 (1H, dd, J = 7.2, 11.2 Hz), 8.26 (1H, d, J = 0.8 Hz).
ESI-MS (m/z): 239 [M+H]⁺.

### Production method - 2

Benzyl [4-(4-aminopyrimidin-6-yloxy)-2,5-difluorophenyl]carbamate (561 mg) was dissolved in methanol (30 ml). 10 % palladium on carbon (321 mg) was added, followed by stirring under a hydrogen atmosphere for 4 hr. The catalyst was filtered off and washed with methanol. The filtrate was concentrated under reduced pressure and the residue was dried under reduced pressure to provide the titled compound as pale yellow powder (360 mg, quantitative).

### Production method - 3

Benzyl [4-(4-azidopyrimidin-6-yloxy)-2,5-difluorophenyl]carbamate (684 mg) was dissolved in methanol (20 ml) - tetrahydrofuran (20 ml). 10 % palladium on carbon (366 mg) was added, followed by stirring under a hydrogen atmosphere for 5 hr. The catalyst was filtered off and washed with methanol. The filtrate was concentrated under reduced pressure and the residue was dried under reduced pressure to provide the titled compound as pale yellow powder (373 mg, 91 %).

### (Reference example H-6) N-{4-[(4-Aminopyrimidin-6-yl)oxy]-2,5-difluorophenyl}-N'-(4-fluorophenyl)cyclopropane-1,1-dicarboxamide

To a solution of 1-(4-fluorophenylaminocarbonyl)cyclopropanecarboxylic acid (378 mg) in N,N-dimethylformamide (3 ml) were added triethylamine (0.236 ml) and HATU (644 mg) at room temperature under a nitrogen atmosphere, followed by stirring for 30 min. To the resultant mixture was added 4-amino-6-(4-amino-2,5-difluorophenoxy)pyrimidine (270 mg) in N,N-dimethylformamide (3 ml) at room temperature, followed by stirring for 6 hr. Triethylamine (0.079 ml) and HATU (215 mg) were added again and the resultant mixture was stirred overnight. The reaction mixture was partitioned between ethyl acetate (20 ml) and a 1N aqueous solution of sodium hydroxide (10 ml). The organic layer was washed with a 1N aqueous solution of sodium hydroxide (10 ml, twice) and brine (10 ml), dried over anhydrous sodium sulfate. The solvent was concentrated under reduced pressure and the resultant residue was purified by silica gel column chromatography (Fuji Silysia NH, eluent; heptane:ethyl acetate = 1:2 to 1:4). Fractions containing the target compound were concentrated under reduced pressure and the residue was dried under reduced pressure to provide the titled compound as pale brown powder (199 mg, 40 %).
¹H-NMR Spectrum (CDCl₃) δ (ppm): 1.60-1.80 (4H, m), 4.99 (2H, br), 6.00 (1H, s), 7.00-7.50 (5H, m), 8.24 (1H, s), 8.26 (1H, m), 8.59 (1H, brs), 9.54 (1H, brs).
ESI-MS (m/z): 466 [M+Na]⁺.

### (Example 97) N-{2,5-Difluoro-4-[(4-{[(3-hydroxyazetidin-1-yl)carbonyl]amino}pyrimidin-6-yl)oxy]phenyl}-N'-(4-fluorophenyl)cyclopropane-1,1-dicarboxamide

N-{4-[(4-Aminopyrimidin-6-yl)oxy]-2,5-difluorophenyl}-N'-(4-fluorophenyl)cyclopropane-1,1-dicarboxamide (100 mg) was dissolved in tetrahydrofuran (5 ml) under a nitrogen atmosphere, triethylamine (0.080 ml) and phenyl chloroformate (0.070 ml) were added dropwise at room temperature, followed by stirring for 10 min. The reaction mixture was partitioned between ethyl acetate and water. The organic layer was separated, washed with a 1N aqueous solution of sodium hydroxide and brine, dried over anhydrous sodium sulfate. The solvent was concentrated under reduced pressure. The residue was dissolved in N,N-dimethylformamide (2.5 ml). To the solution were added 3-hydroxyazetidine hydrochloride (150 mg) and triethylamine (0.250 ml) at room temperature, followed by stirring for 63 hr. The reaction mixture was partitioned between ethyl acetate and water. The organic layer was separated, washed with a 1N aqueous solution of sodium hydroxide and brine, dried over anhydrous sodium sulfate. The solvent was concentrated under reduced pressure. The resultant residue was purified by silica gel column chromatography (Fuji Silysia NH, eluent; ethyl acetate, then ethyl acetate:methanol = 95:5), and fractions containing the target compound were concentrated under reduced pressure. To the resultant residue was added diethyl ether:heptane = 1:2 to precipitate a solid. The solid was collected by filtration and dried under aeration to provide the titled compound as white powder (57.3 mg, 47 %).
¹H-NMR Spectrum (CDCl₃) δ (ppm): 1.60-1.80 (4H, m), 2.27 (1H, m), 4.00 (2H, m), 4.37 (2H, m), 4.75 (1H, m), 6.90-7.10 (4H, m), 7.40-7.55 (2H, m), 7.66 (1H, s), 8.28 (1H, dd, J = 7.2, 12.0 Hz), 8.34 (1H, s), 8.66 (1H, brs), 9.50 (1H, brs).
ESI-MS (m/z): 565 [M+Na]⁺.

### (Example 99) N-(2,5-Difluoro-4-{[4-({[3-(hydroxymethyl)azetidin-1-yl]carbonyl}amino)pyrimidin-6-yl]oxy}phenyl)-N'-(4-fluorophenyl)cyclopropane-1,1-dicarboxamide

N-{4-[(4-Aminopyrimidin-6-yl)oxy]-2,5-difluorophenyl}-N'-(4-fluorophenyl)cyclopropane-1,1-dicarboxamide (100 mg) was dissolved in tetrahydrofuran (7.5 ml) under a nitrogen atmosphere, triethylamine (0.180 ml) and phenyl chloroformate (0.150 ml) were added dropwise at room temperature, followed by stirring for 50 min. The reaction mixture was partitioned between ethyl acetate and water. The organic layer was separated, washed with a 1N aqueous solution of sodium hydroxide and brine, dried over anhydrous sodium sulfate. The solvent was concentrated under reduced pressure. The residue was dissolved in N,N-dimethylformamide (2.5 ml). To the solution were added triethylamine (0.400 ml) and 3-(hydroxymethyl)azetidine hydrochloride (280 mg) at room temperature, followed by stirring overnight. The reaction mixture was partitioned between ethyl acetate and water. The organic layer was separated, washed with a 1N aqueous solution of sodium hydroxide and brine, dried over anhydrous sodium sulfate. The solvent was concentrated under reduced pressure. The resultant residue was purified by silica gel column chromatography (eluent; ethyl acetate, then ethyl acetate:methanol = 95:5), and fractions containing the target compound were concentrated under reduced pressure. To the resultant residue was added tert-butyl methyl ether:heptane = 1:2 to precipitate a solid. The solid was collected by filtration and dried under aeration to provide the titled compound as white powder (15.6 mg, 12 %).
¹H-NMR Spectrum (CDCl₃) δ (ppm): 1.60-1.80 (4H, m), 2.83 (1H, m), 3.82 (2H, d, J = 6.0 Hz), 3.93 (2H, m), 4.16 (2H, m), 6.90-7.15 (4H, m), 7.40-7.55 (2H, m), 7.66 (1H, s), 8.22 (1H, dd, J = 7.2, 12.0 Hz), 8.33 (1H, s), 8.73 (1H, brs), 9.60 (1H, brs).
ESI-MS (m/z): 579 [M+Na]⁺.

### (Example 100) N-(2,5-Difluoro-4-{[4-({[methyl(1-methylpiperidin-4-yl)amino]carbonyl}amino)pyrimidin-6-yl]oxy}phenyl)-N'-(4-fluorophenyl)cyclopropane-1,1-dicarboxamide

N-{4-[(4-Aminopyrimidin-6-yl)oxy]-2,5-difluorophenyl}-N'-(4-fluorophenyl)cyclopropane-1,1-dicarboxamide (100 mg) was dissolved in tetrahydrofuran (7.5 ml) under a nitrogen atmosphere, triethylamine (0.180 ml) and phenyl chloroformate (0.150 ml) were added dropwise at room temperature, followed by stirring for 50 min. The reaction mixture was partitioned between ethyl acetate and water. The organic layer was separated, washed with a 1N aqueous solution of sodium hydroxide and brine, dried over anhydrous sodium sulfate. The solvent was concentrated under reduced pressure. The residue was dissolved in N,N-dimethylformamide (2.5 ml). To the solution was added 1-methyl-(4-methylamino)piperidine (0.330 ml) at room temperature, followed by stirring overnight. The reaction mixture was partitioned between ethyl acetate and water. The organic layer was separated, washed with a 1N aqueous solution of sodium hydroxide and brine, dried over anhydrous sodium sulfate. The solvent was concentrated under reduced pressure. The resultant residue was purified by silica gel column chromatography (Fuji Silysia NH, eluent; ethyl acetate, then ethyl acetate:methanol = 95:5), and fractions containing the target compound were concentrated under reduced pressure. To the resultant residue was added tert-butyl methyl ether:heptane = 1:2 to precipitate a solid. The solid was collected by filtration and dried under aeration to provide the titled compound as white powder (19.5 mg, 14 %).
¹H-NMR Spectrum (CDCl₃) δ (ppm): 1.60-1.80 (8H, m), 2.20-2.60 (2H, m), 2.96 (3H, s), 3.00-3.30 (2H, m), 3.22 (3H, s), 4.33 (1H, m), 6.90-7.15 (4H, m), 7.40-7.55 (2H, m), 7.66 (1H, s), 8.27 (1H, dd, J = 7.2, 12.0 Hz), 8.35 (1H, s), 8.62 (1H, brs), 9.53 (1H, brs).
ESI-MS (m/z): 620 [M+Na]⁺.

### (Example 101) N-(2,5-Difluoro-4-{[4-({[4-(4-methylpiperazin-1-yl)piperidin-1-yl]carbonyl}amino)-pyrimidin-6-yl]oxy}phepyl)-N'-(4-fluorophenyl)cyclopropane-1,1-dicarboxamide

N-{4-[(4-Aminopyrimidin-6-yl)oxy]-2,5-difluorophenyl}-N'-(4-fluorophenyl)cyclopropane-1,1-dicarboxamide (100 mg) was dissolved in tetrahydrofuran (5 ml) under a nitrogen atmosphere, N,N-diisopropylethylamine (0.100 ml) and phenyl chloroformate (0.070 ml) were added dropwise at room temperature, followed by stirring for 15 min. The reaction mixture was partitioned between ethyl acetate and water. The organic layer was separated, washed with a saturated aqueous solution of sodium hydrogencarbonate and brine, dried over anhydrous sodium sulfate. The solvent was concentrated under reduced pressure. The residue was dissolved in N,N-dimethylformamide (2.5 ml). To the solution was added 1-methyl-4-(piperidin-4-yl)piperazine (250 mg) at room temperature, followed by stirring for 25 hr. The reaction mixture was partitioned between ethyl acetate and water. The organic layer was separated, washed with a 1N aqueous solution of sodium hydroxide and brine, dried over anhydrous sodium sulfate. The solvent was concentrated under reduced pressure. The resultant residue was purified by silica gel column chromatography (Fuji Silysia NH, eluent; ethyl acetate, then ethyl acetate:methanol = 95:5), and fractions containing the target compound were concentrated under reduced pressure. To the resultant residue was added diethyl ether:heptane = 1:2 to precipitate a solid. The solid was collected by filtration and dried under aeration to provide the titled compound as white powder (93.4 mg, 63 %).
¹H-NMR Spectrum (CDCl₃) δ (ppm): 1.45-1.60 (2H, m), 1.66-1.76 (4H, m), 1.90-1.98 (2H, m), 2.34 (3H, s), 2.42-2.72 (9H, m), 2.95 (2H, m), 4.12 (2H, m), 7.00-7.10 (3H, m), 7.38 (1H, brs), 7.44-7.55 (2H, m), 7.62 (1H, s), 8.27 (1H, dd, J = 6.8, 12.0 Hz), 8.33 (1H, s), 8.67 (1H, brs), 9.47 (1H, brs).
ESI-MS (m/z): 653 [M+H]⁺.

Other compounds were synthesized by the reaction similar to the above Examples. Chemical formulas of the synthesized compounds including the above Examples are shown in tables 1 to 6.

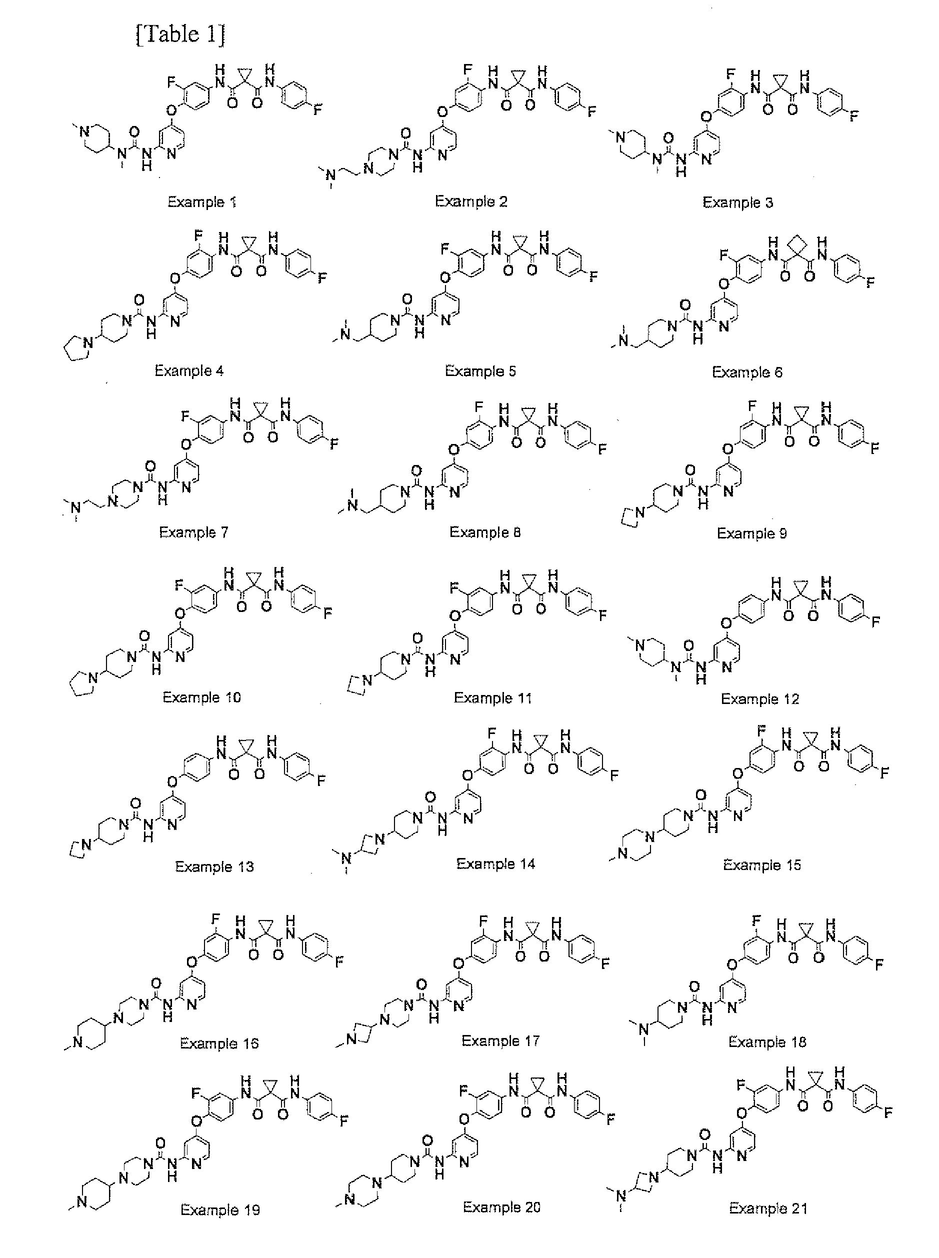

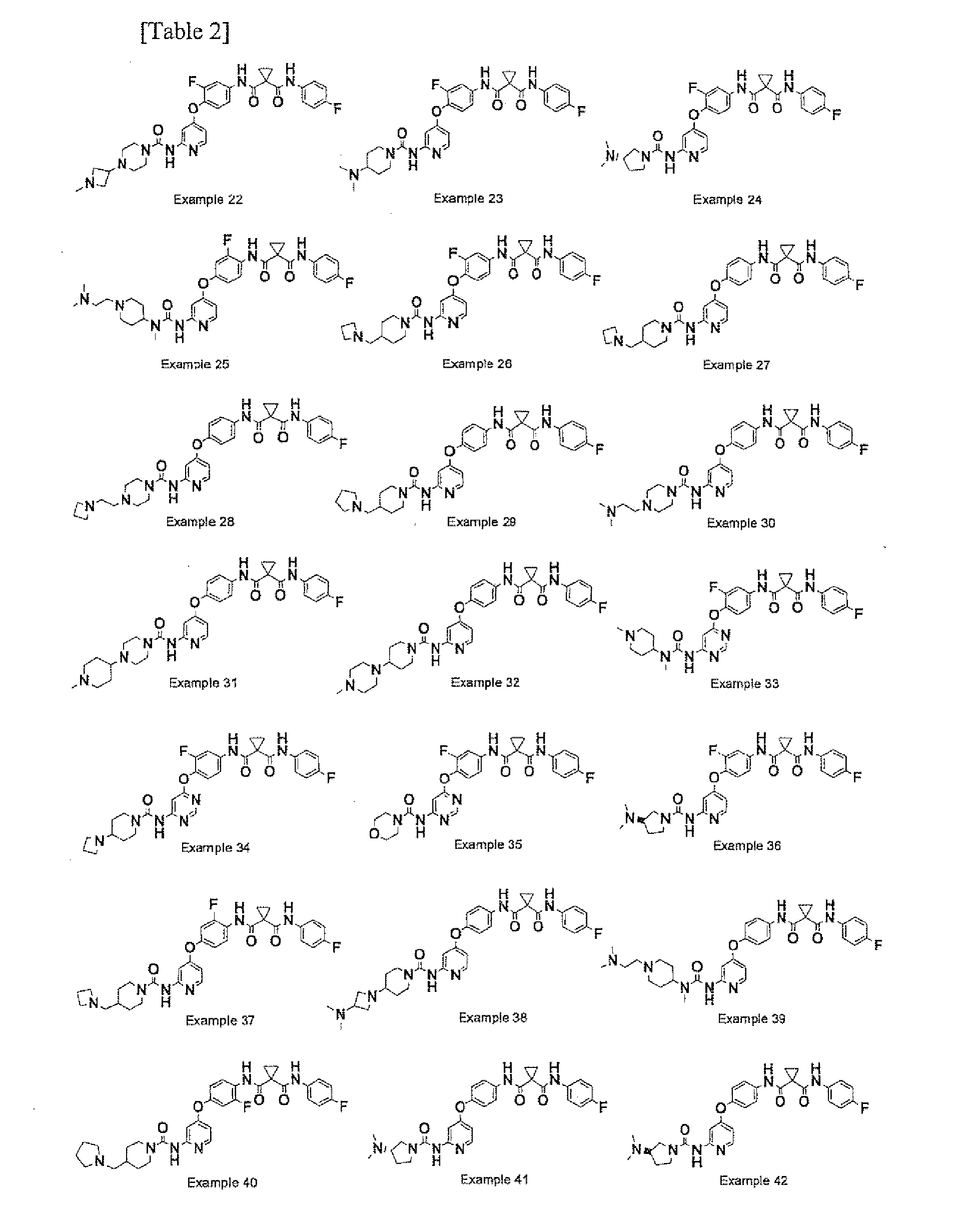

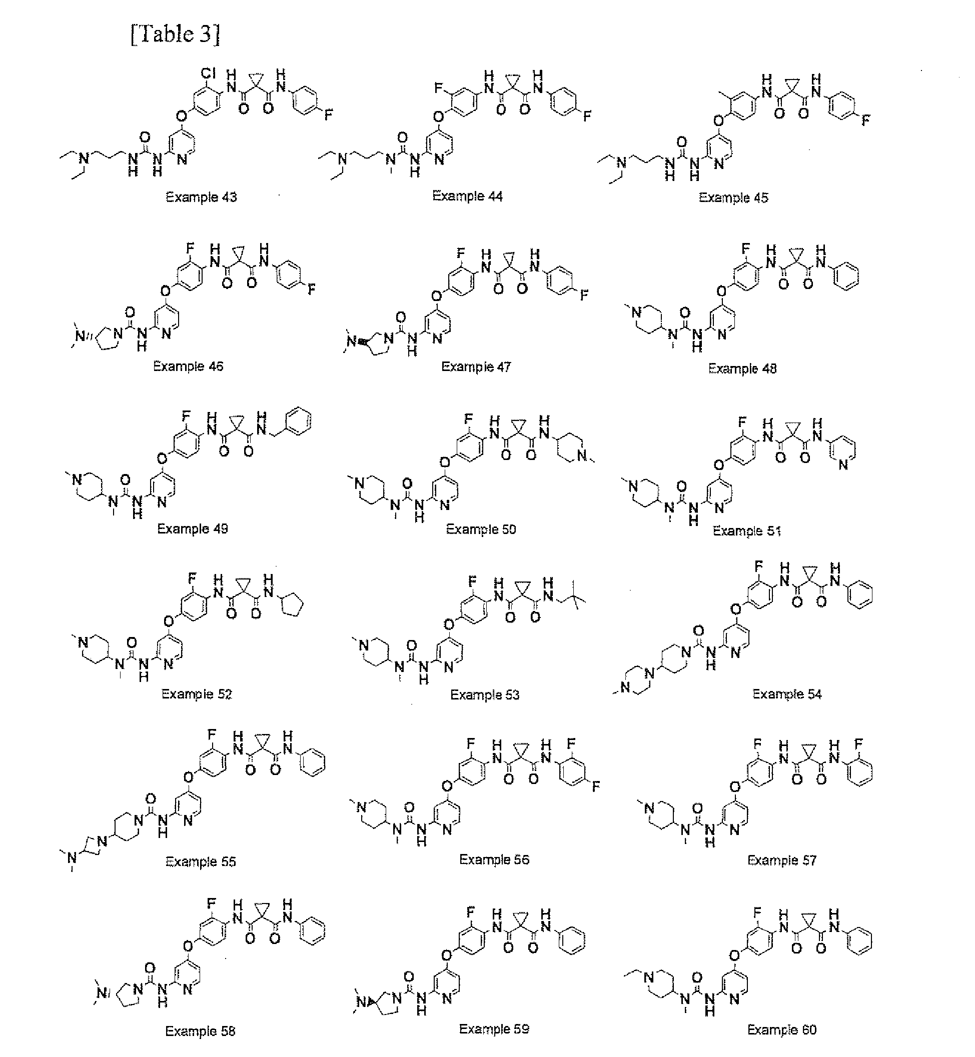

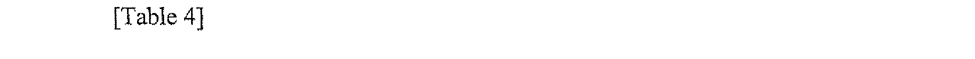

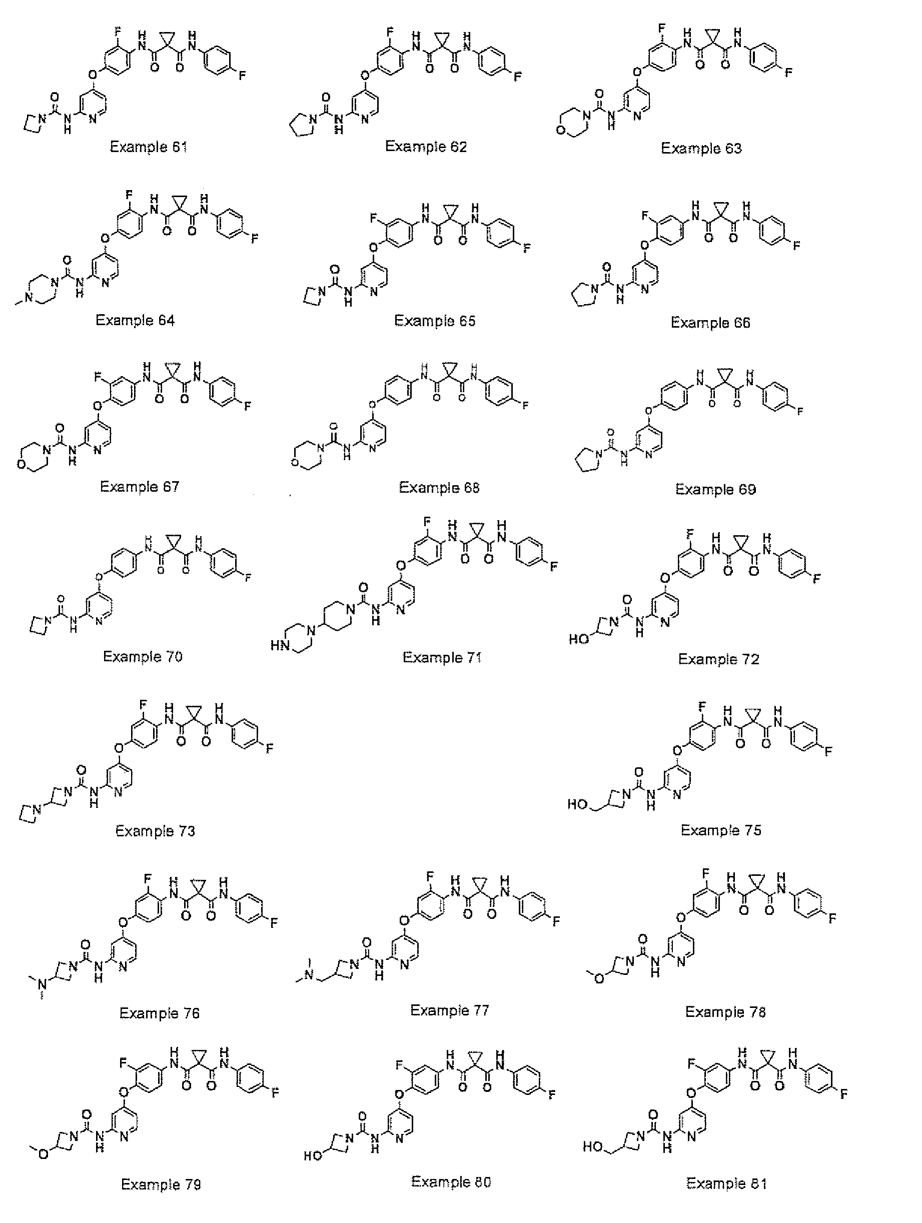

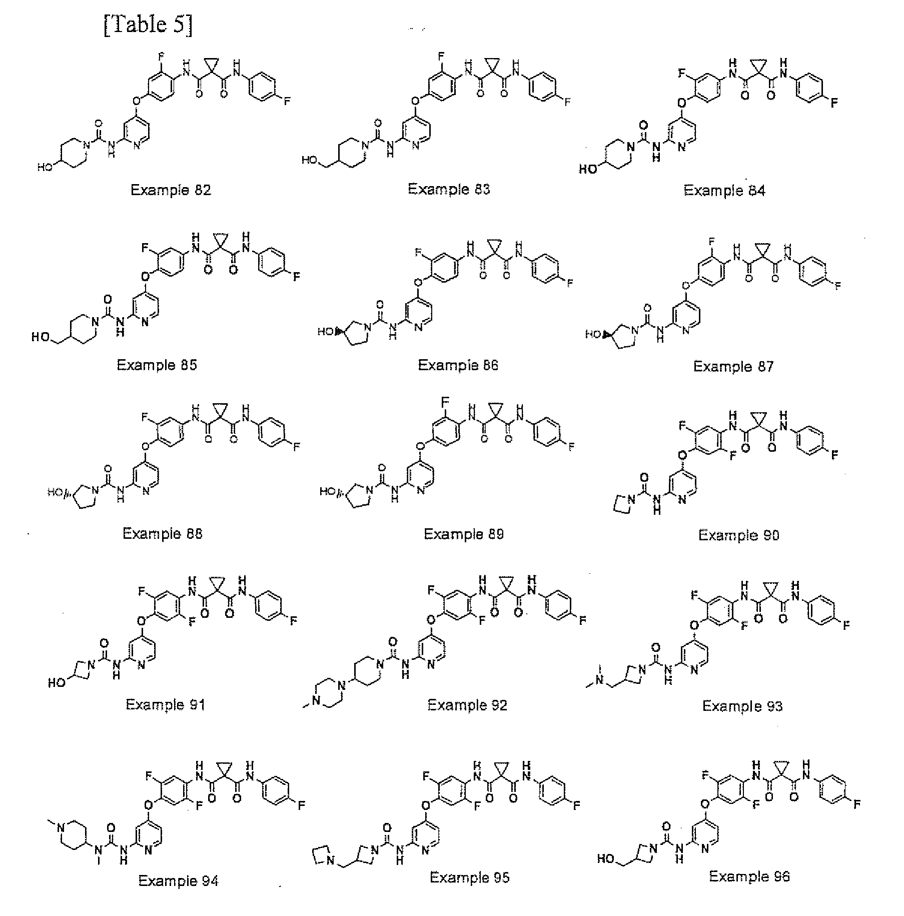

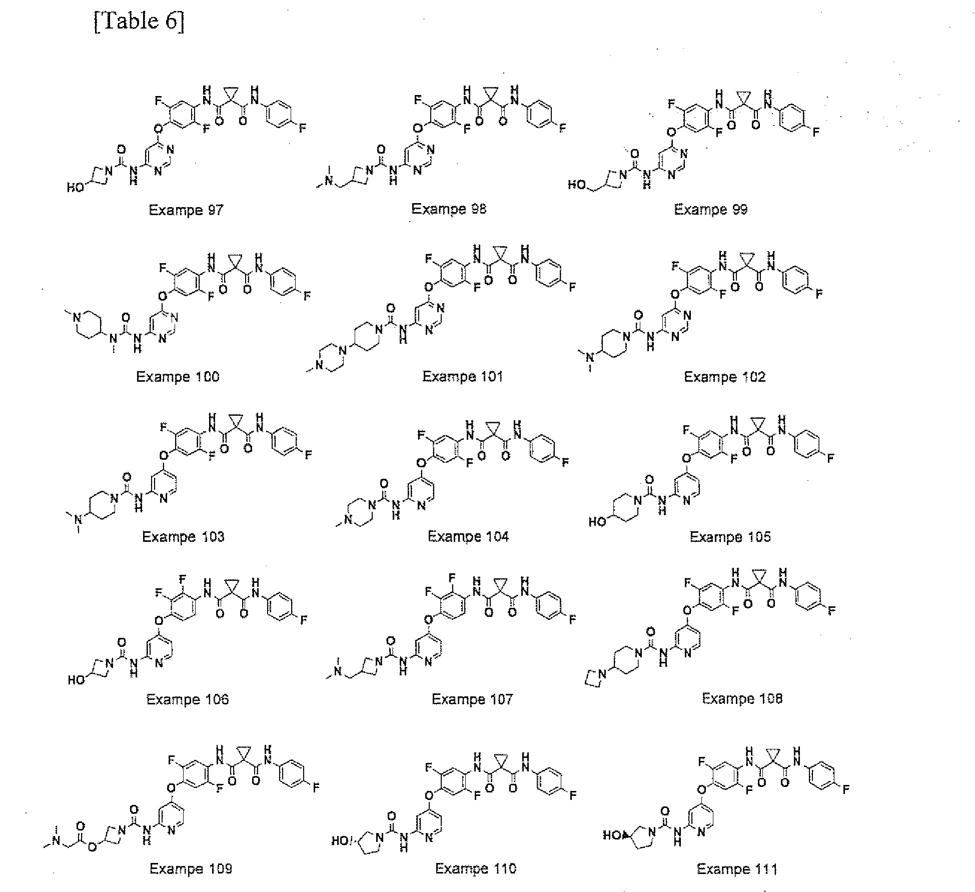

### Pharmacological Test Examples

The biological activity and pharmaceutical effect (inhibitory activity for hepatocyte growth factor receptor) of the pyridine or pyrimidine derivative according to the present invention were evaluated by methods described below.
Abbreviations and terms used in the following Pharmacological Test Examples are listed as follows:
(Abbreviation List)
HGFR (Hepatocyte growth factor receptor)
DNA (Deoxyribonucleic acid)
PCR (Polymerase chain reaction)
FBS (Fetal bovine serum)
PBS (Phosphate buffered saline)
Tris (Tris(hydroxymethyl)aminomethane, Tris(buffer))
PMSF (Phenylmethylsulfonyl fluoride)
NP-40 (Nonidet P-40)
EGTA (O,O-Bis(2-aminoethyleneglycol)-N,N,N',N'-tetraacetic acid)
SDS (Sodium dodecyl sulfate)
BSA (Bovine serum albumin)
Hepes (N-[2-hydroxyethyl]piperazine-N'-[2-ethanesulfonic acid], Hepes(buffer))
ATP (Adenosine 5'-triphosphate)
EDTA (Ethylenediamine tetraacetic acid)
HTRF (Homogenous Time-Resolved Fluorescence)
HRP (Horseradish peroxidase)
ELISA (Enzyme-linked immunosorbent assay)
HGF (Hepatocyte growth factor)
HBSS (Hank's Balanced Salt solution)
MTT (3-[4,5-dimethylthiazol-2-yl]-2,5-diphenyltetrazolium bromide; Thiazolyl blue)
EGM-2 (Endothelial Cell Growth Medium-2)
FISH (Fluorescence in situ hybridization)
BAC (Bacterial Artificial Chromosome)
ssDNA (salmon sperm DNA)
RNase (Ribonuclease)

### Pharmacological Test Example 1: Inhibitory activity against HGFR tyrosine kinase activity

### 1. Cloning of HGFR tyrosine kinases, and preparation of the recombinant baculovirus solutions

The cytoplasmic domain of HGFR (Genbank Accession No. J02958) is a 1.3kb DNA fragment beginning with Lys974 and including a stop codon, and described by Park et al. (Proc. Natl. Acad. Sci. U.S.A. 84(18), 6379-6383, 1987). The DNA fragment was isolated from the human placental cDNA library (purchased from Clontech) by PCR (TaKaRa Ex Taq^{™} Kit, purchased from TaKaRa) using two kinds of primers (SEQ ID NO: 1, 5'-CCGGCCGGATCCAAAAAGAGAAAGCAAATTAAA-3' and SEQ ID NO: 2, 5'-TTAATTCTGCAGCTATGATGTCTCCCAGAAGGA-3', purchased from Invitrogen). The DNA fragment was cloned into a baculovirus transplace vector (pFastBac^{™}-HT (purchased from GIBCO BRL)) to produce a recombinant construct. The construct was transfected into insect cells (*Spodoptera frugiperda* 9(Sf9)) to produce a solution of HGFR transfected baculovirus (preparation of a recombinant baculovirus can be found in the standard text (Bac-to-Bac Baculovirus Expression System (GIBCO BRL)).

### 2. Expression and purification of HGFR tyrosine kinases

To the suspension of Sf9 cells (3×10⁸ cells) in SF-900II medium (purchased from Invitrogen) containing 2% FBS was added a solution of HGFR transfected baculovirus above (4 ml), followed by a shaking culture at 27 °C for 48 hrs. The cells infected with the HGFR transfected baculovirus were centrifuged at 1,000 rpm, 4 °C for 5 min to remove the supernatant. The precipitated infected cells were suspended in 80 ml of ice-cold PBS, and centrifuged at 1,000 rpm, 4 °C for 5 min to remove the supernatant. The precipitated infected cells were suspended in 40 ml of ice-cold Lysis Buffer (50 mM Tris-HCl (pH 8.5), 5 mM 2-mercaptoethanol, 100 mM KCl, 1 mM PMSF and 1 % (v/v) NP-40). The suspension was centrifuged at 12,000 rpm, 4 °C for 30 min to provide a supernatant.
The supernatant was loaded onto an Ni-NTA agarose column (3 ml, purchased from Qiagen) equilibrated with 30 ml of Buffer A (20 mM Tris-HCl (pH 8.5), 5 mM 2-mercaptoethanol, 500 mM KCl, 20 mM imidazole and 10 % (v/v) glycerol). The column was washed with 30 ml of Buffer A, 6 ml of Buffer B (20 mM Tris-HCl (pH 8.5), 5 mM 2-mercaptoethanol, 1 M KCl, and 10 % (v/v) glycerol) and 6 ml of Buffer A in this order. Then, the column was eluted with 6 ml of Buffer C (20 mM Tris-HCl (pH 8.5), 5 mM 2-mercaptoethanol, 100 mM KCl, 100 mM imidazole, and 10 % (v/v) glycerol) to provide a fraction. The fraction was entrapped in a dialysis membrane (purchased from Spectrum Laboratories), dialyzed at 4 °C overnight with 1 L of dialysis buffer (20 mM Tris-HCl (pH 7.5), 10 % (v/v) glycerol, 1 mM dithiothreitol, 0.1 mM Na₃VO₄ and 0.1 mM EGTA), and stored at -80 °C until used. An aliquot of the dialyzed fraction was subjected to SDS electrophoresis, and then a recombinant protein (His6-HGFR, the HGFR cytoplasmic domain fused with six histidine at the N terminus) detected at a molecular weight of about 60 kDa when stained with Coomassie Brilliant Blue, was determined with regard to protein content using BSA (purchased from Sigma) as a standard.

### 3. Assay for the inhibitory activity against HGFR tyrosine kinase activity

To each well of a 96-well round plate (purchased from NUNC, Production No. 163320) were added 10 µl of a solution for kinase reaction (200 mM Hepes (pH 7.4), 80 mM MgCl₂, 16 mM MnCl₂ and 2 mM Na₃VO₄), 250 ng of biotinylated poly(Glu4: Tyrl) (biotin-poly(GT), purchased from Japan Schering) (6 µl, 15-fold diluted with distilled water), 30 ng of His6-HGFR (10 µl, 60-fold diluted with 0.4 % BSA) and a test substance dissolved in dimethylsulfoxide (4 µl, 100-fold diluted with 0.1 % BSA) to mess up to 30 µl. To the well was added 10 µl of 4 µM ATP (purchased from Sigma) diluted with distilled water to incubate at 30 °C for 10 min, followed by adding 10 µl of 500 mM EDTA (pH 8.0) (purchased from Wako Pure Chemicals) to provide a kinase reaction solution.
The tyrosine-phosphorylated biotin-poly(GT) was detected using the Homogenous Time-Resolved Fluorescence (HTRF) method (Analytical Biochemistry, 269, 94-104, 1999). That is, to each well of a 96-well half-area black plate (purchased from COSTAR, Production No. 3694) were added 20 µl of the above kinase reaction solution and 30 µl of a dilution solution (50 mM Hepes (pH 7.4), 20 mM MgCl₂, 4 mM MnCl₂, 0.5 mM Na₃VO₄, 0.1 % BSA and 100 mM EDTA). To the well was added 7.5 ng of an europium cryptate-labeled anti-phosphotyrosine antibody (Eu(K)-PY20, purchased from Japan Schering) (25 µl, 250-fold diluted with 20 mM Hepes (pH 7.0), 0.5 M KF and 0.1 % BSA) and 250 ng of XL665-labeled streptavidin (XL665-SA, purchased from Japan Schering) (25 µl, 62.5-fold diluted with 20 mM Hepes (pH 7.0), 0.5 M KF and 0.1 % BSA), and using a discovery HTRF microplate analyzer (Packard), the well was instantly irradiated at an excitation wavelength of 337 nm to determine fluorescence intensities at 665 nm and 620 nm. The tyrosine phosphorylation rate of a biotin-poly(GT) was calculated using a delta F% value described in the text of a HTRF standard experiment method by Japan Schering. While defining the delta F% value of a well added with His6-HGFR and no test substance as 100 % and the delta F% value of a well added with no His6-HGFR and no test substance as 0 %, ratio (%) of the delta F% value of each well added with the test substance was calculated. The ratio (%) was used to calculate the concentration (IC₅₀) of the test substance necessary to inhibit HGFR kinase activity by 50 %. The results are shown in Table 7.

**[Table 7]**

| Example | IC50 (µM) | Example | IC50 (µM) | Example | IC50 (µM) |
|---|---|---|---|---|---|
| 1 | 0.066 | 41 | 0.044 | 83 | 0.017 |
| 2 | 0.055 | 42 | 0.057 | 84 | 0.009 |
| 3 | 0.039 | 43 | 0.18 | 85 | 0.015 |
| 4 | 0.045 | 44 | 0.091 | 86 | 0.012 |
| 5 | 0.06 | 45 | 0.24 | 87 | 0.009 |
| 6 | 0.64 | 46 | 0.064 | 88 | 0.016 |
| 7 | 0.051 | 47 | 0.083 | 89 | 0.013 |
| 8 | 0.048 | 48 | 0.063 | 90 | 0.012 |
| 9 | 0.053 | 49 | 0.18 | 91 | 0.004 |
| 10 | 0.054 | 51 | 0.25 | 92 | 0.047 |
| 11 | 0.046 | 52 | 0.16 | 93 | 0.042 |
| 12 | 0.037 | 53 | 0.27 | 94 | 0.049 |
| 13 | 0.055 | 54 | 0.064 | 95 | 0.05 |
| 14 | 0.06 | 55 | 0.12 | 96 | 0.017 |
| 15 | 0.053 | 56 | 0.11 | 97 | 0.021 |
| 16 | 0.064 | 57 | 0.18 | 98 | 0.067 |
| 17 | 0.048 | 58 | 0.085 | 99 | 0.033 |
| 18 | 0.053 | 59 | 0.075 | 100 | 0.085 |
| 19 | 0.061 | 60 | 0.082 | 101 | 0.072 |
| 20 | 0.059 | 61 | 0.015 | 102 | 0.072 |
| 21 | 0.062 | 62 | 0.02 | 103 | 0.057 |
| 22 | 0.05 | 63 | 0.014 | 104 | 0.071 |
| 23 | 0.045 | 64 | 0.058 | 105 | 0.015 |
| 24 | 0.048 | 65 | 0.015 | 106 | 0.016 |
| 25 | 0.085 | 66 | 0.02 | 107 | 0.061 |
| 26 | 0.058 | 67 | 0.017 | | |
| 27 | 0.059 | 68 | 0.023 | | |
| 28 | 0.072 | 69 | 0.031 | | |
| 29 | 0.063 | 70 | 0.019 | | |
| 30 | 0.044 | 71 | 0.121 | | |
| 31 | 0.062 | 72 | 0.01 | | |
| 32 | 0.05 | 73 | 0.105 | | |
| 33 | 0.026 | 75 | 0.01 | | |
| 34 | 0.073 | 76 | 0.045 | | |
| 35 | 0.029 | 77 | 0.058 | | |
| 36 | 0.046 | 78 | 0.014 | | |
| 37 | 0.053 | 79 | 0.014 | | |
| 38 | 0.052 | 80 | 0.018 | | |
| 39 | 0.1 | 81 | 0.019 | | |
| 40 | 0.055 | 82 | 0.016 | | |

### Pharmacological Test Example 2: Analysis of HGFR phosphorylation and HGFR expression in cancer cell lines

### 1. Preparation of cell extract

MKN-45 (human gastric cancer cells) was purchased from JCRB, MKN-74 (human gastric cancer cells) was from Immuno-Biological Laboratories Co., Ltd., and SUN-1 (human gastric cancer cells) and SNU-5 (human gastric cancer cells) were from ATCC. EBC-1 (human lung cancer cells) was purchased from Japan Health Sciences Foundation and A549 (human lung cancer cells) was purchased from Dainippon Pharmaceutical Co., Ltd.
The respective cells were cultured in a flask (purchased from FALCON; product number: 353136) containing RPMI1640 medium (purchased from Sigma) containing 10% FBS in a 5% CO₂ incubator (37 °C). When the cells became subconfluent, the supernatant was removed from the flask. The respective flasks were washed twice with PBS, and 1 mL of a lysis buffer (50 mM Hepes (pH 7.4), 150 mM NaCl, 10% (v/v) glycerol, 1% Triton X-100, 1.5 mM MgCl₂, 1 mM EDTA (pH8.0), 100 mM NaF, 1 mM PMSF, 10 µg/ml Aprotinin, 50 µg/ml Leupeptin, 1 µg/ml Pepstatin A, 1 mM Na₃VO₄) was added thereto. The cells containing the solution were collected by a cell scraper and treated with 15,000 rpm at 4 °C for 15 min and the proteins are solubilized by adding an SDS buffer to the supernatant and treating at 94 °C for 5 min, and prepared as a cell extract at 2 µg/µl.

### 2. Electrophoresis and western blotting

10 µg/ 5 µl of the above cell extract was electrophoresed on a 4-20% gradient polyacrylamide gel (purchased from Daiichi Pure Chemicals Co., Ltd.). After the electrophoresis, the proteins were transferred to a PVDF membrane according to an ordinary method. Immunoblot was performed against the transferred membrane by using anti-phosphorylated c-Met antibody (Phospho-Met(Tyr1234/1235) Antibody; purchased from Cell Signaling, product number: 3126), anti-phosphorylated c-Met antibody (Phospho-Met(Tyr1349) Antibody; purchased from Cell Signaling, product number: 3121), anti c-Met antibody (Met(C-12); purchased from Santa Cruz, product number: sc-10) or anti β-actin antibody (MONOCLONAL ANT1-β-ACTIN CLONE AC-15; purchased from Sigma, product number: A-5441) as a primary antibody and using horseradish peroxidase-labeled anti rabbit IgG antibody (anti-rabbit IgG, Hop-plinked Antibody; purchased from Cell Signaling, product number: 7074) or horseradish peroxidase-labeled anti mouse IgG antibody (anti-rabbit IgG, Hop-plinked Antibody; purchased from Cell Signaling, product number: 7076) as a secondary antibody. After washing the membrane, Super Signal (purchased from PIERCE) was used for color development.

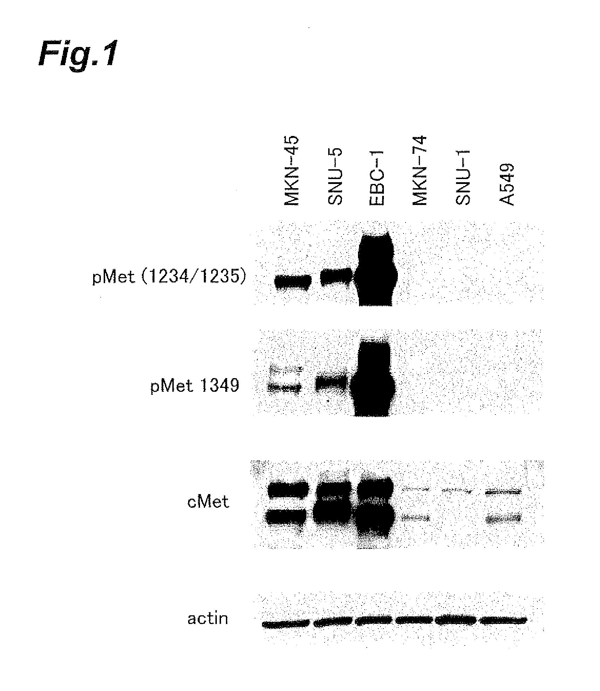

Figure 1 shows the results of HGFR autophosphorylation and HGFR western blotting of the respective cell lines. As is clear from Figure 1, HGFR is strongly expressed and HGFR phosphorylation was confirmed in MKN-45, SNU-5 and EBC-1. On the other hand, HGFR is weakly expressed and no HGFR phosphorylation was confirmed in MKN-74, SNU-1 and A549.

### Pharmacological Test Example 3: Analysis of HGFR gene amplification in cancer cell lines by FISH

### 1. Preparation of probe

HS BAC CLONE-CITB LIB Clone ID 13N12 was purchased from Invitrogen (product number: 96012). Plasmids were purified according to the protocol of HiSpeed Plasmid Maxi Kit (purchased from QIAGEN GmbH, product number: 12662). Plasmids were cleaved by restriction enzyme Not1 and electrophoresed on a 0.7% agarose gel. The electrophoresed agarose gel was immersed into a 0.5% ethidium bromide solution. A CTB-13N12 fragment was cut from the gel by using a transilluminator. Extraction from the agarose gel was performed according to the protocol of QIAEX II Gel Extraction Kit (purchased from QIAGEN GmbH, product number: 20021). The 13N12 fragment was DIG-labeled according to the protocol of DIG-NICK TRANSLATION MIX (purchased from Roche Applied Science; product number: 1745816). To the DIG-labeled sample were added ssDNA (purchased from Invitrogen; product number: 15632011) and HUMAN COT-1 DNA (purchased from Invitrogen; product number: 15279-011). After ethanol precipitation and air drying, the sample was dissolved in mRNA In Situ Hybridization Solution (purchased from DakoCytomation; product number: S3304), which was used as an HGFR probe.

### 2. Preparation of cell smear

Cells (2 x 10⁶) were seeded on a dish (purchased from FALCON, product number: 353004) and cultured overnight in RPMI1640 medium (purchased from Sigma) containing 10% FBS in a CO₂ incubator at 37 °C. A demecolcine solution (purchased from Wako Pure Chemical Industries, Ltd., product number: 045-18761) was added at a final concentration of 0.1 µg/mL and the culture was continued for 8 hours. The supernatant was removed, the cells were washed with PBS and treated with 0.05% trypsin-EDTA, and the medium was added and well suspended. The cell suspension was centrifuged and the supernatant was removed, the cell precipitate was re-suspended in PBS and centrifuged and the supernatant was removed. The cell precipitate was suspended in 10 mL of 0.005M potassium chloride solution and allowed to stand at room temperature for 20 min. After adding 2 mL of Carnoy's fixative (3:1 methanol:acetic acid), centrifuge was performed and the supernatant was removed. The cell precipitate was suspended in 10 mL of Carnoy's fixative and allowed to stand at room temperature for 30 min. Centrifuge was performed and the supernatant was removed. The cell precipitate was washed twice with 10 mL of Carnoy's fixative. The cell precipitate was suspended in 0.2 mL of Carnoy's fixative, and 0.1 mL of the cell suspension was dropped on a slide glass (purchased from MATSUNAMI, production number: S9411) and dried overnight at room temperature. This was used as a cell smear preparation.

### 3. Analysis of HGFR gene amplification by FISH

The cell smear preparation was dried on a heat block at 60 °C for 2 hours. To a 2xSSC solution was added RNaseA (purchased from Nacalai Tesque, Inc., product number 30141-14) at a final concentration of 100 µg/mL and the solution was kept at 37 °C. The preparation was immersed in the solution and allowed to react at 37 °C for 1 hour. The preparation was washed three times with 2xSSC for 3 min. The preparation was dehydrated with 70% ethanol, 85% ethanol and 100% ethanol in this order and dried. Pepsin was dissolve in a 10 mM hydrochloric acid solution at a final concentration of 0.01%(w/v) and the solution was kept at 37 °C. The preparation was immersed in the solution and allowed to react at 37 °C for 10 min. The preparation was washed twice with PBS for 3 min, allowed to react in PBS containing 1% paraformaldehyde for 10 min, and washed twice with PBS for 3 min. The preparation was dehydrated with 70% ethanol, 85% ethanol and 100% ethanol in this order and dried. On the dried preparation were applied 3 µL of the HGFR probe and 2 µL of Chromosome 7 alphasatellite probe (purchased from Qbiogene, product number: PSAT0007-R5) and sealed with a 18mm x 18mm cover glass, and periphery of the cover glass was covered by rubber cement and the cover glass was fixed. The preparation was treated with heat for 5 min on a heat block kept at 80 °C. The preparation was placed in a moistening box and incubated at 37 °C for 16 hours. The sample was taken from the box and the rubber cement was removed. The preparation was washed three times with 50% formaldehyde in 2xSSC at 45 °C for 5 min with changing the solution. The preparation was washed three times with a 2xSSC solution and HGFR probe in the preparation was fluorescent-labeled according to the protocol of Fluorescent Antibody Enhancer Set for DIG Detection (purchased from Roche, product number: 11768506910). DAPI was used for nuclear staining. The sample was sealed with a 18mm x 18mm cover glass. The sample was observed by an upright microscope Axiovert200M (Zeiss).

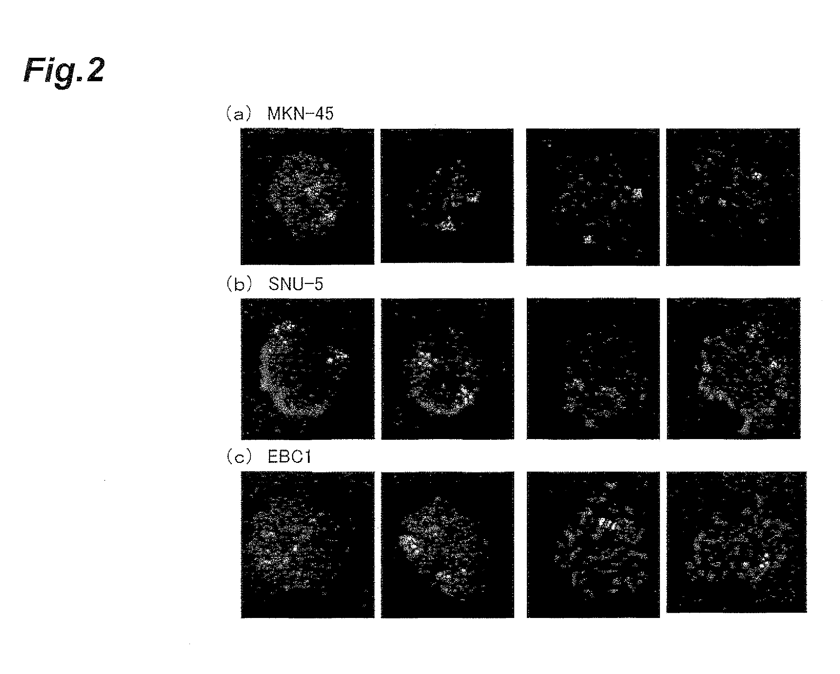

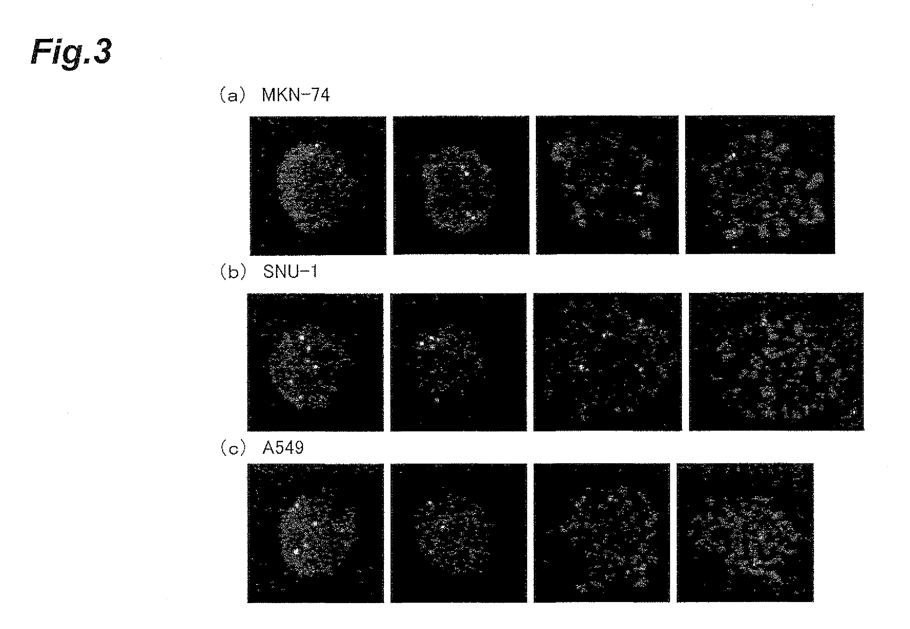

Figures 2 and 3 are fluorescent staining images of the respective cells, which analyze HGFR gene amplification by FISH. Blue indicates nucleus, green indicates HGFR and red indicates Chromosome 7 alphasatellite. As is clear from figure 2, the number of HGFR fluorescence was greater than the number of Chromosome 7 alphasatellite fluorescence in MKN-45, SNU-5 and EBC-1, thus HGFR gene was amplified. On the other hand, as is clear from figure 3, the number of Chromosome 7 alphasatellite fluorescence was nearly equal to the number of HGFR fluorescence in MKN-74, SNU-1 and A549, thus HGFR gene was not amplified.

### Pharmacological Test Example 4: HGFR autophosphorylation inhibitory effect using MKN-45 cells

### 1. Preparation of cell extract

Human gastric cancer cells (MKN-45) was suspended in RPMI1640 medium (purchased from Sigma) containing 10% FBS. The cell suspension (3.3 x 10⁴ cells/ml) was added to a cell culture 6-well plate (purchased from FALCON, product number; 353046) at 3 ml/well, and cultured for 72 hours in a 5% CO₂ incubator (37 °C). After the culture, the supernatant was removed from each well, and 1.8 mL of RPMI1640 medium containing 10% FBS. 0.2 mL of a test substance dissolved in DMSO (diluted with RPMI1640 medium containing 10% FBS) and the culture was continued for 2 hours in a 5% CO₂ incubator (37 °C). The supernatant was removed from each well, and each well was washed twice with 2mL of PBS, and 0.2 mL of a lysis buffer (50 mM Hepes (pH 7.4), 150 mM NaCl, 10% (v/v) glycerol, 1% Triton X-100, 1.5 mM MgCl₂, 1 mM EDTA (pH8.0), 100 mM NaF, 1 mM PMSF, 10 µg/ml Aprotinin, 50 µg/ml Leupeptin, 1 µg/ml Pepstatin A, 1 mM Na₃VO₄) was added thereto. The cells containing the solution were collected by a cell scraper and treated with 15,000 rpm at 4 °C for 15 min, and the proteins are solubilized by adding an SDS buffer to the supernatant and treating at 94 °C for 5 min, and prepared as a cell extract at 5 µg/ 10 µl.

### 2. Electrophoresis and western blotting

5 µg/ 10 µl of the above cell extracts were electrophoresed on a 4-20% gradient polyacrylamide gel (purchased from Daiichi Pure Chemicals Co., Ltd.). After the electrophoresis, the proteins were transferred to a PVDF membrane according to an ordinary method. Immunoblot was performed against the transferred membrane by using anti-phosphorylated c-Met antibody (Phospho-Met(Tyr1234/1235) Antibody; purchased from Cell Signaling, product number: 3126), anti-phosphorylated c-Met antibody (Phospho-Met(Tyr1349) Antibody; purchased from Cell Signaling, product number: 3121) or anti c-Met antibody (Met(C-12); purchased from Santa Cruz, product number: sc-10) as a primary antibody and using horseradish peroxidase-labeled anti rabbit IgG antibody (anti-rabbit IgG, HRP-linked Antibody; purchased from Cell Signaling, product number: 7074) as a secondary antibody. After washing the membrane, Super Signal (purchased from PIERCE) was used for color development.

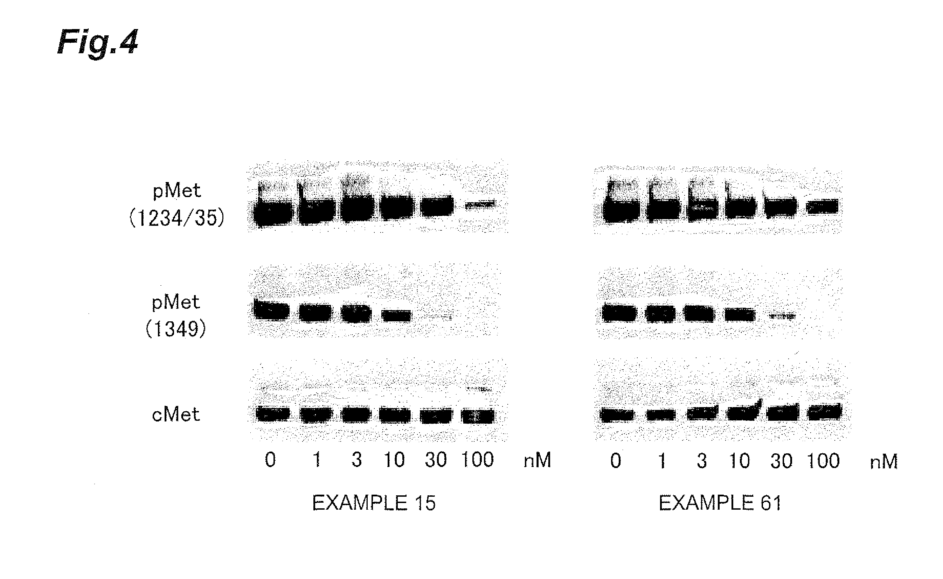

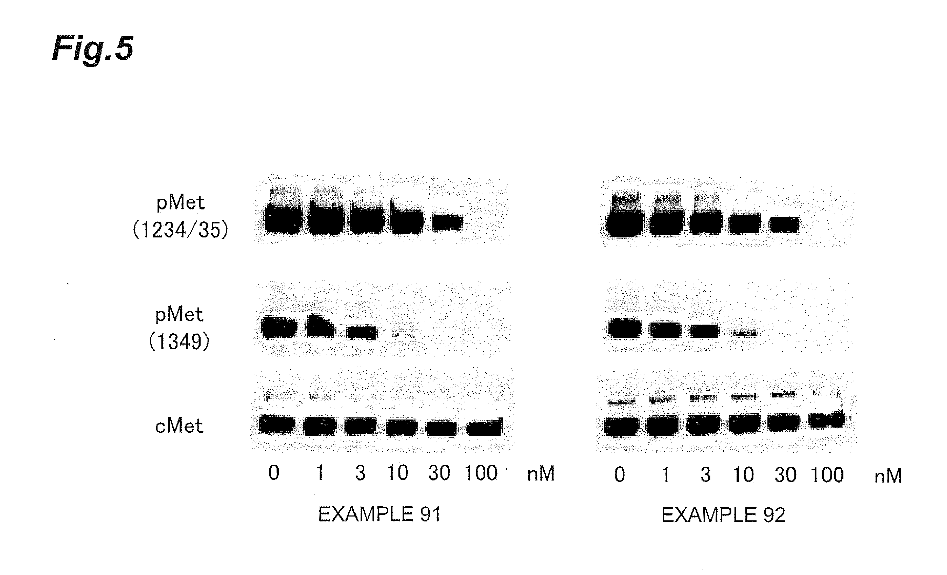

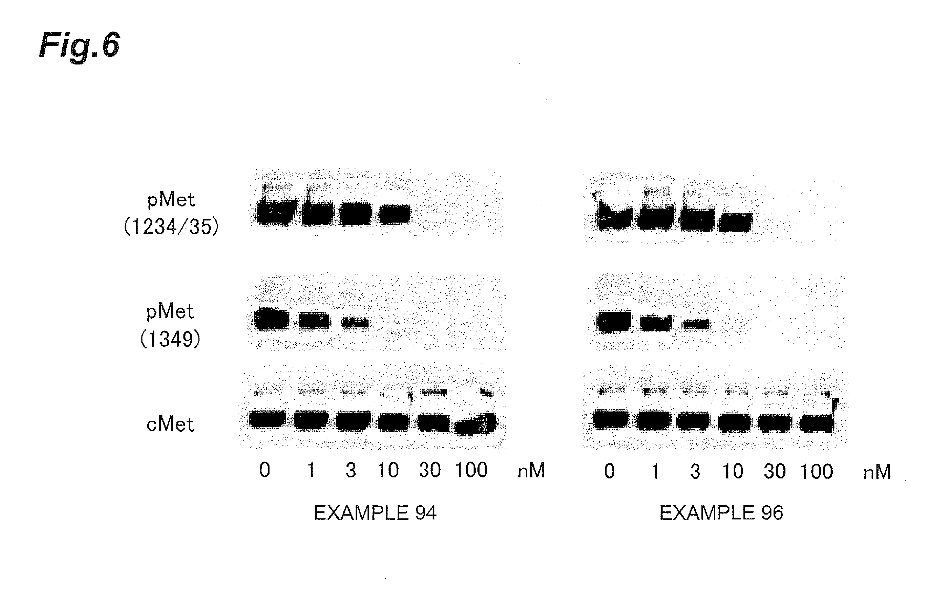

Figures 4 to 6 shows the results of HGFR autophosphorylation and HGFR western blotting at respective concentrations with varying the concentrations of the test substance at several steps. As is clear from figures 4 to 6, the pyridine or pyrimidine derivative according to the present invention inhibits HGFR autophosphorylation in a concentration-dependent manner.

### Pharmacological Test Example 5: Proliferation inhibitory effect against human cancer cells

Six kinds of human cancer cells are suspended in RPMI1640 medium (purchased from Sigma) containing 10% FBS, respectively. The cell suspensions (MKN-45: 1.5 x 10⁴ cells/ml, SNU-5: 5 x 10⁴ cells/ml, EBC-1: 3 x 10⁴ cells/ml, MKN-74: 2.5 x 10⁴ cells/ml, SNU-1: 2.5 x 10⁴ cells/ml, A549: 1.5 x 10⁴ cells/ml) were added to a cell culture 96-well plate (purchased from NUNC, product number: 167008) at 0.1 ml/well, and cultured overnight in a 5% CO₂ incubator (37 °C). After the culture, 0.1 ml of a test substance diluted with RPMI1640 medium containing 10% FBS and the culture was continued for 3 days in a 5% CO₂ incubator (37 °C). After the culture, 10 µl of Cell Counting Kit-8 (purchased from DOJINDO, product number: 343-07623) was added to each well and the incubation was performed for 0.5 to 3 hours in a 5% CO₂ incubator (37 °C). After the incubation, absorbance of each well was measured by a plate reader MTP-500 (Corona Electric Co.,Ltd.) with measurement wavelength 450nm and reference wavelength 660nm. The ratio (%) of the absorbance in each well with a test substance to the absorbance in each well without the test substance was calculated, and the concentration of the test substance necessary to inhibit cell proliferation by 50% (IC₅₀) based on the ratio was calculated and shown in table 8.

**[Table 8]**

| Example # | c-met amplified cell | | | c-met non-amplified cell | | |
|---|---|---|---|---|---|---|
| | MKN-45 | SNU-5 | EBC-1 | MKN-74 | SNU-1 | A549 |
| 15 | 0.015 | 0.0062 | 0.017 | 4.3 | 4.2 | 2.6 |
| 61 | 0.060 | 0.038 | 0.065 | 4.2 | >10 | >10 |
| 91 | 0.0060 | 0.0060 | 0.0064 | 3.0 | 2.0 | 1.9 2. |
| 92 | 0.0038 | 0.0017 | 0.0038 | 2.0 | 2.3 | 9 1. |
| 94 | 0.0023 | 0.0017 | 0.0024 | 1.9 | 1.6 | 8 2. |
| 96 | 0.0047 | 0.0049 | 0.0045 | 3.3 | 3.6 | 2.0 |

As is clear from this table, the pyridine or pyrimidine derivative according to the present invention inhibits cell proliferation more effectively in HGFR amplified cancer cells than in HGFR non-amplified cancer cells.

### Pharmacological Test Example 6: Tumor proliferation inhibitory effect against human cancer cells (MKN-45)

Human gastric cancer cells (MKN-45) were suspended in HBSS (purchased from GIBCO BRL). The cell suspension (5 x 10⁷ cells/ml; 0.1 ml) was transplanted to the subcutaneous parts of the right flank of 7-week female BALB/c(nu/nu) mice. When the tumor volume reached 100-200 mm³, mice were grouped so that the average of the tumor volume was equable in each group, and 0.5% methylcellulose in hydrochloric acid-glucose solution (0.1N hydrochloric acid:5% glucose solution=1:9) or a test substance suspended in dimethylsulfoxide-Tween-glucose solution (dimethylsulfoxide:Tween:5% glucose solution (containing equimolar hydrochloric acid of the test substance)=7:13:80) was orally administered to mice daily twice a day. The tumor volume was measured on the fifth day from the administration of the test substance. The long and short diameters of the tumor were measured with a caliper and the tumor volume was calculated according to the equation: 1/2 x long diameter x short diameter x short diameter. The experiment was conducted in a control group (vehicle administered group) of 10 animals as well as in the test substance administered group of 5 animals. Tumor proliferation rate (%) was defined as the ration of the tumor volume of the test substance administered group to the tumor volume of control group and shown in table 9

**[Table 9]**

| Example # | Dose (mg/kg weight/administration) | tumor proliferation rate(%) |
|---|---|---|
| 15 | 30 | 29.7 |
| 15 | 100 | 19.9 |
| 61 | 12.5 | 35.8 |
| 91 | 12.5 | 22.9 |
| 91 | 1 | 73.0 |
| 91 | 3 | 55.5 |
| 91 | 10 | 27.4 |
| 91 | 30 | 19.6 |
| 91 | 100 | 16.0 |
| 92 | 12.5 | 27.9 |

As is clear from the table, the pyridine or pyrimidine derivative according to the present invention inhibits proliferation of tumors with amplified HGFR *in vivo.*

### Industrial Applicability

The pyridine or pyrimidine derivative according to the present invention is useful as an anti-tumor agent, especially an anti-tumor agent against tumors with amplified HGFR gene.

## Claims

1. A method for predicting anti-tumor effect of a pyridine or pyrimidine derivative comprising the steps of:
assaying expression level of hepatocyte growth factor receptor in tumor cells; and
determining whether a pyridine or pyrimidine derivative is effective or not against the tumor cells by using the expression level of hepatocyte growth factor receptor as an index based on the assayed expression level,
wherein the pyridine or pyrimidine derivative is at least one compound, salt thereof or solvate of the foregoing selected from the compound represented by the formula (I): 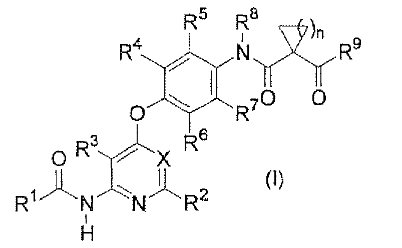
wherein R¹ represents a 3- to 10-membered non-aromatic heterocyclic group wherein the group is limited to a group having nitrogen as a ring constituent atom and the nitrogen having a bonding hand, or a group represented by the formula -NR^{11a}R^{11b}, wherein R^{11a} and R^{11b} may be the same or different and each represents hydrogen, C₁₋₆ alkyl, C₃₋₆ alkenyl, C₃₋₆ alkynyl, C₃₋₁₀ cycloalkyl, C₆₋₁₀ aryl, 5- to 10-membered heteroaryl or a 4- to 10-membered non-aromatic heterocyclic group, and R^{11a} and R^{11b} may be substituted with a substituent selected from Substituent Group A or Substituent Group B and R¹ may be substituted with a substituent selected from Substituent Group A or Substituent Group B;
R² and R³ represent hydrogen;
R⁴, R⁵, R⁶ and R⁷ may be the same or different and each represents hydrogen, halogen, hydroxyl, cyano, trifluoromethyl, C₁₋₆ alkyl, C₂₋₆ alkenyl, C₂₋₆ alkynyl, C₁₋₆ alkoxy, amino, mono-C₁₋₆ alkylamino, di-C₁₋₆ alkylamino or a group represented by the formula -CO-R¹², wherein R¹² represents hydrogen, hydroxyl, C₁₋₆ alkyl, C₁₋₆ alkoxy, amino, mono-C₁₋₆ alkylamino or di-C₁₋₆ alkylamino;
R⁸ represents hydrogen or C₁₋₆ alkyl;
R⁹ represents a 3- to 10-membered non-aromatic heterocyclic group wherein the group is limited to a group having nitrogen as a ring constituent atom and the nitrogen having a bonding hand, or a group represented by the formula - NR^{11a}R^{11b}, wherein R^{11a} and R^{11b} represent the same meaning as described above and R⁹ may be substituted with a substituent selected from Substituent Group A or Substituent Group B;
n represents an integer of 1 or 2; and
X represents a group represented by the formula -C(R¹⁰)= or nitrogen, wherein R¹⁰ represents hydrogen, halogen, cyano, C₁₋₆ alkyl, C₂₋₆ alkenyl, C₂₋₆ alkynyl or a group represented by the formula -CO-R¹², wherein R¹² represents the same meaning as recited above;
wherein Substituent Group A consists of halogen, hydroxyl, mercapto, nitro, cyano and oxo;
wherein Substituent Group B consists of C₁₋₆ alkyl, C₂₋₆ alkenyl, C₂₋₆ alkynyl, C₃₋₁₀ cycloalkyl, C₆₋₁₀ aryl, 5- to 10-membered heteroaryl, a 3- to 10-membered non-aromatic heterocyclic group, C₁₋₆ alkoxy, C₃₋₆ alkenyloxy, C₃₋₆ alkynyloxy, C₃₋₁₀ cycloalkoxy, C₆₋₁₀ aryloxy, 5- to 10-membered heteroaryloxy, 4-to 10-membered non-aromatic heterocyclicoxy, C₁₋₆ alkylthio, C₃₋₆ alkenylthio, C₃₋₆ alkynylthio, C₃₋₁₀ cycloalkylthio, C₆₋₁₀ arylthio, 5- to 10-membered heteroarylthio, 4- to 10-membered non-aromatic heterocyclicthio and a group represented by the formula -T¹-T²-T³, and each group in Substituent Group B may be substituted with a substituent selected from Substituent Group C, wherein T¹ represents a direct bond or C₁₋₆ alkylene, T² represents carbonyl, sulfinyl, sulfonyl, a group represented by the formula -C(=O)-O-, a group represented by the formula -O-C(=O)-, a group represented by the formula -SO₂-O-, a group represented by the formula -O-SO₂-, a group represented by the formula -NR^{T1}-, a group represented by the formula -C(=O)-NR^{T1}-, a group represented by the formula -NR^{T1}-C(=O)-, a group represented by the formula -SO₂-NR^{T1}- or a group represented by the formula -NR^{T1}-SO₂-, T³ represents hydrogen, C₁₋₆ alkyl, C₃₋₆ alkenyl, C₃₋₆ alkynyl, €₃₋₁₀ cycloalkyl, C₆₋₁₀ aryl, 5- to 10-membered heteroaryl or a 4- to 10-membered non-aromatic heterocyclic group, and R^{T1} represents hydrogen or C₁₋₆ alkyl; and
wherein Substituent Group C consists of halogen, hydroxyl, mercapto, nitro, cyano, oxo, C₁₋₆ alkyl, C₂₋₆ alkenyl, C₂₋₆ alkynyl, C₃₋₁₀ cycloalkyl, C₆₋₁₀ aryl, 5- to 10-membered heteroaryl, a 3- to 10-membered non-aromatic heterocyclic group, C₁₋₆ alkoxy, C₁₋₆ alkylthio, mono-C₁₋₆ alkylamino and di-C₁₋₆ alkylamino.

2. The method according to claim 1, wherein the method of assaying expression level of hepatocyte growth factor receptor is an immunological method.

3. The method according to claim 2, wherein the immunological method is an immunostaining method.

4. The method according to claim 1, wherein the method of assaying expression level of hepatocyte growth factor receptor is a method of assaying expression level of the gene.

5. The method according to claim 4, wherein the method of assaying expression level of the gene is a method of assaying gene amplification.

6. The method according to claim 5, wherein the method of assaying gene amplification is fluorescence *in situ* hybridization.

7. The method according to any one of claims 1 to 6, wherein R¹ represents a 3- to 10-membered non-aromatic heterocyclic group optionally substituted with a substituent selected from Substituent Group A or Substituent Group B recited in claim 1, wherein the group is limited to a group having nitrogen as a ring constituent atom and the nitrogen having a bonding hand.

8. The method according to any one of claims 1 to 6, wherein R¹ represents a group represented by the formula (II): 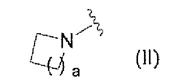 wherein a represents an integer of 1 to 4;
or a group represented by the formula (III): 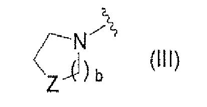 wherein b represents an integer of 1 to 3, and Z represents oxygen, sulfur, carbonyl, sulfonyl, or a group represented by the formula -NR^{Z}-, wherein R^{Z} represents hydrogen or C₁₋₆ alkyl, and the groups represented by the formula (II) or (III) may be substituted with a substituent selected from Substituent Group A or Substituent Group B recited in claim 1.

9. The method according to any one of claims 1 to 6, wherein R¹ represents azetidin-1-yl optionally substituted with a substituent selected from Substituent Group D, pyrrolidin-1-yl optionally substituted with a substituent selected from Substituent Group D, pipexidin-1-yl optionally substituted with a substituent selected from Substituent Group D, azepan-1-yl optionally substituted with a substituent selected from Substituent Group D, piperazin-1-yl optionally substituted with a substituent selected from Substituent Group D, diazepan-1-yl optionally substituted with a substituent selected from Substituent Group D, morpholin-4-yl optionally substituted with a substituent selected from Substituent Group D, thiomorpholin-4-yl optionally substituted with a substituent selected from Substituent Group D, 1,1-dioxothiomorpholin-4-yl optionally substituted with a substituent selected from Substituent Group D,
wherein Substituent Group D consists of halogen, hydroxyl, mercapto, cyano, formyl, oxo, C₁₋₆ alkyl, C₃₋₁₀ cycloalkyl, C₁₋₆ alkoxy, amino, mono-C₁₋₆ alkylamino, di-C₁₋₆ alkylamino, azetidinyl, pyrrolidinyl, piperidinyl, piperazinyl, diazepanyl and a group represented by -T⁴-T⁵, wherein T⁴ represents carbonyl or sulfonyl, and T⁵ represents C₁₋₆ alkyl, C₃₋₁₀ cycloalkyl, azetidinyl, pyrrolidinyl, piperidinyl, hydroxyl, C₁₋₆ alkoxy, amino, mono-C₁₋₆ alkylamino or di-C₁₋₆ alkylamino,
where each group included in Substituent Group D may be substituted with hydroxyl, C₁₋₆ alkyl, di-C₁₋₆ alkylamino, azetidinyl or pyrrolidinyl.

10. The method according to any one of claims 1 to 6, wherein R¹ represent azetidin-1-yl optionally substituted with a substituent selected from Substituent Group E, pyrrolidin-1-yl optionally substituted with a substituent selected from Substituent Group E, piperidin-1-yl optionally substituted with a substituent selected from Substituent Group E, piperazin-1-yl optionally substituted with a substituent selected from Substituent Group E, diazepan-1-yl optionally substituted with a substituent selected from Substituent Group E or morpholin-4-yl optionally substituted with a substituent selected from Substituent Group E,
wherein Substituent Group E consists of methyl, ethyl, dimethylamino, azetidinyl, pyrrolidinyl, piperidinyl and piperazinyl,
where each group included in Substituent Group E may be substituted with hydroxyl, methyl, dimethylamino, azetidinyl, pyrrolidinyl or piperidinyl.

11. The method according to any one of claims 1 to 6, wherein R¹ represents azetidin-1-yl optionally substituted with a substituent selected from Substituent Group G, pyrrolidin-1-yl optionally substituted with a substituent selected from Substituent Group G, piperidin-1-yl optionally substituted with a substituent selected from Substituent Group G or piperazin-1-yl optionally substituted with a substituent selected from Substituent Group G,
wherein Substituent Group G consists of dimethylamino, azetidinyl, pyrrolidinyl, piperidinyl, piperazinyl, dimethylaminomethyl, dimethylaminoethyl, azetidin-1-ylmethyl, pyrrolidin-1-ylmethyl and piperidin-1-ylmethyl,
where each group included in Substituent Group G may be substituted with methyl or dimethylamino.

12. The method according to any one of claims 1 to 6, wherein R¹ represents a group represented by the formula -NR^{11a}R^{11b}, wherein R^{11a} and R^{11b} represent the same meaning as recited in claim 1.

13. The method according to any one of claims 1 to 6, wherein R¹ represents a group represented by the formula -NR^{11c}R^{11d}, wherein R^{11c} represents hydrogen or C₁₋₆ alkyl, and R^{11d} represents C₁₋₆ alkyl or a group represented by the formula (IV): 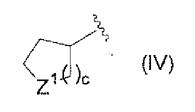 wherein c represents an integer of 1 to 3, and Z¹ represents oxygen, sulfur, carbonyl, sulfonyl or a group represented by the formula -NR^{Z1}-, wherein R^{Z1} represents hydrogen or C₁₋₆ alkyl, and R^{11d} may be substituted with a substituent selected from Substituent Group A or Substituent Group B recited in claim 1.

14. The method according to any one of claims 1 to 6, wherein R¹ represents a group represented by the formula -NR^{11e}R^{11f}, wherein R^{11e} represents hydrogen or C₁₋₆ alkyl, and R^{11f} represents C₁₋₆ alkyl, pyrrolidin-3-yl, piperidirl-3-yl, piperidin-4-yl or tetrahydropyran-4-yl, and R^{11f} may be substituted with a substituent selected from Substituent Group D recited in claim 9.

15. The method according to any one of claims 1 to 6, wherein R¹ represents a group represented by the formula -NR^{11g}R^{11h}, wherein R^{11g} represents hydrogen or methyl, and R^{11h} represents n-propyl, n-butyl, pyrrolidin-3-yl, piperidin-3-yl, piperidin-4-yl or tetrahydropyran-4-yl, and R^{11h} may be substituted with a substituent selected from Substituent Group F,
wherein Substituent Group F consists of methyl, ethyl, n-propyl, acetyl, dimethylamino, diethylamino, azetidinyl, pyrrolidinyl and piperazinyl,
where each group included in Substituent Group F may be substituted with methyl or dimethylamino.

16. The method according to any one of claims 1 to 6, wherein R¹ represents a group represented by the formula -N(CH₃)R¹¹ⁱ wherein R¹¹ⁱ represents n-propyl, n-butyl, pyrrolidin-3-yl or piperidin-4-yl, and R¹¹ⁱ may be substituted with a substituent selected from Substituent Group H,
wherein Substituent Group H consists of dimethylamino, diethylamino, dimethylaminoethyl, dimethylaminopropyl and 1-methylazetidin-3-yl.

17. The method according to any one of claims 1 to 6, wherein R¹ represents a group represented by the formula N(CH₃)R^{11j}, wherein R^{11j} represents 1-methylpiperidin-4-yl or 1-ethylpiperidin-4-yl.

18. The method according to any one of claims 1 to 17, wherein R⁴, R⁵, R⁶ and R⁷ may be the same or different and each represents hydrogen, halogen or C₁₋₆ alkyl.

19. The method according to any one of claims 1 to 18, wherein R⁸ represents hydrogen.

20. The method according to any one of claims 1 to 19, wherein X represents a group represented by the formula -C(R^{10a})=, wherein R^{10a} represents hydrogen, halogen or cyano.

21. The method according to any one of claims 1 to 19, wherein X represents nitrogen.

22. The method according to any one of claims 1 to 21, wherein n represents 1.

23. The method according to any one of claims 1 to 22, wherein R⁹ represents mono-C₁₋₆ alkylamino optionally substituted with a substituent selected from Substituent Group A or Substituent Group B recited in claim 1, mono-C₃₋₁₀ cycloalkylamino optionally substituted with a substituent selected from Substituent Group A or Substituent Group B recited in claim 1, mono-C₆₋₁₀ arylamino optionally substituted with a substituent selected from Substituent Group A or Substituent Group B recited in claim 1, mono-5- to 10-membered heteroarylamino optionally substituted with a substituent selected from Substituent Group A or Substituent Group B recited in claim 1 or mono-4- to 10-membered non-aromatic heterocyclic amino optionally substituted with a substituent selected from Substituent Group A or Substituent Group B recited in claim 1.

24. The method according to any one of claims 1 to 22, wherein R⁹ represents mono-C₃₋₁₀ cycloalkylamino optionally substituted with a substituent selected from Substituent Group A or Substituent Group B recited in claim 1 or mono-C₆₋₁₀ arylamino optionally substituted with a substituent selected from Substituent Group A or Substituent Group B recited in claim 1.

25. The method according to any one of claims 1 to 6, wherein the compound represented by the formula (I) is
(1) N-[4-({2-[({4-[2-(Dimethylamino)ethyl]piperazin-1-yl}carbanyl)amino]pyridin-4-yl}oxy)-2-fluorophenyl]-N'-(4-fluorophenyl)cyclopropane-1,1-dicarboxamide,
(2) N-(2-Fluoro-4-{[2-({[methyl(1-memylpiperidin-4-yl)amino]carbonyl}amino)pyridin-4-yl]oxy}phenyl)-N'-(4-fluorophenyl)cyclopropane-1,1-dicarboxamide,
(3) N-(4-Fluorophenyl)-N'-{2-fluoro-4-[(2-{[(4-pyrrolidin-1-yipiperidin-1-yl)carbonyl]amino}pyridin-4-yl)oxy]phenyl}cyclopropane-1,1-dicarboxamide,
(4) N-[4-({2-[({4-[(Dimethylamino)methyl]piperidin-1-yl}carbonyl)amino]pyridin-4-yl}oxy)-2-fluorophenyl]-N'-(4-fluorophenyl)cyclopropane-1,1-dicarboxamide,
(5) N-{4-[(2-{[(4-Azetidin-1-ylpiperidin-1-yl)carbonyl]amino}pyridin-4-yl)oxy]-2-fluorophenyl}-N'-{4-fluorophenyl}cyclapropane-1,1-dicarboxamide,
(6) N-[4-({2-[({4-[3-(Dimethylamino)azetidin-1-yl]piperidin-1-yl}carbonyl)amino]pyridin-4-yl}oxy)-2-fluorophenyl]-N'-(4-fluorophenyl)cyclopropane-1,1-dicarboxamide,
(7) N-(2-Fluaro-4-{[2-({[4-(4-methylpiperazin-1-yl)piperidin-1-yl]carbonyl}amino)pyridin-4-yl]oxy}phenyl)-N'-(4-fluorophenyl)cyclopropane-1,1-dicarboxamide,
(8) N-(2-Fluoro-4-{[2-({[4-(1-methylpiperidin-4-yl)piperazin-1-yl]carbonyl}amino)pyridin-4-yl]oxy}phenyl)-N'-(4-fluorophenyl)cyclopropane-1,1-dicarboxamide,
(9) N-(2-Fluoro-4-{[2-({[4-(1-methylazetidin-3-yl)piperazin-1-yl]carbonyl}amino)pyridin-4-yl]oxy}phenyl)-N'-(4-fluorophenyl)cyclopropane-1,1-dicarboxamide,
(10) N-(4-{[2-({[4-(Dimethylamino)piperidin-1-yl]carbonyl}amino)pyridin-4-yl]oxy}-2-fluorophenyl)-N'-(4-fluorophenyl)cyclopropane-1,1-dicarboxamide,
(11) N-(4-{[2-({[4-(Azetidin-1-ylmethyl)piperidin-1-yl]carbonyl}amino)pyridn-4-yl]oxy}-2-fluorophenyl)-N'-(4-fluorophenyl)cyclopropane-1,1-dicarboxamide,
(12) N-(4-Fluorophenyl)-N'-(2-fluoro-4-{[2-({[4-(pyrrolidin-1-ylmethyl)piperidin-1-yl]carbonyl}amino)pyridin-4-yl]oxy}phenyl)cyclopropane-1,1-dicarboxamide,
(13) N-(4-{[2-({[(3S)-3-(Dimethylamino)pyrrolidin-1-yl]carbonyl}amino)pyridin-4-yl]oxy}-2-fluorophenyl)-N'-(4-fluorophenyl)cyclopropane-1,1-dicarboxamide,
(14) N-(4-{[2-({[(3R)-3-(Dimethylamino)pyrrolidin-1-yl]carbonyl}amino)pyridin-4-yl]oxy}-2-fluorophenyl)-N'-(4-fluorophenyl)cyclopropane-1,1-dicarboxamide,
(15) N-(2-Fluoro-4-{[2-({[methyl(1-methylpiperidin-4-yl)amino]carbonyl}amino)pyridin-4-yl]oxy}phenyl)-N'-phenylcyclopropane-1,1-dicarboxamide,
(16) N-(2-Fluoro-4-{[2-({[4-(4-methylpiperazin-1-yl)piperidin-1-yl]carbonyl}amino)pyridin-4-yl]oxy}phenyl)-N'-phenylcyclopropane-1,1-dicarboxamide,
(17) N-[4-({2-[({4-[3-(Dimethylamino)azetidin-1-yl]piperidin-1-yl}carbonyl)amino]pyridin-4-yl}oxy)-2-fluorophenyl]-N'-phenylcyclopropane-1,1-dicarboxamide,
(18) N-(4-{[2-({[(1-Ethylpiperidin-4-yl)(methyl)amino]carbonyl}amino)pyridin-4-yl]oxy}-2-fluorophenyl)-N'-phenylcyclopropane-1,1-dicarboxamide,
(19) N-[4-({2-[(Azetidin-1-ylcarbonyl)amino]pyridin-4-yl}oxy)-2-fluorophenyl]-N'-(4-fluorophenyl)cyclopropane-1,1-dicarboxamide,
(20) N-(4-Fluorophenyl)-N'-[2-fluoro-4-({2-[(pyrrolidin-1-ylcarbonyl)amino]pyridin-4-yl}oxy)phenyl]cyclopropane-1,1-dicarboxamide,
(21) N-{2-Fluoro-4-[(2-{[(3-hydroxyazetidin-1-yl)carbonyl]amino}pyridin-4-yl)oxy]phenyl}-N'-(4-fluorophenyl)cyclopropane-1,1-dicarboxamide,
(22) N-[4-({2-[(1,3'-Biazetidin-1'-ylcarbonyl)amino]pyridin-4-yl}oxy)-2-fluorophenyl]-N'-(4-fluorophenyl)cymopropane-1,1-dicarboxamide,
(23) N-(2-Fluoro-4-{[2-({[3-(hydroxymethyl)azetidin-1-yl]carbonyl}amino)pyridin-4-yl]oxy}phenyl)-N'-(4-fluorophenyl)cyclopropane-1,1-dicarboxamide,
(24) N-(4-{[2-({[3-(Dimethylamino)azetidin-1-yl]carbonyl}amino)pyridin-4-yl]oxy}-2-fluorophenyl)-N'-(4-fluorophenyl)cyclopropane-1,1-dicarboxamde,
(25) N-[4-({2-[({3-[(Dimethylamino)methyl]azetidin-1-yl}carbonyl)amino]pyridin-4-yl}oxy)-2-fluorophenyl]-N'-(4-fluorophenyl)cyclopropane-1,1-dicarboxamide,
(26) N-{2-Fluoro-4-[(2-{[(4-hydroxypiperidin-1-yl)carbonyl]amino}pyridin-4-yl)oxy]phenyl}-N'-(4-fluorophenyl)cyclopropane-1,1-dicarboxamide,
(27) N-(2-Fluoro-4-{[2-({[4-(hydroxymethyl)piperidin-1-yl]carbonyl}amino)pyridin-4-yl]oxy}phenyl)-N'-(4-fluorophenyl)cyclopropane-1,1-dicarboxamide,
(28) N-(2-Fluoro-4-{[2-({[(3R)-3-hydroxypyrrolidin-1-yl]carbonyl}amino)pyridin-4-yl]oxy}phenyl)-N'-(4-fluorophenyl)cyclopropane-1,1-dicarboxamide,
(29) N-(2-Fluoro-4-{[2-({[(3S)-3-hydroxypyrrolidin-1-yl]carbonyl}amino)pyridin-4-yl]oxy}phenyl)-N'-(4-fluorophenyl)cyclopropane-1,1-dicarboxamide,
(30) N-[4-({2-[(Azetidin-1-ylcarbonyl)amino]pyridin-4-yl}oxy)-2,5-difluorophenyl]-N'-(4-fluorophenyl)cyclopropane-1,1-dicarboxamide,
(31) N-{2,5-Difluoro-4-[(2-{[(3-hydroxyazetidin-1-yl)carbonyl]amino}pyridin-4-yl)oxy]phenyl}-N'-(4-fluorophenyl)cyclopropane-1,1-dicarboxamide,
(32) N-(2,5-Difluoro-4-{[2-({[4-(4-methylpiperazin-1-yl)piperidin-1-yl]carbonyl}amino)pyridin-4-yl]oxy}phenyl)-N'-(4-fluorophenyl)cyclopropane-1,1-dicarboxamide,
(33) N-[2,5-Difluro-4-({2-[({3-[(dimethylamino)methyl]azetidin-1-yl}carbonyl)amino]pyridin-4-yl}oxy)phenyl]-N'-(4-fluorophenyl)cyclopropane-1,1-dicarboxamide,
(34) N-(2,5-Difluoro-4-{[2-({[methyl(1-methylpiperidin-4-yl)amino]carbonyl}amino)pyridin-4-yl]oxy}phenyl)-N'-(4-fluorophenyl)cyclopropane-1,1-dicarboxamide,
(35) N-{4-[(2-{[3-(Azetidin-1-ylmethyl)azetidin-1-ylcarbonyl]amino}pyridin-4-yl)oxy]-2,5-difluorophenyl}-N'-(4-fluoropheyl)cyclopropane-1,1-dicarboxamide,
(36) N-(2,5-Difluoro-4-{[2-({[3-(hydroxymethyl)azetidin-1-yl]carbonyl}amino)pyridin-4-yl]oxy}phenyl)-N'-(4-fluorophenyl)cyclopropane-1,1-dicarboxamide,
(37) N-{2,5-Difluoro-4-[(4-{[(3-hydroxyazetidin-1-yl)carbonyl]amino}pyrimidin-6-yl)oxy]phenyl}-N'-(4-fluorophenyl)cyclopropane-1,1-dicarboxamide,
(38) N-[4-({4-[({3-[(Dimethylamino)methyl]azetidin-1-yl}carbonyl)amino]pyrimidin-6-yl}oxy)-2,5-difluorophenyl]-N'-(4-fluorophenyl)cyclopropane-1,1-dicarboxamide,
(39) N-(2,5-Difluoro-4-{[4-({[3-(hydroxymethyl)azetidin-1-yl]carbonyl}amino)pyrimidin-6-yl]oxy}phenyl)-N'-(4-fluorophenyl)cyclopropane-1,1-dicarboxamide,
(40) N-(2,5-Difluoro-4-{[4-({[methyl(1-methylpiperidin-4-yl)amino]carbonyl}amino)pyrimidin-6-yl]oxy}phenyl)-N' -(4-fluorophenyl)cyclopropane-1,1-dicarboxamide,
(41) N-(2,5-Difluoro-4-{[4-({[4-(4-methylpiperazin-1-yl)piperidin-1-yl]carbonyl}amino)pyrimidin-6-yl]oxy}phenyl)-N'-(4-fluorophenyl)cyclopropane-1,1-dicarboxamide,
(42) N-(4-{[2-({[4-(Dimethylamino)piperidin-1-yl]carbonyl}amino)pyridin-4-yl]oxy}-2,5-difluorophenyl)-N'-(4-fluorophenyl)cyclopropane-1,1-dicarboxamide,
(43) N-{2,5-Difluoro-4-[(2-{[(4-methylpiperazin-1-yl)carbonyl]amino}pyridin-4-yl]oxy}phenyl)-N'-(4-fluorophenyl)cyclopropane-1,1-dicarboxamide,
(44) N-{2,5-difluoro-4-[(2-{[(4-hydroxypiperidin-1-yl)carbonyl]amino}pyridin-4-yl)oxy]phenyl}-N'-(4-fluorophenyl)cyclopropane-1,1-dicarboxamide,
(45) N-{4-[(2-{[(4-Azetidin-1-ylpiperidin-1-yl)carbonyl]amino}pyridin-4-yl)oxy]oxy}-2,5-difluorophenyl}-N'-(4-fluorophenyl)cyclopropane-1,1-dicarboxamide,
(46) N-(2,5-Difluoro-4-{[2-({[3-(2-dimethylaminoacetoxy)azetidin-1-\yl]carbonyl}amino)pyridin-4-yl]oxy}phenyl)-N'-(4-fluorophenyl)cyclopropane-1,1-dicarboxamide,
(47) N-(2,5-Difluoro-4-{[2-({[(3S)-3-hydroxypyrrolidin-1-yl]carbonyl}amino)pyridin-4-yl]oxy}phenyl)-N'-(4-fluorophenyl)cyclopropane-1,1-dicarboxamide,
(48) N-(2,5-Difluoro-4-{[2-({[(3R)-3-hydroxypyrrolidin-1-yl]carbonyl}amino)pyridin-4-yl]oxy}phenyl)-N'-(4-fluorophenyl)cyclopropane-1,1-dicarboxamide or
(49) N-(3-Fluoro-4-{[6-({[methyl(1-methylpiperidin-4-yl)amino]carbonyl}amino)pyrimidin-4-yl]oxy}phenyl)-N'-(4-fluorophenyl)cyclopropane-1,1-dicarboxamide.

26. The method according to any one of claims 1 to 6, wherein the compound represented by the formula (I) is
(1) N-(2-Fluoro-4-{[2-({[4-(4-methylpiperazin-1-yl)piperidin-1-yl]carbonyl}amino)pyridin-4-yl]oxy}phenyl)-N'-(4-fluorophenyl)cyclopropane-1,1-dicarboxamide,
(2) N-[4-({2-[(Azetidin-1-ylcarbonyl)amino]pyridin-4-yl}oxy)-2-fluorophenyl]-N'-(4-fluorophenyl)cyclopropane-1,1-dicarboxamide,
(3) N-{2,5-Difluoro-4-[(2-{[(3-hydroxyazetidin-1-yl)carbonyl]amino}pyridin-4-yl)oxy]phenyl}-N'-(4-fluorophenyl)cyclopropane-1,1-dicarboxamide,
(4) N-(2,5-Difluoro-4-{[2-({[4-(4-methylpiperazin-1-yl)piperidin-1-yl]carbonyl}amino)pyridin-4-yl]oxy}phenyl)-N'-(4-fluorophenyl)cyclopropane-1,1-dicarboxamide,
(5) N-(2,5-Difluoro-4-{[2-({[methyl(1-methylpiperidin-4-yl)amino]carbonyl}amino)pyridin-4-yl]oxy}phenyl)-N'-(4-fluorophenyl)cyclopropane-1,1-dicarboxamide or
(6) N-(2,5-Difluoro-4-{[2-({[3-(hydroxymethyl)azetidin-1-yl]carbonyl}amino)pyridin-4-yl]oxy}phenyl)-N'-(4-fluorophenyl)cyclopropane-1,1-dicarboxamide.

27. A method for examining sensitivity of tumor cells to a pyridine or pyrimidine derivative comprising the steps of:
assaying expression levels of hepatocyte growth factor receptor in tumor cells extracted from a tumor patient before and after administration of a pyridine or pyrimidine derivative; and
determining that the tumor cells are sensitive to the pyridine or pyrimidine derivative if the expression level of hepatocyte growth factor receptor after administration of the pyridine or pyrimidine derivative is lower than the expression level of hepatocyte growth factor receptor before administration of the pyridine or pyrimidine derivative,
wherein the pyridine or pyrimidine derivative is at least one compound, salt thereof or solvate of the foregoing selected from the compound represented by the formula (I): 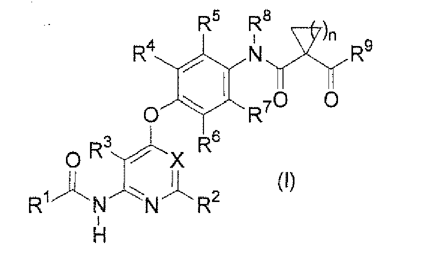
wherein R¹ represents a 3- to 10-membered non-aromatic heterocyclic group wherein the group is limited to a group having nitrogen as a ring constituent atom and the nitrogen having a bonding hand, or a group represented by the formula -NR^{11a}R^{11b}, wherein R^{11a} and R^{11b} may be the same or different and each represents hydrogen, C₁₋₆ alkyl, C₃₋₆ alkenyl, C₃₋₆ alkynyl, C₃₋₁₀ cycloalkyl, C₆₋₁₀ aryl, 5- to 10-membered heteroaryl or a 4- to 10-membered non-aromatic heterocyclic group, and R^{11a} and R^{11b} may be substituted with a substituent selected from Substituent Group A or Substituent Group B and R¹ may be substituted with a substituent selected from Substituent Group A or Substituent Group B;
R² and R³ represent hydrogen;
R⁴, R⁵, R⁶ and R⁷ may be the same or different and each represents hydrogen, halogen, hydroxyl, cyano, trifluoromethyl, C₁₋₆ alkyl, C₂-₆ alkenyl, C₂₋₆ alkynyl, C₁₋₆ alkoxy, amino, mono-C₁₋₆ alkylamino, di-C₁₋₆ alkylamino or a group represented by the formula -CO-R¹², wherein R¹² represents hydrogen, hydroxyl, C₁₋₆ alkyl, C₁₋₆ alkoxy, amino, mono-C₁₋₆ alkylamino or di-C₁₋₆ alkylamino;
R⁸ represents hydrogen or C₁₋₆ alkyl;
R⁹ represents a 3- to 10-membered non-aromatic heterocyclic group wherein the group is limited to a group having nitrogen as a ring constituent atom and the nitrogen having a bonding hand, or a group represented by the formula - NR^{11a}R^{11b} wherein R^{11a} and R^{11b} represent the same meaning as described above and R⁹ may be substituted with a substituent selected from Substituent Group A or Substituent Group B;
n represents an integer of 1 or 2; and
X represents a group represented by the formula -C(R¹⁰)= or nitrogen,
wherein R¹⁰ represents hydrogen, halogen, cyano, C₁₋₆ alkyl, C₂₋₆ alkenyl, C₂₋₆ alkynyl or a group represented by the formula -CO-R¹², wherein R¹² represents the same meaning as recited above;
wherein Substituent Group A consists of halogen, hydroxyl, mercapto, nitro, cyano and oxo;
wherein Substituent Group B consists of C₁₋₆ alkyl, C₂₋₆ alkenyl, C₂₋₆ alkynyl, C₃₋₁₀ cycloalkyl, C₆₋₁₀ aryl, 5- to 10-membered heteroaryl, a 3- to 10-membered non-aromatic heterocyclic group, C₁₋₆ alkoxy, C₃₋₆ alkenyloxy, C₃₋₆ alkynyloxy, C₃₋₁₀ cycloalkoxy, C₆₋₁₀ aryloxy, 5- to 10-membered heteroaryloxy, 4-to 10-membered non-aromatic heterocyclicoxy, C₁₋₆ alkylthio, C₃₋₆ alkenylthio, C₃₋₆ alkynylthio, C₃₋₁₀ cycloalkylthio, C₆₋₁₀ arylthio, 5- to 10-membered heteroarylthio, 4- to 10-membered non-aromatic heterocyclicthio and a group represented by the formula T¹-T²-T³, and each group in Substituent Group B may be substituted with a substituent selected from Substituent Group C, wherein T¹ represents a direct bond or C₁₋₆ alkylene, T² represents carbonyl, sulfinyl, sulfonyl, a group represented by the formula -C(=O)-O-, a group represented by the formula -O-C(=O)-, a group represented by the formula -SO₂-O-, a group represented by the formula -O-SO₂-, a group represented by the formula -NR^{T1}-, a group represented by the formula -C(=O)-NR^{T1}-, a group represented by the formula -NR^{T1}-C(=O)-, a group represented by the formula -SO₂-NR^{T1}- or a group represented by the formula -NR^{T1}-SO₂-, T³ represents hydrogen, C₁₋₆ alkyl, C₃₋₆ alkenyl, C₃₋₆ alkynyl, C₃₋₁₀ cycloalkyl, C₆₋₁₀ aryl, 5- to 10-membered heteroaryl or a 4- to 10-membered non-aromatic heterocyclic group, and R^{T1} represents hydrogen or C₁₋₆ alkyl; and
wherein Substituent Group C consists of halogen, hydroxyl, mercapto, nitro, cyano, oxo, C₁₋₆ alkyl, C₂₋₆ alkenyl, C₂₋₆ alkynyl, C₃₋₁₀ cycloalkyl, C₆₋₁₀ aryl, 5- to 10-membered heteroaryl, a 3- to 10-membered non-aromatic heterocyclic group, C₁₋₆ alkoxy, C₁₋₆ alkylthio, mono-C₁₋₆ alkylamino and di-C₁₋₆ alkylamino.

28. The method according to claim 27, wherein the method of assaying expression level of hepatocyte growth factor receptor is an immunological method.

29. The method according to claim 28, wherein the immunological method is an immunostaining method.

30. The method according to claim 27, wherein the method of assaying expression level of hepatocyte growth factor receptor is a method of assaying expression level of the gene.

31. The method according to claim 30, wherein the method of assaying expression level of the gene is a method of assaying gene amplification.

32. The method according to claim 31, wherein the method of assaying gene amplification is fluorescence *in situ* hybridization.

33. The method according to any one of claims 27 to 32, wherein R¹ represents a 3- to 10-membered non-aromatic heterocyclic group optionally substituted with a substituent selected from Substituent Group A or Substituent Group B recited in claim 27, wherein the group is limited to a group having nitrogen as a ring constituent atom and the nitrogen having a bonding hand.

34. The method according to any one of claims 27 to 32, wherein R¹ represents a group represented by the formula (II): 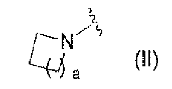 wherein a represents an integer of 1 to 4;
or a group represented by the formula (III): 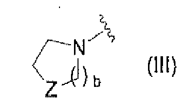 wherein b represents an integer of 1 to 3, and Z represents oxygen, sulfur, carbonyl, sulfonyl, or a group represented by the formula -NR^{Z}-, wherein R^{Z} represents hydrogen or C₁₋₆ alkyl, and the groups represented by the formula (II) or (III) may be substituted with a substituent selected from Substituent Group A or Substituent Group B recited in claim 27.

35. The method according to any one of claims 27 to 32, wherein R¹ represents azetidin-1-yl optionally substituted with a substituent selected from Substituent Group D, pyrrolidin-1-yl optionally substituted with a substituent selected from Substituent Group D, piperidin-1-yl optionally substituted with a substituent selected from Substituent Group D, azepan-1-yl optionally substituted with a substituent selected from Substituent Group D, piperazin-1-yl optionally substituted with a substituent selected from Substituent Group D, diazepan-1-yl optionally substituted with a substituent selected from Substituent Group D, morpholin-4-yl optionally substituted with a substituent selected from Substituent Group D, thiomorpholin-4-yl optionally substituted with a substituent selected from Substituent Group D, 1,1-dioxothiomorpholin-4-yl optionally substituted with a substituent selected from Substituent Group D,
wherein Substituent Group D consists of halogen, hydroxyl, mercapto, cyano, formal, oxo, C₁₋₆ alkyl, C₃₋₁₀ cycloalkyl, C₁₋₆ alkoxy, amino, mono-C₁₋₆ alkylamino, di-C₁₋₆ alkylamino, azetidinyl, pyrrolidinyl, piperidinyl, piperazinyl, diazepanyl and a group represented by -T⁴-T⁵, wherein T⁴ represents carbonyl or sulfonyl, and T⁵ represents C₁₋₆ alkyl, C₃₋₁₀ cycloalkyl, azetidinyl, pyrrolidinyl, piperidinyl, hydroxyl, C₁₋₆ alkoxy, amino, mono-C₁₋₆ alkylamino or di-C₁₋₆ alkylamino,
where each group included in Substituent Group D may be substituted with hydroxyl, C₁₋₆ alkyl, di-C₁₋₆ alkylamino, azetidinyl or pyrrolidinyl.

36. The method according to any one of claims 27 to 32, wherein R¹ represent azetidin-1-yl optionally substituted with a substituent selected from Substituent Group E, pyrrolidin-1-yl optionally substituted with a substituent selected from Substituent Group E, piperidin-1-yl optionally substituted with a substituent selected from Substituent Group E, piperazin-1-yl optionally substituted with a substituent selected from Substituent Group E, diazepan-1-yl optionally substituted with a substituent selected from Substituent Group E or morpholin-4-yl optionally substituted with a substituent selected from Substituent Group E,
wherein Substituent Group E consists of methyl, ethyl, dimethylamino, azetidinyl, pyrrolidinyl, piperidinyl and piperazinyl,
where each group included in Substituent Group E may be substituted with hydroxyl, methyl, dimethylamino, azetidinyl, pyrrolidinyl or piperidinyl.

37. The method according to any one of claims 27 to 32, wherein R¹ represents azetidin-1-yl optionally substituted with a substituent selected from Substituent Group G, pyrrolidin-1-yl optionally substituted with a substituent selected from Substituent Group G, piperidin-1-yl optionally substituted with a substituent selected from Substituent Group G or piperazin-1-yl optionally substituted with a substituent selected from Substituent Group G,
wherein Substituent Group G consists of dimethylamino, azetidinyl, pyrrolidinyl, piperidinyl, piperazinyl, dimethylaminomethyl, dimethylaminoethyl, azetidin-1-ylmethyl, pyrrolidin-1-ylmethyl and piperidin-1-ylmethyl,
where each group included in Substituent Group G may be substituted with methyl or dimethylamino.

38. The method according to any one of claims 27 to 32, wherein R¹ represents a group represented by the formula -NR^{11a}R^{11b}, wherein R^{11a} and R^{11b} represent the same meaning as recited in claim 27.

39. The method according to any one of claims 27 to 32, wherein R¹ represents a group represented by the formula -NR^{11c}R^{11d}, wherein R^{11c} represents hydrogen or C₁₋₆ alkyl, and R^{11d} represents C₁₋₆ alkyl or a group represented by the formula (IV): 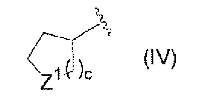 wherein c represents an integer of 1 to 3, and Z¹ represents oxygen, sulfur, carbonyl, sulfonyl or a group represented by the formula -NR^{Z1}-, wherein R^{Z1} represents hydrogen or C₁₋₆ alkyl, and R^{11d} may be substituted with a substituent selected from Substituent Group A or Substituent Group B recited in claim 27.

40. The method according to any one of claims 27 to 32, wherein R¹ represents a group represented by the formula -NR^{11e}R^{11f}, wherein R¹¹e represents hydrogen or C₁₋₆ alkyl, and R^{11f} represents C₁₋₆ alkyl, pyrrolidin-3-yl, piperidin-3-yl, piperidin-4-yl or tetrahydropyran-4-yl, and R^{11f} may be substituted with a substituent selected from Substituent Group D recited in claim 35.

41. The method according to any one of claims 27 to 32, wherein R¹ represents a group represented by the formula -NR^{11g}R^{11h}, wherein R^{11g} represents hydrogen or methyl, and R^{11h} represents n-propyl, n-butyl, pyrrolidin-3-yl, piperidin-3-yl, piperidin-4-yl or tetrahydropyran-4-yl, and R^{11h} may be substituted with a substituent selected from Substituent Group F,
wherein Substituent Group F consists of methyl, ethyl, n-propyl, acetyl, dimethylamino, diethylamino, azetidinyl, pyrrolidinyl and piperazinyl,
where each group included in Substituent Group F may be substituted with methyl or dimethylamino.

42. The method according to any one of claims 27 to 32, wherein R¹ represents a group represented by the formula -N(CH₃)R¹¹ⁱ, wherein R¹¹ⁱ represents n-propyl, n-butyl, pyrrolidin-3-yl or piperidin-4-yl, and R¹¹ⁱ may be substituted with a substituent selected from Substituent Group H,
wherein Substituent Group H consists of dimethylamino, diethylamino, dimethylaminoethyl, dimethylaminopropyl and 1-methylazetidin-3-yl,

43. The method according to any one of claims 27 to 32, wherein R¹ represents a group represented by the formula N(CH₃)R^{11j}, wherein R^{11j} represents 1-methylpiperidin-4-yl or 1-ethylpiperidin-4-yl.

44. The method according to any one of claims 27 to 43, wherein R⁴, R⁵, R⁶ and R⁷ may be the same or different and each represents hydrogen, halogen or C₁₋₆ alkyl.

45. The method according to any one of claims 27 to 44, wherein R⁸ represents hydrogen.

46. The method according to any one of claims 27 to 45, wherein X represents a group represented by the formula -C(R^{10a})=, wherein R^{10a} represents hydrogen, halogen or cyano.

47. The method according to any one of claims 27 to 45, wherein X represents nitrogen.

48. The method according to any one of claims 27 to 47, wherein n represents 1.

49. The method according to any one of claims 27 to 48, wherein R⁹ represents mono-C₁₋₆ alkylamino optionally substituted with a substituent selected from Substituent Group A or Substituent Group B recited in claim 27, mono-C₃₋₁₀ cycloalkylamino optionally substituted with a substituent selected from Substituent Group A or Substituent Group B recited in claim 27, mono-C₆₋₁₀ arylamino optionally substituted with a substituent selected from Substituent Group A or Substituent Group B recited in claim 27, mono-5- to 10-membered heteroarylamino optionally substituted with a substituent selected from Substituent Group A or Substituent Group B recited in claim 27 or mono-4- to 10-membered non-aromatic heterocyclic amino optionally substituted with a substituent selected from Substituent Group A or Substituent Group B recited in claim 27.

50. The method according to any one of claims 27 to 48, wherein R⁹ represents mono-C₃₋₁₀ cycloalkylamino optionally substituted with a substituent selected from Substituent Group A or Substituent Group B recited in claim 27 or mono-C₆₋₁₀ arylamino optionally substituted with a substituent selected from Substituent Group A or Substituent Group B recited in claim 27.

51. The method according to any one of claims 27 to 32, wherein the compound represented by the formula (I) is
(1) N-[4-({2-[({4-[2-(Dimethylamino)ethyl]piperazin-1-yl}carbonyl)amino]pyridin-4-yl}oxy)-2-fluorophenyl]-N'-(4-fluorophenyl)cyclopropane-1,1-dicarboxamide,
(2) N-(2-Fluoro-4-{[2-({[methyl(1-methylpiperidin-4-yl)amino]carbonyl}amino)pyridin-4-yl]oxy}phenyl)-N'-(4-fluorophenyl)cyclopropane-1,1-dicarboxamide,
(3) N-(4-Fluorophenyl)-N'-{2-fluoro-4-[(2-{[(4-pyrrolidin-1-ylpipendin-1-yl)carbonyl]amino}pyridin-4-yl)oxy]phenyl}cyclopropane-1,1-dicarboxamide,
(4) N-[4-({2-[({4-[(Dimethylamino)methyl]piperidin-1-yl}carbonyl)amino]pyridin-4-yl}oxy)-2-fluorophenyl]-N'-(4-fluorophenyl)cyclopropane-1,1-dicarboxamide,
(5) N-{4-[(2-{[(4-Azetidin-1-ylpiperidin-1-yl)carbonyl]amino}pyridin-4-yl)oxy]-2-fluoropheny}-N'-(4-fluorophenyl)cyclopropane-1,1-dicarboxamide,
(6) N-[4-({2-[({4-[3-(Dimethylamino)azetidin-1-yl]piperidin-1-yl}carbonyl)amino]pyridin-4-yl}oxy)-2-fluorophenyl]-N'-(4-fluorophenyl)cyclopropane-1,1-dicarboxamide,
(7) N-(2-Fluoro-4-{[2-({[4-(4-methylpiperazin-1-yl)piperidin-1-yl]carbonyl}amino)pyridin-4-yl]oxy}phenyl)-N'-(4-fluorophenyl)cyclopropane-1,1-dicarboxamide,
(8) N-(2-Fluoro-4-{[2-({[4-(1-methylpiperidin-4-yl)piperazin-1-yl]carbonyl}amino)pyridin-4-yl]oxy}phenyl)-N'-(4-fluorophenyl)cyclopropane-1,1-dicarboxamide,
(9) N-(2-Fluoro-4-{[2-({[4-(1-methylazetidin-3-yl)piperazin-1-yl]carbonyl}amino)pyridin-4-yl]oxy}phenyl)-N'-(4-fluorophenyl)cyclopropane-1,1-dicarboxamide,
(10) N-(4-{[2-({[4-(Dimethylamino)piperidin-1-yl]carbonyl}amino)pyridin-4-yl]oxy}-2-fluorophenyl)-N'-(4-fluorophenyl)cyclopropane-1,1-dicarboxamide,
(11) N-(4-{[2-({[4-(Azetidin-1-ylmethyl)pipendin-1-yl]carbonyl}amino)pyridin-4-yl]oxy}-2-fluorophenyl)-N'-(4-fluorophenyl)cyclopropane-1,1-dicarboxamide,
(12) N-(4-Fluorophenyl)-N'-(2-fluoro-4-{[2-({[4-(pyrrolidin-1-ylmethyl)piperidin-1-yl]carbonyl}amino)pyridin-4-yl]oxy}phenyl)cyclopropane-1,1-dicarboxamide,
(13) N-(4-{[2-({[(3S)-3-(Dimethylamino)pyrrolidin-1-yl]carbonyl}amino)pyridin-4-yl]oxy}-2-fluorophenyl)-N'-(4-fluorophenyl)cyclopropane-1,1-dicarboxamide,
(14) N-(4-{[2-({[(3R)-3-(Dimethylamino)pyrrolidin-1-yl]carbonyl}amino)pyridin-4-yl]oxy}-2-fluorophenyl)-N'-(4-fluorophenyl)cyclopropane-1,1-dicarboxamide,
(15) N-(2-Fluoro-4-{[2-({[methyl(1-methylpiperdin-4-yl)amino]carbonyl}amino)pyridin-4-yl]oxy}phenyl)-N'-phenylcyclopropane-1,1-dicarboxamide,
(16) N-(2-Fluoro-4-{[2-({[4-(4-methylpiperazin-1-yl)piperidin-1-yl]carbonyl}amino)pyridin-4-yl]oxy}phenyl)-N'-phenylcyclopropane-1,1-dicarboxamide,
(17) N-[4-({2-[({4-[3-(Dimethylamino)azetidin-1-yl]piperidin-1-yl}carbonyl)amino]pyridin-4-yl}oxy)-2-fluorophenyl]-N'-phenylcyclopropane-1,1-dicarboxamide,
(18) N-(4-{[2-({[(1-Ethylpiperidin-4-yl)(methyl)amino]carbonyl}amino)pyridin-4-yl]oxy}-2-fluorophenyl)-N'-phenylcyclopropane-1,1-dicarboxamide,
(19) N-[4-({2-[(Azetidin-1-ylcarbonyl)amino]pyridin-4-yl}oxy)-2-fluorophenyl]-N'-(4-fluorophenyl)cycyopropane-1,1-dicarboxamide,
(20) N-(4-Fluorophenyl)-N'-[2-fluoro-4-({2-[(pyrrolidin-1-ylcarbonyl)amino]pyridin-4-yl}oxy)phenyl]cyclopropane-1,1-dicarboxamide,
(21) N-{2-Fluoro-4-[(2-{[(3-hydroxyazetidin-1-yl)carbonyl]amino}pyridin-4-yl)oxy]phenyl}-N'-(4-fluorophenyl)cyclopropane-1,1-dicarboxamide,
(22) N-[4-({2-[(1,3'-Biazetidin-1i-ylcarbonyl)amino]pyridin-4-yl}oxy)-2-fluorophenyl]-N'-(4-fluorophenyl)cyclopropane-1,1-dicarboxamide,
(23) N-(2-Fluoro-4-{[2-({[3-(hydroxymethyl)azetidin-1-yl]carbonyl}amino)pyridin-4-yl]oxy}phenyl)-N'-(4-fluorophenyl)cyclopropane-1,1-dicarboxamide,
(24) N-(4-{[2-({[3-(Dimethylamino)azetidin-1-yl]carbonyl}amino)pyridin-4-yl]oxy}-2-fluorophenyl)-N'-(4-fluorophenyl)cyclopropane-1,1-dicarboxamide,
(25) N-[4-({2-[({3-[(Dimethylamino)methyl]azetidin-1-yl}carbonyl)amino]pyridin-4-yl}oxy)-2-fluorophenyl]-N'-(4-fluorophenyl)cyclopropane-1,1-dicarboxamide,
(26) N-{2-Fluoro-4-[(2-{[(4-hydroxypiperidin-1-yl)carbonyl]amino}pyridin-4-yl)oxy]phenyl}-N'-(4-fluorophenyl)cyclopropane-1,1-dicarboxamide,
(27) N-(2-Fluoro-4-{[2-({[4-(hydroxyznethyl)piperidin-1-yl]carbonyl}amino)pyridin-4-yl]oxy}phenyl)-N'-(4-fluorophenyl)cyclopropane-1,1-dicarboxamide,
(28) N-(2-Fluoro-4-{[2-({[(3R)-3-hydroxypyrrolidin-1-yl]carbonyl}amino)pyridin-4-yl]oxy}phenyl)-N'-(4-fluorophenyl)cyclopropane-1,1-dicarboxamide,
(29) N-(2-Fluoro-4-{[2-({[(3S)-3-hydroxypyrrolidin-1-yl]carbonyl}amino)pyridin-4-yl]oxy}phenyl)-N'-(4-fluorophenyl)cyclopropane-1,1-dicarboxamide,
(30) N-[4-({2-[(Azetidin-1-ylcarbonyl)amino]pyridin-4-yl}oxy)-2,5-difluorophenyl]-N'-(4-fluorophenyl)cyclopropane-1,1-dicarboxamide,
(31) N-{2,5-Difluoro-4-[(2-{[(3-hydroxyazetidin-1-yl)carbonyl]amino}pyridin-4-yl)oxy]phenyl}-N'-(4-fluorophenyl)cyclopropane-1,1-dicarboxamide,
(32) N-(2,5-Difluoro-4-{[2-({[4-(4-methylpiperazin-1-yl)piperidin-1-yl]carbonyl}amino)pyridin-4-yl]oxy}phenyl)-N'-(4-fluorophenyl)cyclopropane-1,1-dicarboxamide,
(33) N-[2,5-Difluoro-4-({2-[({3-[(dimethylamino)methyl]azetidin-1-yl}carbonyl)amino]pyridin-4-yl}oxy)phenyl]-N'-(4-fluorophenyl)cyclopropane-1,1-dicarboxamide,
(34) N-(2,5-Difluoro-4-{[2-({[methyl(1-methylpiperidin-4-yl)amino]carbonyl}amino)pyridin-4-yl]oxy}phenyl)-N'-(4-fluorophenyl)cyclopropane-1,1-dicarboxamide,
(35) N-{4-[(2-{[3-(Azetidin-1-ylmethyl)azetidin-1-ylcarbonyl]amino}pyridin-4-yl)oxy]-2,5-difluorophenyl}-N'-(4-fluorophenyl)cyclopropane-1,1-dicarboxamide,
(36) N-(2,5-Difluoro-4-{[2-({[3-(hydroxymethyl)azetidin-1-yl]carbonyl}amino)pyridin-4-yl]oxy}phenyl)-N'-(4-fluorophenyl)cyclopropane-1,1-dicarboxamide,
(37) N-{2,5-Difluoro-4-[(4-{[(3-hydroxyazetidin-1-yl)carbonyl]aimno}pyrimidin-6-yl)oxy]phenyl}-N'-(4-fluorophenyl)cyclopropane-1,1-dicarboxamide,
(38) N-[4-({4-[({3-[(Dimethylamino)methyl]azetidin-1-yl} carbonyl)amino]pyrimidin-6-yl}oxy)-2,5-difluorophenyl]-N'-(4-fluorophenyl)cyclopropane-1,1-dicarboxamide,
(39) N-(2,5-Difluoro-4-{[4-({[3-(hydroxymethyl)azetidin-1-yl]carbonyl}amino)pyrimidin-6-yl]oxy}phenyl)-N'-(4-fluorophenyl)cyclopropane-1,1-dicarboxamide,
(40) N-(2,5-Difluoro-4-{[4-({[methyl(1-methylpipendin-4-yl)amino]carbonyl}amino)pyrimidin-6-yl]oxy}phenyl)-N'-(4-fluorophenyl)cyclopropane-1,1-dicarboxamide,
(41) N-(2,5-Difluoro-4-{[4-({[4-(4-methylpiperazin-1-yl)piperidin-1-yl]carbonyl}amino)pyrimidin-6-yl]oxy}phenyl)-N'-(4-fluorophenyl)cyclopropane-1,1-dicarboxamide,
(42) N-(4-{[2-({[4-(Dimethylamino)piperidin-1-yl]carbonyl}amino)pyridin-4-yl]oxy}-2,5-difluorophenyl)-N'-(4-fluorophenyl)cyclopropane-1,1-dicarboxamide,
(43) N-{2,5-Difluoro-4-[(2-{[(4-methylpiperazin-1-yl)carbonyl]amino}pyridin-4-yl]oxy}phenyl)-N'-(4-fluorophenyl)cyclopropane-1,1-dicarboxamide,
(44) N-{2,5-Difluoro-4-[(2-{[(4-hydroxypiperidin-1-yl)carbonyl]amino}pyridin-4-yl)oxy]phenyl}-N'-(4-fluorophenyl)cyclopropane-1,1-dicarboxamide,
(45) N-{4-[(2-{[(4-Azetidin-1-ylpiperidin-1-yl)carbonyl]amino}pyridin-4-yl)oxy]oxy}-2,5-difluorophenyl}-N'-(4-fluorophenyl)cyclopropane-1,1-dicarboxamide,
(46) N-(2,5-Difluoro-4-{[2-({[3-(2-dimethylaminoacetoxy)azetidin-1-yl]carbonyl}amino)pyridin-4-yl]oxy}phenyl)-N'-(4-fluorophenyl)cyclopropane-1,1-dicarboxamide,
(47) N-(2,5-Difluoro-4-{[2-({[(3S)-3-hydroxypyrrolidin-1-yl]carbonyl}amino)pyridin-4-yl]oxy}phenyl)-N'-(4-fluorophenyl)cylopropane-1,1-dicarboxamide,
(48) N-(2,5-Difluoro-4-{[2-({[(3R)-3-hydroxypyrrolidin-1-yl]carbonyl}amino)pyridin-4-yl]oxy}phenyl)-N'-(4-fluorophenyl)cyclopropane-1,1-dicarboxamide or
(49) N-(3-Fluoro-4-{[6-({[methyl(1-methylpiperidin-4-yl)amino]carbonyl}amino)pyrimidin-4-yl]oxy}phenyl)-N'-(4-fluorophenyl)cyclopropane-1,1-dicarboxamide.

52. The method according to any one of claims 27 to 32, wherein the compound represented by the formula (I) is
(1) N-(2-Fluoro-4-{[2-({[4-(4-methylpiperazin-1-yl)piperidin-1-yl]carbonyl}amino)pyridin-4-yl]oxy}phenyl)-N'-(4-fluorophenyl)cyclopropane-1,1-dicarboxamide,
(2) N-[4-({2-[(Azetidin-1-ylcarbonyl)amino]pyridin-4-yl}oxy)-2-fluorophenyl]-N'-(4-fluorophenyl)cyclopropane-1,1-dicarboxamide,
(3) N-{2,5-Difluoro-4-[(2-{[(3-hydroxyazetidin-1-yl)carbonyl]amino}pyridin-4-yl)oxy]phenyl}-N'-(4-fluorophenyl)cyclopropane-1,1-dicarboxamide,
(4) N-(2,5-Difluoro-4-{[2-({[4-(4-methylpiperazin-1-yl)piperidin-1-yl]carbonyl}amino)pyridin-4-yl]oxy}phenyl)-N'-(4-fluorophenyl)cyclopropane-1,1-dicarboxamide,
(5) N-(2,5-Difluoro-4-{[2-({[methyl(1-methylpiperidin-4-yl)amino]carbonyl}amino)pyridin-4-yl]oxy}phenyl)-N'-(4-fluorophenyl)cyclopropane-1,1-dicarboxamide or
(6) N-(2,5-Difluoro-4-{[2-({[3-(hydroxymethyl)azetidin-1-yl]carbonyl}amino)pyridin-4-yl]oxy}phenyl)-N'-(4-fluorophenyl)cyclopropane-1,1-dicarboxamide.

53. A pharmaceutical composition against tumors in which expression of hepatocyte growth factor receptor is enhanced, comprising at least one compound, salt thereof or solvate of the foregoing selected from the compound represented by the formula (I): 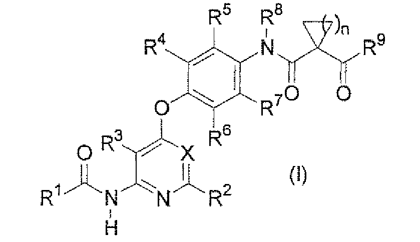 wherein R¹ represents a 3- to 10-membered non-aromatic heterocyclic group wherein the group is limited to a group having nitrogen as a ring constituent atom and the nitrogen having a bonding hand, or a group represented by the formula -NR^{11a}R^{11b} wherein R^{11a} and R^{11b} may be the same or different and each represents hydrogen, C₁₋₆ alkyl, C₃₋₆ alkenyl, C₃₋₆ alkynyl, C₃₋₁₀ cycloalkyl, C₆₋₁₀ aryl, 5- to 10-membered heteroaryl or a 4- to 10-membered non-aromatic heterocyclic group, and R^{11a} and R^{11b} may be substituted with a substituent selected from Substituent Group A or Substituent Group B and R¹ may be substituted with a substituent selected from Substituent Group A or Substituent Group B;
R² and R³ represent hydrogen;
R⁴, R⁵, R⁶ and R⁷ may be the same or different and each represents hydrogen, halogen, hydroxyl, cyano, trifluoromethyl, C₁₋₆ alkyl, C₂₋₆ alkenyl, C₂₋₆ alkynyl, C₁₋₆ alkoxy, amino, mono-C₁₋₆ alkylamino, di-C₁₋₆ alkylamino or a group represented by the formula -CO-R¹², wherein R¹² represents hydrogen, hydroxyl, C₁₋₆ alkyl, C₁₋₆ alkoxy, amino, mono-C₁₋₆ alkylamino or di-C₁₋₆ alkylamino;
R⁸ represents hydrogen or C₁₋₆ alkyl;
R⁹ represents a 3- to 10-membered non-aromatic heterocyclic group wherein the group is limited to a group having nitrogen as a ring constituent atom and the nitrogen having a bonding hand, or a group represented by the formula - NR^{11a}R^{11b}, wherein R^{11a} and R^{11b} represent the same meaning as described above and R⁹ may be substituted with a substituent selected from Substituent Group A or Substituent Group B;
n represents an integer of 1 or 2; and
X represents a group represented by the formula -C(R¹⁰)= or nitrogen, wherein R¹⁰ represents hydrogen, halogen, cyano, C₁₋₆ alkyl, C₂₋₆ alkenyl, C₂₋₆ alkynyl or a group represented by the formula -CO-R¹², wherein R¹² represents the same meaning as recited above;
wherein Substituent Group A consists of halogen, hydroxyl, mercapto, nitro, cyano and oxo;
wherein Substituent Group B consists of C₁₋₆ alkyl, C₂₋₆ alkenyl, C₂₋₆ alkynyl, C₃₋₁₀ cycloalkyl, C₆₋₁₀ aryl, 5- to 10-membered heteroaryl, a 3- to 10-membered non-aromatic heterocyclic group, C₁₋₆ alkoxy, C₃₋₆ alkenyloxy, C₃₋₆ alkynyloxy, C₃₋₁₀ cycloalkoxy, C₆₋₁₀ aryloxy, 5- to 10-membered heteroaryloxy, 4-to 10-membered non-aromatic heterocyclicoxy, C₁₋₆ alkylthio, C₃₋₆ alkenylthio, C₃₋₆ alkynylthio, C₃₋₁₀ cycloalkylthio, C₆₋₁₀ arylthio, 5- to 10-membered heteroarylthio, 4- to 10-membered non-aromatic heterocyclicthio and a group represented by the formula T¹-T²-T³, and each group in Substituent Group B may be substituted with a substituent selected from Substituent Group C, wherein T¹ represents a direct bond or C₁₋₆ alkylene, T² represents carbonyl, sulfinyl, sulfonyl, a group represented by the formula -C(=O)-O-, a group represented by the formula -O-C(=O)-, a group represented by the formula -SO₂-O, a group represented by the formula -O-SO₂-, a group represented by the formula -NR^{T1}-, a group represented by the formula -C(=O)-NR^{T1}-, a group represented by the formula -NR^{T1}-C(=O)-, a group represented by the formula -SO₂-NR^{T1}- or a group represented by the formula -NR^{T1}-SO₂-, T³ represents hydrogen, C₁₋₆ alkyl, C₃₋₆ alkenyl, C₃₋₆ alkynyl, C₃-₁₀ cycloalkyl, C₆₋₁₀ aryl, 5- to 10-membered heteroaryl or a 4- to 10-membered non-aromatic heterocyclic group, and R^{T1} represents hydrogen or C₁₋₆ alkyl; and
wherein Substituent Group C consists of halogen, hydroxyl, mercapto, nitro, cyano, oxo, C₁₋₆ alkyl, C₂₋₆ alkenyl, C₂₋₆ alkynyl, C₃₋₁₀ cycloalkyl, C₆₋₁₀ aryl, 5- to 10-membered heteroaryl, a 3- to 10-membered non-aromatic heterocyclic group, C₁₋₆ alkoxy, C₁₋₆ alkylthio, mono-C₁₋₆ alkylamino and di-C₁₋₆ alkylamino.

54. A hepatocyte growth factor receptor inhibitor against tumors in which expression of hepatocyte growth factor receptor is enhanced, comprising at least one compound, salt thereof or solvate of the foregoing selected from the compound represented by the formula (I): 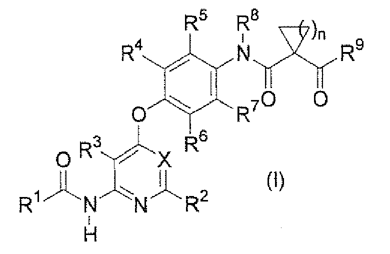 wherein R¹ represents a 3- to 10-membered non-aromatic heterocyclic group wherein the group is limited to a group having nitrogen as a ring constituent atom and the nitrogen having a bonding hand, or a group represented by the formula -NR^{11a}R^{11b}, wherein R^{11a} and R^{11b} may be the same or different and each represents hydrogen, C₁₋₆ alkyl, C₃₋₆ alkenyl, C₃₋₆ alkynyl, C₃₋₁₀ cycloalkyl, C₆₋₁₀ aryl, 5- to 10-membered heteroaryl or a 4- to 10-membered non-aromatic heterocyclic group, and R^{11a} and R^{11b} may be substituted with a substituent selected from Substituent Group A or Substituent Group B and R¹ may be substituted with a substituent selected from Substituent Group A or Substituent Group B;
R² and R³ represent hydrogen;
R⁴, R⁵, R⁶ and R⁷ may be the same or different and each represents hydrogen, halogen, hydroxyl, cyano, trifluoromethyl, C₁₋₆ alkyl, C₂₋₆ alkenyl, C₂₋₆ alkynyl, C₁₋₆ alkoxy, amino, mono-C₁₋₆ alkylamino, di-C₁₋₆ alkylamino or a group represented by the formula -CO-R¹², wherein R¹² represents hydrogen, hydroxyl, C₁₋₆ alkyl, C₁₋₆ alkoxy, amino, mono-C₁₋₆ alkylamino or di-C₁₋₆ alkylamino;
R⁸ represents hydrogen or C₁₋₆ alkyl;
R⁹ represents a 3- to 10-membered non-aromatic heterocyclic group
wherein the group is limited to a group having nitrogen as a ring constituent atom and the nitrogen having a bonding hand, or a group represented by the formula - NR^{11a}R^{11b}, wherein R^{11a} and R^{11b} represent the same meaning as described above and R⁹ may be substituted with a substituent selected from Substituent Group A or Substituent Group B;
n represents an integer of 1 or 2; and
X represents a group represented by the formula -C(R¹⁰)= or nitrogen, wherein R¹⁰ represents hydrogen, halogen, cyano, C₁₋₆ alkyl, C₂₋₆ alkenyl, C₂₋₆ alkynyl or a group represented by the formula -CO-R¹², wherein R¹² represents the same meaning as recited above;
wherein Substituent Group A consists of halogen, hydroxyl, mercapto, nitro, cyano and oxo;
wherein Substituent Group B consists of C₁₋₆ alkyl, C₂₋₆ alkenyl, C₂₋₆ alkynyl, C₃₋₁₀ cycloalkyl, C₆₋₁₀ aryl, 5- to 10-membered heteroaryl, a 3- to 10-membered non-aromatic heterocyclic group, C₁₋₆ alkoxy, C₃₋₆ alkenyloxy, C₃₋₆ alkynyloxy, C₃₋₁₀ cycloalkoxy, C₆₋₁₀ aryloxy, 5- to 10-membered heteroaryloxy, 4-to 10-membered non-aromatic heterocyclicoxy, C₁₋₆ alkylthio, C₃₋₆ alkenylthio, C₃₋₆ alkynylthio, C₃₋₁₀ cycloalkylthio, C₆₋₁₀ arylthio, 5- to 10-membered heteroarylthio, 4- to 10-membered non-aromatic heterocyclicthio and a group represented by the formula T¹-T²-T³, and each group in Substituent Group B may be substituted with a substituent selected from Substituent Group C, wherein T¹ represents a direct bond or C₁₋₆ alkylene, T² represents carbonyl, sulfinyl, sulfonyl, a group represented by the formula -C(=O)-O-, a group represented by the formula -O-C(=O)-, a group represented by the formula -SO₂-O-, a group represented by the formula -O-SO₂-, a group represented by the formula -NR^{T1}-, a group represented by the formula -C(=O)-NR^{T1}-, a group represented by the formula -NR^{T1}-C(=O)-, a group represented by the formula -SO₂-NR^{T1}- or a group represented by the formula -NR^{T1}-SO₂-, T³ represents hydrogen, C₁₋₆ alkyl, C₃₋₆ alkenyl, C₃₋₆ alkynyl, C₃₋₁₀ cycloalkyl, C₆₋₁₀ aryl, 5- to 10-membered heteroaryl or a 4- to 10-membered non-aromatic heterocyclic group, and R^{T1} represents hydrogen or C₁₋₆ alkyl; and
wherein Substituent Group C consists of halogen, hydroxyl, mercapto, nitro, cyano, oxo, C₁₋₆ alkyl, C₂₋₆ alkenyl, C₂₋₆ alkynyl, C₃₋₁₀ cycloalkyl, C₆₋₁₀ aryl, 5- to 10-membered heteroaryl, a 3- to 10-membered non-aromatic heterocyclic group, C₁₋₆ alkoxy, C₁₋₆ alkylthio, mono-C₁₋₆ alkylamino and di-C₁₋₆ alkylamino.

55. An anti-tumor agent against tumors in which expression of hepatocyte growth factor receptor is enhanced, comprising at least one compound, salt thereof or solvate of the foregoing selected from the compound represented by the formula (I): 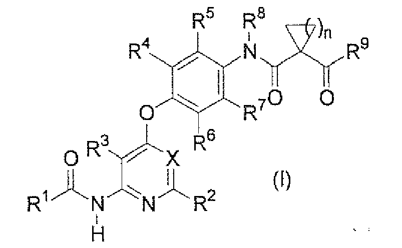 wherein R¹ represents a 3- to 10-membered non-aromatic heterocyclic group wherein the group is limited to a group having nitrogen as a ring constituent atom and the nitrogen having a bonding hand, or a group represented by the formula -NR^{11a}R^{11b}, wherein R^{11a} and R^{11b} may be the same or different and each represents hydrogen, C₁₋₆ alkyl, C₃₋₆ alkenyl, C₃₋₆ alkynyl, C₃₋₁₀ cycloalkyl, C₆₋₁₀ aryl, 5- to 10-membered heteroaryl or a 4- to 10-membered non-aromatic heterocyclic group, and R^{11a} and R^{11b} may be substituted with a substituent selected from Substituent Group A or Substituent Group B and R¹ may be substituted with a substituent selected from Substituent Group A or Substituent Group B;
R² and R³ represent hydrogen;
R⁴, R⁵, R⁶ and R⁷ may be the same or different and each represents hydrogen, halogen, hydroxyl, cyano, trifluoromethyl, C₁₋₆ alkyl, C₂₋₆ alkenyl, C₂₋₆ alkynyl, C₁₋₆ alkoxy, amino, mono-C₁₋₆ alkylamino, di-C₁₋₆ alkylamino or a group represented by the formula -CO-R¹², wherein R¹² represents hydrogen, hydroxyl, C₁₋₆ alkyl, C₁₋₆ alkoxy, amino, mono-C₁₋₆ alkylamino or di-C₁₋₆ alkylamino;
R⁸ represents hydrogen or C₁₋₆ alkyl;
R⁹ represents a 3- to 10-membered non-aromatic heterocyclic group
wherein the group is limited to a group having nitrogen as a ring constituent atom and the nitrogen having a bonding hand, or a group represented by the formula - NR^{11a}R^{11b}, wherein R^{11a} and R^{11b} represent the same meaning as described above and R⁹ may be substituted with a substituent selected from Substituent Group A or Substituent Group B;
n represents an integer of 1 or 2; and
X represents a group represented by the formula -C(R¹⁰)= or nitrogen, wherein R¹⁰ represents hydrogen, halogen, cyano, C₁₋₆ alkyl, C₂₋₆ alkenyl, C₂₋₆ alkynyl or a group represented by the formula -CO-R¹², wherein R¹² represents the same meaning as recited above;
wherein Substituent Group A consists of halogen, hydroxyl, mercapto, nitro, cyano and oxo;
wherein Substituent Group B consists of C₁₋₆ alkyl, C₂₋₆ alkenyl, C₂₋₆ alkynyl, C₃₋₁₀ cycloalkyl, C₆₋₁₀ aryl, 5- to 10-membered heteroaryl, a 3- to 10-membered non-aromatic heterocyclic group, C₁₋₆ alkoxy, C₃₋₆ alkenyloxy, C₃₋₆ alkynyloxy, C₃₋₁₀ cycloalkoxy, C₆₋₁₀ aryloxy, 5- to 10-membered heteroaryloxy, 4-to 10-membered non-aromatic heterocyclicoxy, C₁₋₆ alkylthio, C₃₋₆ alkenylthio, C₃₋₆ alkynylthio, C₃₋₁₀ cycloalkylthio, C₆₋₁₀ arylthio, 5- to 10-membered heteroarylthio, 4- to 10-membered non-aromatic heterocyclicthio and a group represented by the formula -T¹-T²-T³, and each group in Substituent Group B may be substituted with a substituent selected from Substituent Group C, wherein T¹ represents a direct bond or C₁₋₆ alkylene, T² represents carbonyl, sulfinyl, sulfonyl, a group represented by the formula -C(=O)-O-, a group represented by the formula -O-C(=O)-, a group represented by the formula -SO₂-O-, a group represented by the formula -O-SO₂-, a group represented by the formula -NR^{T1}-, a group represented by the formula -C(=O)-NR^{T1}-, a group represented by the formula -NR^{T1}-C(=O)-, a group represented by the formula -SO₂-NR^{T1}- or a group represented by the formula -NR^{T1}-SO₂-, T³ represents hydrogen, C₁₋₆ alkyl, C₃₋₆ alkenyl, C₃₋₆ alkynyl, C₃₋₁₀ cycloalkyl, C₆₋₁₀ aryl, 5- to 10-membered heteroaryl or a 4- to 10-membered non-aromatic heterocyclic group, and R^{T1} represents hydrogen or C₁₋₆ alkyl; and
wherein Substituent Group C consists of halogen, hydroxyl, mercapto, nitro, cyano, oxo, C₁₋₆ alkyl, C₂₋₆ alkenyl, C₂₋₆ alkynyl, C₃₋₁₀ cycloalkyl, C₆₋₁₀ aryl, 5- to 10-membered heteroaryl, a 3- to 10-membered non-aromatic heterocyclic group, C₁₋₆ alkoxy, C₁₋₆ alkylthio, mono-C₁₋₆ alkylamino and di-C₁₋₆ alkylamino.

56. A method for administering a pyridine or pyrimidine derivative to a tumor patient, comprising the steps of:
assaying expression level of hepatocyte growth factor receptor in tumor cells of a tumor patient;
determining whether a pyridine or pyrimidine derivative is effective or not against the tumor by using the expression level of hepatocyte growth factor receptor as an index based on the assayed expression level; and
administering the pyridine or pyrimidine derivative to the tumor patient in case of having determined effective,
wherein the pyridine or pyrimidine derivative is at least one compound, salt thereof or solvate of the foregoing selected from the compound represented by the formula (I): 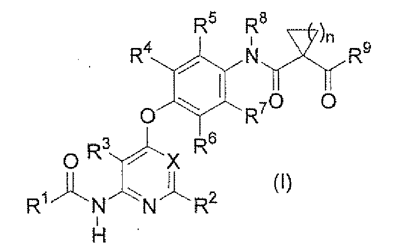
wherein R¹ represents a 3- to 10-membered non-aromatic heterocyclic group wherein the group is limited to a group having nitrogen as a ring constituent atom and the nitrogen having a bonding hand, or a group represented by the formula -NR^{11a}R^{11b}, wherein R^{11a} and R^{11b} may be the same or different and each represents hydrogen, C₁₋₆ alkyl, C₃₋₆ alkenyl, C₃₋₆ alkynyl, C₃₋₁₀ cycloalkyl, C₆₋₁₀ aryl, 5- to 10-membered heteroaryl or a 4- to 10-membered non-aromatic heterocyclic group, and R^{11a} and R^{11b} may be substituted with a substituent selected from Substituent Group A or Substituent Group B and R¹ may be substituted with a substituent selected from Substituent Group A or Substituent Group B;
R² and R³ represent hydrogen;
R⁴, R⁵, R⁶ and R⁷ may be the same or different and each represents hydrogen, halogen, hydroxyl, cyano, trifluoromethyl, C₁₋₆ alkyl, C₂₋₆ alkenyl, C₂₋₆ alkynyl, C₁₋₆ alkoxy, amino, mono-C₁₋₆ alkylamino, di-C₁₋₆ alkylamino or a group represented by the formula -CO-R¹², wherein R¹² represents hydrogen, hydroxyl, C₁₋₆ alkyl, C₁₋₆ alkoxy, amino, mono-C₁₋₆ alkylamino or di-C₁₋₆ alkylamino;
R⁸ represents hydrogen or C₁₋₆ alkyl;
R⁹ represents a 3- to 10-membered non-aromatic heterocyclic group wherein the group is limited to a group having nitrogen as a ring constituent atom and the nitrogen having a bonding hand, or a group represented by the formula - NR^{11a}R^{11b}, wherein R^{11a} and R^{11b} represent the same meaning as described above and R⁹ may be substituted with a substituent selected from Substituent Group A or Substituent Group B;
n represents an integer of 1 or 2; and
X represents a group represented by the formula -C(R¹⁰)= or nitrogen, wherein R¹⁰ represents hydrogen, halogen, cyano, C₁₋₆ alkyl, C₂₋₆ alkenyl, C₂₋₆ alkynyl or a group represented by the formula -CO-R¹², wherein R¹² represents the same meaning as recited above;
wherein Substituent Group A consists of halogen, hydroxyl, mercapto, nitro, cyano and oxo;
wherein Substituent Group B consists of C₁₋₆ alkyl, C₂₋₆ alkenyl, C₂₋₆ alkenyl, C₃₋₁₀ cycloalkyl, C₆₋₁₀ aryl, 5- to 10-membered heteroaryl, a 3- to 10-membered non-aromatic heterocyclic group, C₁₋₆ alkoxy, C₃₋₆ alkenyloxy, C₃₋₆ alkynyloxy, C₃₋₁₀ cycloalkoxy, C₆₋₁₀ aryloxy, 5- to 10-membered heteroaryloxy, 4-to 10-membered non-aromatic heterocyclicoxy, C₁₋₆ alkylthio, C₃₋₆ alkenylthio, C₃₋₆ alkynylthio, C₃₋₁₀ cycloalkylthio, C₆₋₁₀ arylthio, 5- to 10-membered heteroarylthio, 4- to 10-membered non-aromatic heterocyclicthio and a group represented by the formula -T¹-T²-T³, and each group in Substituent Group B may be substituted with a substituent selected from Substituent Group C, wherein T¹ represents a direct bond or C₁₋₆ alkylene, T² represents carbonyl, sulfinyl, sulfonyl, a group represented by the formula -C(=O)-O-, a group represented by the formula -O-C(=O)-, a group represented by the formula -SO₂-O-, a group represented by the formula -O-SO₂-, a group represented by the formula -NR^{T1}-, a group represented by the formula -C(=O)-NR^{T1}-, a group represented by the formula -NR^{T1}-C(=O)-, a group represented by the formula -SO₂-NR^{T1}- or a group represented by the formula -NR^{T1}-SO₂-, T³ represents hydrogen, C₁₋₆ alkyl, C₃₋₆ alkenyl, C₃₋₆ alkynyl, C₃₋₁₀ cycloalkyl, C₆₋₁₀ aryl, 5- to 10-membered heteroaryl or a 4- to 10-membered non-aromatic heterocyclic group, and R^{T1} represents hydrogen or C₁₋₆ alkyl; and
wherein Substituent Group C consists of halogen, hydroxyl, mercapto, nitro, cyano, oxo, C₁₋₆ alkyl, C₂₋₆ alkenyl, C₂₋₆ alkynyl, C₃₋₁₀ cycloalkyl, C₆₋₁₀ aryl, 5- to 10-membered heteroaryl, a 3- to 10-membered non-aromatic heterocyclic group, C₁₋₆ alkoxy, C₁₋₆ alkylthio, mono-C₁₋₆ alkylamino and di-C₁₋₆ alkylamino.

57. The method according to claim 56, wherein the method of assaying expression level of hepatocyte growth factor receptor is an immunological method.

58. The method according to claim 57, wherein the immunological method is an immunostaining method.

59. The method according to claim 56, wherein the method of assaying expression level of hepatocyte growth factor receptor is a method of assaying expression level of the gene.

60. The method according to claim 59, wherein the method of assaying expression level of the gene is a method of assaying gene amplification.

61. The method according to claim 60, wherein the method of assaying gene amplification is fluorescence *in situ* hybridization.

62. The method according to any one of claims 56 to 61, wherein R¹ represents a 3- to 10-membered non-aromatic heterocyclic group optionally substituted with a substituent selected from Substituent Group A or Substituent Group B recited in claim 56, wherein the group is limited to a group having nitrogen as a ring constituent atom and the nitrogen having a bonding hand.

63. The method according to any one of claims 56 to 61, wherein R¹ represents a group represented by the formula (II): 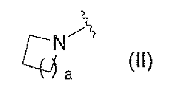 wherein a represents an integer of 1 to 4;
or a group represented by the formula (III): 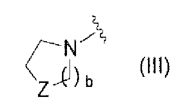 wherein b represents an integer of 1 to 3, and Z represents oxygen, sulfur, carbonyl, sulfonyl, or a group represented by the formula -NR^{Z}-, wherein R^{Z} represents hydrogen or C₁₋₆ alkyl, and the groups represented by the formula (II) or (III) may be substituted with a substituent selected from Substituent Group A or Substituent Group B recited in claim 56.

64. The method according to any one of claims 56 to 61, wherein R¹ represents azetidin-1-yl optionally substituted with a substituent selected from Substituent Group D, pyrrolidin-1-yl optionally substituted with a substituent selected from Substituent Group D, piperidin-1-yl optionally substituted with a substituent selected from Substituent Group D, azepan-1-yl optionally substituted with a substituent selected from Substituent Group D, piperazin-1-yl optionally substituted with a substituent selected from Substituent Group D, diazepan-1-yl optionally substituted with a substituent selected from Substituent Group D, morpholin-4-yl optionally substituted with a substituent selected from Substituent Group D, thiomorpholin-4-yl optionally substituted with a substituent selected from Substituent Group D, 1,1-dioxothiomorpholin-4-yl optionally substituted with a substituent selected from Substituent Group D,
wherein Substituent Group D consists of halogen, hydroxyl, mercapto, cyano, formyl, oxo, C₁₋₆ alkyl, C₃₋₁₀ cycloalkyl, C₁₋₆ alkoxy, amino, mono-C₁₋₆ alkylamino, di-C₁₋₆ alkylamino, azetidinyl, pyrrolidinyl, piperidinyl, piperazinyl, diazepanyl and a group represented by -T⁴-T⁵, wherein T⁴ represents carbonyl or sulfonyl, and T⁵ represents C₁₋₆ alkyl, C₃₋₁₀ cycloalkyl, azetidinyl, pyrrolidinyl, piperidinyl, hydroxyl, C₁₋₆ alkoxy, amino, mono-C₁₋₆ alkylamino or di-C₁₋₆ alkylamino,
where each group included in Substituent Group D may be substituted with hydroxyl, C₁₋₆ alkyl, di-C₁₋₆ alkylamino, azetidinyl or pyrrolidinyl.

65. The method according to any one of claims 56 to 61, wherein R¹ represent azetidin-1-yl optionally substituted with a substituent selected from Substituent Group E, pyrrolidin-1-yl optionally substituted with a substituent selected from Substituent Group E, piperidin-1-yl optionally substituted with a substituent selected from Substituent Group E, piperazin-1-yl optionally substituted with a substituent selected from Substituent Group E, diazepan-1-yl optionally substituted with a substituent selected from Substituent Group E or morpholin-4-yl optionally substituted with a substituent selected from Substituent Group E,
wherein Substituent Group E consists of methyl, ethyl, dimethylamino, azetidinyl, pyrrolidinyl, piperidinyl and piperazinyl,
where each group included in Substituent Group E may be substituted with hydroxyl, methyl, dimethylamino, azetidinyl, pyrrolidinyl or piperidinyl.

66. The method according to any one of claims 56 to 61, wherein R¹ represents azetidin-1-yl optionally substituted with a substituent selected from Substituent Group G, pyrrolidin-1-yl optionally substituted with a substituent selected from Substituent Group G, piperidin-1-yl optionally substituted with a substituent selected from Substituent Group G or piperazin-1-yl optionally substituted with a substituent selected from Substituent Group G,
wherein Substituent Group G consists of dimethylamino, azetidinyl, pyrrolidinyl, piperidinyl, piperazinyl, dimethylaminomethyl, dimethylaminoethyl, azetidin-1-ylmethyl, pyrrolidin-1-ylmethyl and piperidin-1-ylmethyl,
where each group included in Substituent Group G may be substituted with methyl or dimethylamino.

67. The method according to any one of claims 56 to 61, wherein R¹ represents a group represented by the formula -NR^{11a}R^{11b}, wherein R^{11a} and R^{11b} represent the same meaning as recited in claim 1.

68. The method according to any one of claims 56 to 61, wherein R¹ represents a group represented by the formula -NR^{11c}R^{11b}, wherein R^{11c} represents hydrogen or C₁₋₆ alkyl, and R^{11d} represents C₁₋₆ alkyl or a group represented by the formula (IV): 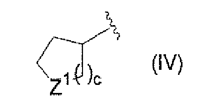 wherein c represents an integer of 1 to 3, and Z¹ represents oxygen, sulfur, carbonyl, sulfonyl or a group represented by the formula -NR^{Z1}-, wherein R^{Z1} represents hydrogen or C₁₋₆ alkyl, and R^{11d} may be substituted with a substituent selected from Substituent Group A or Substituent Group B recited in claim 56.

69. The method according to any one of claims 56 to 61, wherein R¹ represents a group represented by the formula -NR^{11e}R^{11f}, wherein R^{11e} represents hydrogen or C₁₋₆ alkyl, and R^{11f} represents C₁₋₆ alkyl, pyrrolidin-3-yl, piperidin-3-yl, piperidin-4-yl or tetrahydropyran-4-yl, and R^{11f} may be substituted with a substituent selected from Substituent Group D recited in claim 64.

70. The method according to any one of claims 56 to 61, wherein R¹ represents a group represented by the formula -NR^{11g}R^{11h}, wherein R^{11g} represents hydrogen or methyl, and R^{11h} represents n-propyl, n-butyl, pyrrolidin-3-yl, piperidin-3-yl, piperidin-4-yl or tetrahydropyran-4-yl, and R^{11h} may be substituted with a substituent selected from Substituent Group F,
wherein Substituent Group F consists of methyl, ethyl, n-propyl, acetyl, dimethylamino, diethylamino, azetidinyl, pyrrolidinyl and piperazinyl,
where each group included in Substituent Group F may be substituted with methyl or dimethylamino.

71. The method according to any one of claims 56 to 61, wherein R¹ represents a group represented by the formula -N(CH₃)R¹¹ⁱ, wherein R¹¹ⁱ represents n-propyl, n-butyl, pyrrolidin-3-yl or piperidin-4-yl, and R¹¹ⁱ may be substituted with a substituent selected from Substituent Group H,
wherein Substituent Group H consists of dimethylamino, diethylamino, dimethylaminoethyl, dimethylaminopropyl and 1-methylazetidin-3-yl.

72. The method according to any one of claims 56 to 61, wherein R¹ represents a group represented by the formula-N(CH₃)R^{11j}, wherein R^{11j} represents 1-methylpiperidin-4-yl or 1-ethylpiperidin-4-yl.

73. The method according to any one of claims 56 to 72, wherein R⁴, R⁵, R⁶ and R⁷ may be the same or different and each represents hydrogen, halogen or C₁₋₆ alkyl.

74. The method according to any one of claims 56 to 73, wherein R⁸ represents hydrogen.

75. The method according to any one of claims 56 to 74, wherein X represents a group represented by the formula -C(R^{10a})=, wherein R^{10a} represents hydrogen, halogen or cyano.

76. The method according to any one of claims 56 to 74, wherein X represents nitrogen.

77. The method according to any one of claims 56 to 76, wherein n represents 1.

78. The method according to any one of claims 56 to 77, wherein R⁹ represents mono-C₁₋₆ alkylamino optionally substituted with a substituent selected from Substituent Group A or Substituent Group B recited in claim 56, mono-C₃₋₁₀ cycloalkylamino optionally substituted with a substituent selected from Substituent Group A or Substituent Group B recited in claim 56, mono-C₆₋₁₀ arylamino optionally substituted with a substituent selected from Substituent Group A or Substituent Group B recited in claim 56, mono-5- to 10-membered heteroarylamino optionally substituted with a substituent selected from Substituent Group A or Substituent Group B recited in claim 56 or mono-4- to 10-membered non-aromatic heterocyclic amino optionally substituted with a substituent selected from Substituent Group A or Substituent Group B recited in claim 56.

79. The method according to any one of claims 56 to 77, wherein R⁹ represents mono-C₃₋₁₀ cycloalkylamino optionally substituted with a substituent selected from Substituent Group A or Substituent Group B recited in claim 56 or mono-C₆₋₁₀ arylamino optionally substituted with a substituent selected from Substituent Group A or Substituent Group B recited in claim 56.

80. The method according to any one of claims 56 to 61, wherein the compound represented by the formula (I) is
(1) N-[4-({2-[({4-[2-(Dimethylamino)ethyl]piperazin-1-yl}carbonyl)amino]pyridin-4-yl}oxy)-2-fluorophenyl]-N'-(4-fluorophenyl)cyclopropane-1,1-dicarboxamide,
(2) N-(2-Fluoro-4-{[2-({[methyl(1-methylpiperidin-4-yl)amino]carbonyl}amino)pyridin-4-yl]oxy}phenyl)-N'-(4-fluorophenyl)cyclopropane-1,1-dicarboxamide,
(3) N-(4-Fluorophenyl)-N'-{2-fluoro-4-[(2-{[(4-pyrrolidin-1-ylpiperidin-1-yl)carbonyl]amino}pyridin-4-yl)oxy]phenyl}cyclopropane-1,1-dicarboxamide,
(4) N-[4-({2-[({4-[(Dimethylamino)methyl]piperidin-1-yl}carbonyl)amino]pyridin-4-yl}oxy)-2-fluorophenyl]-N'-(4-fluorophenyl)cyclopropane-1,1-dicarboxamide,
(5) N-{4-[(2-{[(4-Azetidin-1-ylpiperidin-1-yl)carbonyl]amino}pyridin-4-yl)oxy]-2-fluorophenyl}-N'-(4-fluorophenyl)cyclopropane-1,1-dicarboxamide,
(6) N-[4-({2-[({4-[3-(Dimethylamino)azetidin-1-yl]piperidin-1-yl}carbonyl)amino]pyridin-4-yl}oxy)-2-fluorophenyl]-N'-(4-fluorophenyl)cyclopropane-1,1-dicarboxamide,
(7) N-(2-Fluoro-4-{[2-({[4-(4-methylpiperazin-1-yl)piperidin-1-yl]carbonyl}amino)pyridin-4-yl]oxy}phenyl)-N'-(4-fluorophenyl)cyclopropane-1,1-dicarboxamide,
(8) N-(2-Fluoro-4-{[2-({[4-(1-methylpiperidin-4-yl)piperazin-1-yl]carbonyl}amino)pyridin-4-yl]oxy}phenyl)-N'-(4-fluorophenyl)cyclopropane-1,1-dicarboxamide,
(9) N-(2-Fluoro-4-{[2-({[4-(1-methylazetidin-3-yl)piperazin-1-yl]carbonyl}amino)pyridin-4-yl]oxy}phenyl)-N'-(4-fluorophenyl)cyclopropane-1,1-dicarboxamide,
(10) N-(4-{[2-({[4-(Dimethylamino)piperidin-1-yl]carbonyl}amino)pyridin-4-yl]oxy}-2-fluorophenyl)-N'-(4-fluorophenyl)cyclopropane-1,1-dicarboxamide,
(11) N-(4-{[2-({[4-(Azetidin-1-ylmethyl)piperidin-1-yl]carbonyl}amino)pyridin-4-yl]oxy}-2-fluorophenyl)-N'-(4-fluorophenyl)cyclopropane-1,1-dicarboxamide,
(12) N-(4-Fluorophenyl)-N'-(2-fluoro-4-{[2-({[4-(pyrrolidin-1-ylmethyl)piperidin-1-yl]carbonyl}amino)pyridin-4-yl]oxy}phenyl)cyclopropane-1,1-dicarboxamide,
(13) N-(4-{[2-({[(3S)-3-(Dimethylamino)pyrrolidin-1-yl]carbonyl}ammo)pyridin-4-yl]oxy}-2-fluorophenyl)-N'-(4-fluorophenyl)cyclopropane-1,1-dicarboxamide,
(14) N-(4-{[2-({[(3R)-3-(Dimethylamino)pyrrolidin-1-yl]carbonyl}amino)pyridin-4-yl]oxy}-2-fluorophenyl)-N'-(4-fluorophenyl)cyclopropane-1,1-dicarboxamide,
(15) N-(2-Fluoro-4-{[2-({[methyl(1-methylpiperidin-4-yl)amino]carbonyl}amino)pyridin-4-yl]oxyl}phenyl)-N'-phenylcyclopropane-1,1-dicarboxamide,
(16) N-(2-Fluoro-4-{[2-({[4-(4-methylpiperazin-1-yl)piperidin-1-yl]carbonyl}amino)pyridin-4-yl]oxy}phenyl)-N'-phenylcycopropane-1,1-dicarboxamide,
(17) N-[4-({2-[({4-[3-(Dimethylamino)azetidin-1-yl]piperidin-1-yl}carbonyl)ammo]pyridin-4-yl}oxy)-2-fluorophenyl]-N'-phenylcyclopropane-1,1-dicarboxamide,
(18) N-(4-{[2-({[(1-Ethylpiperidin-4-yl)(methyl)amino]carbonyl}amino)pyridin-4-yl]oxy}-2-fluorophenyl)-N-phenylcyclopropane-1,1-dicarboxamide,
(19) N-[4-({2-[(Azetidin-1-ylcarbonyl)amino]pyridin-4-yl}oxy)-2-fluorophenyl]-N'-(4-fluorophenyl)cyclopropane-1,1-dicarboxamide,
(20) N-(4-Fluorophenyl)-N'-[2-fluoro-4-({2-[(pyrrolidin-1-ylcarbonyl)amino]pyridin-4-yl}oxy)phenyl]cyclopropane-1,1-dicarboxamide,
(21) N-{2-Fluoro-4-[(2-{[(3-hydroxyazetidin-1-yl)carbonyl]amino}pyridin-4-yl)oxy]phenyl}-N'-(4-fluorophenyl)cycopropane-1,1-dicarboxamide,
(22) N-[4-({2-[(1,3'-Biazetidin-1'-ylcarbonyl)anino]pyridin-4-yl}oxy)-2-fluorophenyl]-N'-(4-fluorophenyl)cycopropane-1,1-dicarboxamide,
(23) N-(2-Fluoro-4-{[2-({[3-(hydroxymethyl)azetidin-1-yl]carbonyl}amino)pyridin-4-yl]oxy}phenyl)-N'-(4-fluorophenyl)cyclopropane-1,1-dicarboxamide,
(24) N-(4-{[2-({[3-(Dimethylamino)azetidin-1-yl]carbonyl}amino)pyridin-4-yl]oxy}-2-fluorophenyl)-N'-(4-fluorophenyl)cyclopropane-1,1-dicarboxamide,
(25) N-[4-({2-[({3-[(Dimethylamino)methyl]azetidin-1-yl}carbonyl)amino]pyridin-4-yl}oxy)-2-fluorophenyl]-N'-(4-fluorophenyl)cyclopropane-1,1-dicarboxamide,
(26) N-{2-Fluoro-4- [(2-{[(4-hydroxypiperidin-1-yl)carbonyl]amino}pyridm-4-yl)oxy]phenyl}-N'-(4-fluorophenyl)cyclopropane-1,1-dicarboxamide,
(27) N-(2-Fluoro-4-{[2-({[4-(hydroxymethyl)piperidin-1-yl]carbonyl}amino)pyridin-4-yl]oxy}phenyl)-N'-(4-fluorophenyl)cyclopropane-1,1-dicarboxamide,
(28) N-(2-Fluoro-4-{[2-({[(3R)-3-hydroxypyrrolidin-1-yl]carbonyl}amino)pyridin-4-yl]oxy}phenyl)-N'-(4-fluorophenyl)cycopropane-1,1-dicarboxamide,
(29) N-(2-Fluoro-4-{[2-({[(3S)-3-hydroxypyrrolidin-1-yl]carbonyl}amino)pyridin-4-yl]oxy}phenyl)-N'-(4-fluorophenyl)cyclopropane-1,1-dicarboxamide,
(30) N-[4-({2-[(Azetidin-1-ylcarbonyl)amino]pyridin-4-yl}oxy)-2,5-difluorophenyl]-N'-(4-fluorophenyl)cyclopropane-1,1-dicarboxamide,
(31) N-{2,5-Difluoro-4-[(2-{[(3-hydroxyazetidin-1-yl)carbonyl]amino}pyridin-4-yl)oxy]phenyl}-N'-(4-fluorophenyl)cyclopropane-1,1-dicarboxamide,
(32) N-(2,5-Difluoro-4-{[2-({[4-(4-methylpiperazin-1-yl)piperidin-1-yl]carbonyl}amino)pyridin-4-yl]oxy}phenyl)-N'-(4-fluorophenyl)cyclopropane-1,1-dicarboxamide,
(33) N-[2,5-Difluoro-4-({2-[({3-[(dimethylamino)methy]azetidin-1-yl}carbonyl)amino]pyridin-4-yl}oxy)phenyl]-N'-(4-fluorophenyl)cyclopropane-1,1-dicarboxamide,
(34) N-(2,5-Difluoro-4-{[2-({[methyl(1-methylpiperidin-4-yl)amino]carbonyl}amino)pyridin-4-yl]oxy}phenyl)-N'-(4-fluorophenyl)cyclopropane-1,1-dicarboxamide,
(35) N-{4-[(2-{[3-(Azetidin-1-ylmethyl)azetidin-1-ylcarbonyl]amino}pyridin-4-yl)oxy]-2,5-difluorophenyl}-N'-(4-fluorophenyl)cyclopropane-1,1-dicarboxamide,
(36) N-(2,5-Difluoro-4-{[2-({[3-(hydroxymethyl)azetidin-1-yl]carbonyl}amino)pyridin-4-yl]oxy}phenyl)-N'-(4-fluorophenyl)cyclopropane-1,1-dicarboxamide,
(37) N-{2,5-Difluoro-4-[(4-{[(3-hydroxyazetidin-1-yl)carbonyl]ammo}pyrimidin-6-yl)oxy]phenyl}-N'-(4-fluorophenyl)cyclopropane-1,1-dicarboxamide,
(38) N-[4-({4-[({3-[(Dimethylamino)methyl]azetidin-1-yl}carbonyl)amino]pyrimidin-6-yl}oxy)-2,5-difluorophenyl]-N'-(4-fluorophenyl)cyclopropane-1,1-dicarboxamide,
(39) N-(2,5-Difluoro-4-{[4-({[3-(hydroxymethyl)azetidin-1-yl]carbonyl}amino)pyrimidin-6-yl]oxy}phenyl)-N'-(4-fluorophenyl)cyclopropane-1,1-dicarboxamide,
(40) N-(2,5-Difluoro-4-{[4-({[methyl(1-methylpiperidin-4-yl)amino]carbonyl}amino)pyrimidin-6-yl]oxy}phenyl)-N'-(4-fluorophenyl)cyclopropane-1,1-dicarboxamide,
(41) N-(2,5-Difluoro-4-{[4-({[4-(4-methylpiperazin-1-yl)piperidin-1-y1]carbonyl}amino)pyrimidin-6-yl]oxy}phenyl)-N'-(4-fluorophenyl)cyclopropane-1,1-dicarboxamide,
(42) N-(4-{[2-({[4-(Dimethylamino)piperidin-1-yl]carbonyl}amino)pyridin-4-yl]oxy}-2,5-difluorophenyl)-N'-(4-fluorophenyl)cyclopropane-1,1-dicarboxamide,
(43) N-{2,5-Difluoro-4-[(2-{[(4-methylpiperazin-1-yl)carbonyl]amino}pyridin-4-yl]oxy}phenyl)-N'-(4-fluorophenyl)cyclopropane-1,1-dicarboxamide,
(44) N-{2,5-Difluoro-4-[(2-{[(4-hydroxypiperidin-1-yl)carbonyl]amino}pyridin-4-yl)oxy]phenyl}-N'-(4-fluorophenyl)cyclopropane-1,1-dicarboxamide,
(45) N-{4-[(2-{[(4-Azetidin-1-ylpiperidin-1-yl)carbonyl]amino}pyridin-4-yl)axy]oxy}-2,5-difluorophenyl}-N'-(4-fluorophenyl)cyclopropane-1,1-dicarboxamide,
(46) N-(2,5-Difluoro-4-{[2-({[3-(2-dimethylaminoacetoxy)azetidin-1-yl]carbonyl}amino)pyridin-4-yl]oxy}phenyl)-N'-(4-fluorophenyl)cyclopropane-1,1-dicarboxamide,
(47) N-(2,5-Difluoro-4-{[2-({[(3S)-3-hydroxypyrrolidin-1-yl]carbonyl}amino)pyridin-4-yl]oxy}phenyl)-N'-(4-fluorophenyl)cyclopropane-1,1-dicarboxamide,
(48) N-(2,5-Difluoro-4-{[2-({[(3R)-3-hydroxypyrrolidin-1-yl]carbonyl}amino)pyridin-4-yl]oxy}phenyl)-N'-(4-fluorophenyl)cyclopropane-1,1-dicarboxamide or
(49) N-(3-Fluoro-4-{[6-({[methyl(1-methylpiperidin-4-yl)amino]carbonyl}amino)pyrimidin-4-y]oxy}phenyl)-N'-(4-fluorophenyl)cyclopropane-1,1-dicarboxamide.

81. The method according to any one of claims 56 to 61, wherein the compound represented by the formula (I) is
(1) N-(2-Fluoro-4-{[2-({[4-(4-methylpiperazin-1-yl)piperidin-1-yl]carbonyl}amino)pyridin-4-yl]oxy}phenyl)-N'-(4-fluorophenyl)cyclopropane-1,1-dicarboxamide,
(2) N-[4-({2-[(Azetidin-1-ylcarbonyl)amino]pyridin-4-yl}oxy)-2-fluorophenyl]-N'-(4-fluorophenyl)cyclopropane-1,1-dicarboxamide,
(3) N-{2,5-Difluoro-4-[(2-{[(3-hydroxyazetidin-1-yl)carbonyl]amino}pyridin-4-yl)oxy]phenyl}-N'-(4-fluorophenyl)cyclopropane-1,1-dicarboxamide,
(4) N-(2,5-Difluoro-4-{[2-({[4-(4-methylpiperazin-1-yl)piperidin-1-yl]carbonyl}amino)pyridin-4-yl]oxy}phenyl)-N'-(4-fluorophenyl)cyclopropane-1,1-dicarboxamide,
(5) N-(2,5-Difluoro-4-{[2-({[methyl(1-methylpiperidin-4-yl)amino]carbonyl}amino)pyridin-4-yl]oxy}phenyl)-N'-(4-fluorophenyl)cyclopropane-1,1-dicarboxamide or
(6) N-(2,5-Difluoro-4-{[2-({[3-(hydroxymethyl)azetidin-1-yl]carbonyl}amino)pyridin-4-yl]oxy}phenyl)-N'-(4-fluorophenyl)cyclopropane-1,1-dicarboxamide.

82. A method for administering a pyridine or pyrimidine derivative to a tumor patient, comprising the steps of:
assaying expression levels of hepatocyte growth factor receptor in tumor cells of a tumor patient and a non-tumor individual; and
administering a pyridine or pyrimidine derivative to the tumor patient if the expression level of hepatocyte growth factor receptor in tumor cells of the tumor patient is higher than the expression level of hepatocyte growth factor receptor in tumor cells of the non-tumor individual,
wherein the pyridine or pyrimidine derivative is at least one compound, salt thereof or solvate of the foregoing selected from the compound represented by the formula (1): 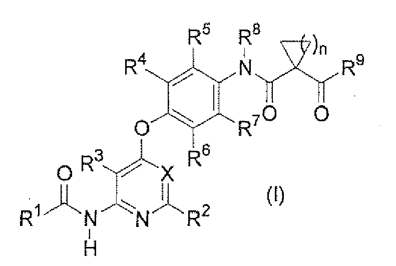
wherein R¹ represents a 3- to 10-membered non-aromatic heterocyclic group wherein the group is limited to a group having nitrogen as a ring constituent atom and the nitrogen having a bonding hand, or a group represented by the formula -NR^{11a}R^{11b}, wherein R^{11a} and R^{11b} may be the same or different and each represents hydrogen, C₁₋₆ alkyl, C₃₋₆ alkenyl, C₃₋₆ alkynyl, C₃₋₁₀ cycloalkyl, C₆₋₁₀ aryl, 5- to 10-membered heteroaryl or a 4- to 10-membered non-aromatic heterocyclic group, and R^{11a} and R^{11b} may be substituted with a substituent selected from Substituent Group A or Substituent Group B and R¹ may be substituted with a substituent selected from Substituent Group A or Substituent Group B;
R² and R³ represent hydrogen;
R⁴, R⁵, R⁶ and R⁷ may be the same or different and each represents hydrogen, halogen, hydroxyl, cyano, trifluoromethyl, C₁₋₆ alkyl, C₂₋₆ alkenyl, C₂₋₆ alkynyl, C₁₋₆ alkoxy, amino, mono-C₁₋₆ alkylamino, di-C₁₋₆ alkylamino or a group represented by the formula -CO-R¹², wherein R¹² represents hydrogen, hydroxyl, C₁₋₆ alkyl, C₁₋₆ alkoxy, amino, mono-C₁₋₆ alkylamino or di-C₁₋₆ alkylamino;
R⁸ represents hydrogen or C₁₋₆ alkyl;
R⁹ represents a 3- to 10-membered non-aromatic heterocyclic group wherein the group is limited to a group having nitrogen as a ring constituent atom and the nitrogen having a bonding hand, or a group represented by the formula - NR^{11a}R^{11b}, wherein R^{11a} and R^{11b} represent the same meaning as described above and R⁹ may be substituted with a substituent selected from Substituent Group A or Substituent Group B;
n represents an integer of 1 or 2; and
X represents a group represented by the formula -C(R¹⁰)= or nitrogen, wherein R¹⁰ represents hydrogen, halogen, cyano, C₁₋₆ alkyl, C₂₋₆ alkenyl, C₂₋₆ alkynyl or a group represented by the formula -CO-R¹², wherein R¹² represents the same meaning as recited above;
wherein Substituent Group A consists of halogen, hydroxyl, mercapto, nitro, cyano and oxo;
wherein Substituent Group B consists of C₁₋₆ alkyl, C₂₋₆ alkenyl, C₂₋₆ alkynyl, C₃₋₁₀ cycloalkyl, C₆₋₁₀ aryl, 5- to 10-membered heteroaryl, a 3- to 10-membered non-aromatic heterocyclic group, C₁₋₆ alkoxy, C₃₋₆ alkenyloxy, C₃₋₆ alkynyloxy, C₃₋₁₀ cycloalkoxy, C₆₋₁₀ aryloxy, 5- to 10-membered heteroaryloxy, 4-to 10-membered non-aromatic heterocyclicoxy, C₁₋₆ alkylthio, C₃₋₆ alkenylthio, C₃₋₆ alkynylthio, C₃₋₁₀ cycloalkylthio, C₆₋₁₀ arylthio, 5- to 10-membered heteroarylthio, 4- to 10-membered non-aromatic heterocyclicthio and a group represented by the formula -T¹-T²-T³, and each group in Substituent Group B may be substituted with a substituent selected from Substituent Group C, wherein T¹ represents a direct bond or C₁₋₆ alkylene, T² represents carbonyl, sulfmyl, sulfonyl, a group represented by the formula -C(=O)-O-, a group represented by the formula -O-C(=O)-, a group represented by the formula -SO₂-O-, a group represented by the formula -O-SO₂-, a group represented by the formula -NR^{T1}-, a group represented by the formula -C(=O)-NR^{T1}-, a group represented by the formula -NR^{T1}-C(=O)-, a group represented by the formula -SO₂-NR^{T1}- or a group represented by the formula -NR^{T1}-SO₂-, T³ represents hydrogen, C₁₋₆ alkyl, C₃₋₆ alkenyl, C₃₋₆ alkynyl, C₃₋₁₀ cycloalkyl, C₆₋₁₀ aryl, 5- to 10-membered heteroaryl or a 4- to 10-membered non-aromatic heterocyclic group, and R^{T1} represents hydrogen or C₁₋₆ alkyl; and
wherein Substituent Group C consists of halogen, hydroxyl, mercapto, nitro, cyano, oxo, C₁₋₆ alkyl, C₂₋₆ alkenyl, C₂₋₆ alkynyl, C₃₋₁₀ cycloalkyl, C₆₋₁₀ aryl, 5- to 10-membered heteroaryl, a 3- to 10-membered non-aromatic heterocyclic group, C₁₋₆ alkoxy, C₁₋₆ alkylthio, mono-C₁₋₆ alkylamino and di-C₁₋₆ alkylamino.

83. The method according to claim 82, wherein the method of assaying expression level of hepatocyte growth factor receptor is an immunological method.

84. The method according to claim 83, wherein the immunological method is an immunostaining method.

85. The method according to claim 82, wherein the method of assaying expression level of hepatocyte growth factor receptor is a method of assaying expression level of the gene.

86. The method according to claim 85, wherein the method of assaying expression level of the gene is a method of assaying gene amplification.

87. The method according to claim 86, wherein the method of assaying gene amplification is fluorescence *in situ* hybridization.
